# NOUVEAU FASCICULE DE BREVET EUROPEEN

(11) **EP 0 962 219 B2**
(45) Date de publication et mention de la décision concernant l'opposition: **04.08.2010**
(45) Mention de la délivrance du brevet: 06.10.2004
(21) Numéro de dépôt: 99401099.9
(22) Date de dépôt: 05.05.1999
(51) Int. Cl.: A61K 8/34

(54) **Compositon de teinture directe pour fibres kératiniques avec un colorant direct cationique et un polyol et/ou un ether de polyol**
Zusammensetzung für die direktziehende Färbung von keratinischen Fasern mit einem direkt ziehenden kationischen Farbstoff und einem Polyol und/oder einem Polyether
Composition for the direct dying of keratinous fibres with a direct cationic dye and a polyol and/or a polyolether

(30) Priorité: 28.05.1998 FR 9806751
(43) Date de publication de la demande: 08.12.1999
(73) Titulaire: L'Oréal, 75008 Paris (FR)
(72) Inventeur: Rondeau, Christine, 78500 Sartrouville (FR)
(74) Mandataire: Miszputen, Laurent

(56) Documents cités:
- EP-A- 0 850 636
- EP-A- 0 850 637
- EP-A- 0 850 638
- EP-B1- 0 806 198
- WO-A1-95/01772
- WO-A1-95/15144
- WO-A1-97/39727
- DE-A- 3 829 870
- FR-A- 2 282 860
- US-A- 3 985 499

## Description

L'invention concerne une composition de teinture directe pour fibres kératiniques, en particulier pour fibres kératiniques humaines telles que les cheveux comprenant, dans un milieu approprié pour la teinture exempt d'oxydases ou d'oxydoréductases, au moins un colorant direct cationique de formule donnée, et au moins un polyol et/ou un éther de polyol particulier.

L'invention a également pour objets les procédés et dispositifs de teinture mettant en oeuvre ladite composition.

Dans le domaine capillaire, la coloration semi-permanente ou temporaire, ou coloration directe, fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la tenacité, en terme de résistance aux diverses agressions que peuvent subir les cheveux (lumière, intempéries,shampooings), sur le plan de l'homogénéité de la couleur répartie le long de la fibre ("unisson"), on dit alors que la coloration est trop sélective, et sur le plan de la puissance.

Or, après d'importantes recherches menées sur la question, la demanderesse vient maintenant de découvrir qu'il est possible d'obtenir de nouvelles compositions pour la teinture directe des fibres kératiniques capables de conduire à des colorations résistant bien aux diverses agressions que peuvent subir les cheveux, plus puissantes et moins sélectives, en associant au moins un polyol et/ou un éther de polyol particulier à au moins un colorant direct cationique connu de l'art antérieur et de formules respectivement définies ci-après.

Cette découverte est à la base de la présente invention.

La présente invention a donc pour premier objet une composition pour la teinture directe des fibres kératiniques et en particulier des fibres kératiniques humaines telles que les cheveux, renfermant dans un milieu approprié pour la teinture, **(i)** au moins un colorant direct cationique dont la structure répond aux formules suivantes, caractérisée par le fait qu'elle contient en outre (ii)au moins un polyol et/ou un éther de polyol particulier
**(i)** Le colorant direct cationique utilisable selon la présente invention est un composé choisi parmi ceux de formules (I), (II), (III), (III') suivantes :
   **a) les composés de formule (I) suivante :** 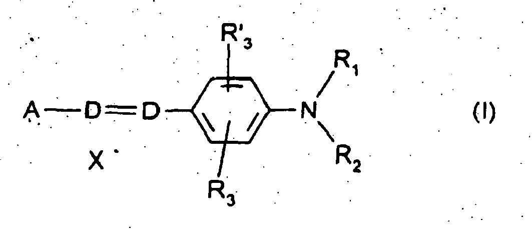 dans laquelle :
      D représente un atome d'azote ou le groupement -CH,
      R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
      R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
      X⁻ représente un anion de préférence choisi parmi le chlorure, le méthyl sulfate et l'acétate,
      A représente un groupement choisi par les structures A1 à A18 suivantes : 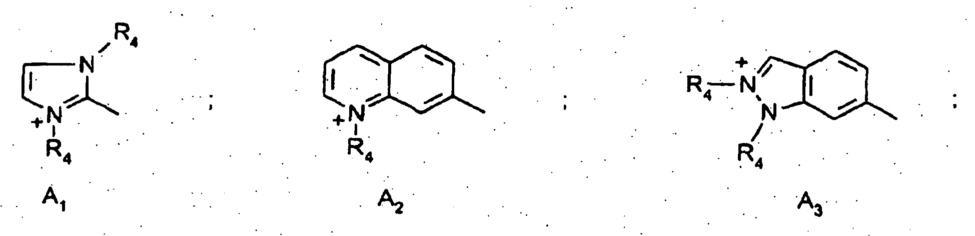 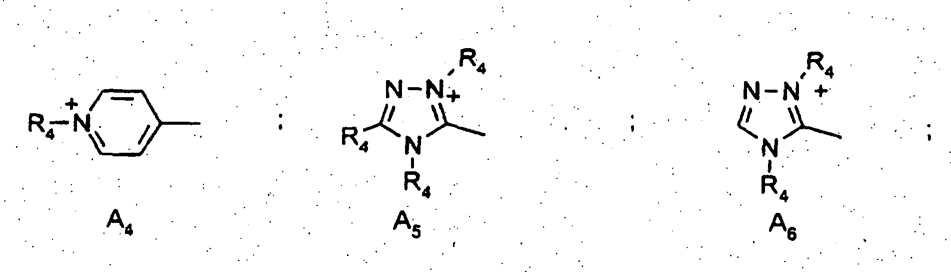 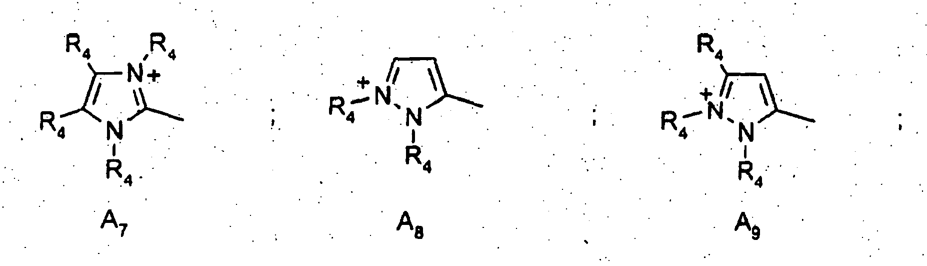 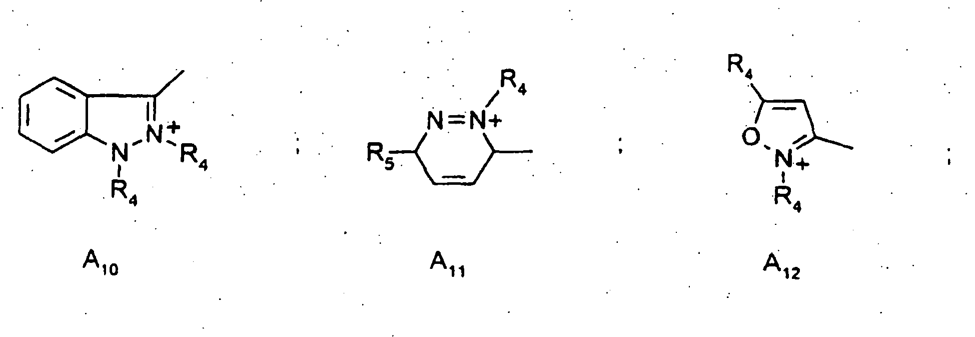 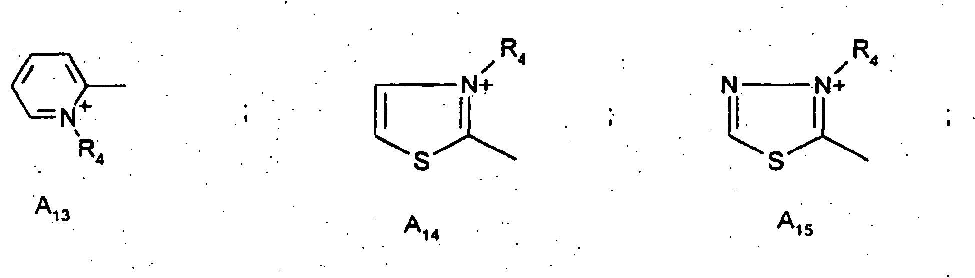 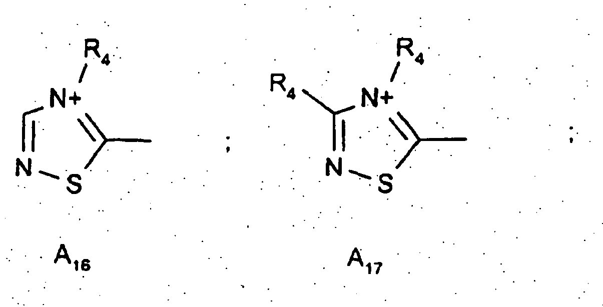 et 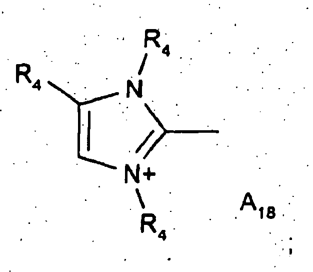
      dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R, et R₂ ne désignent pas simultanément un atome d'hydrogène ;
   **b) les composés de formule (II) suivante :** 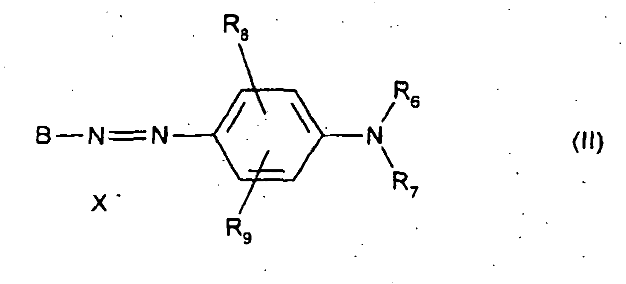 dans laquelle :
      R₆ représente un atome d'hydrogène ou un radical alkyle en C₁-C₄,
      R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
      R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
      X⁻ représente un anion de préférence choisi parmi le chlorure, le méthyl sulfate et l'acétate,
      B représente un groupement choisi par les structures B1 à B6 suivantes : 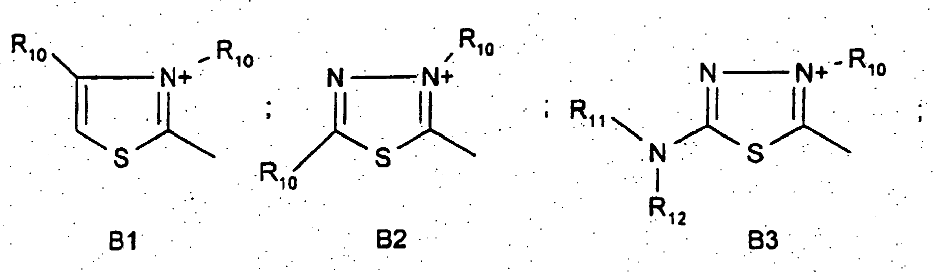 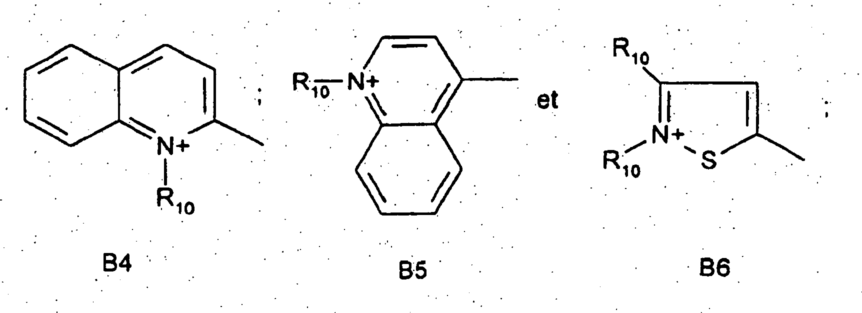
      dans lesquelles R₁₀ représente un radical alkyle en C₁-C₄, R₁₁ et R₁₂, identiques ou différents, représentent un atome d'hydrogène ou un radical alkyle en C₁-C₄ ;
   **c) les composés de formules (III) et (III') suivantes :** 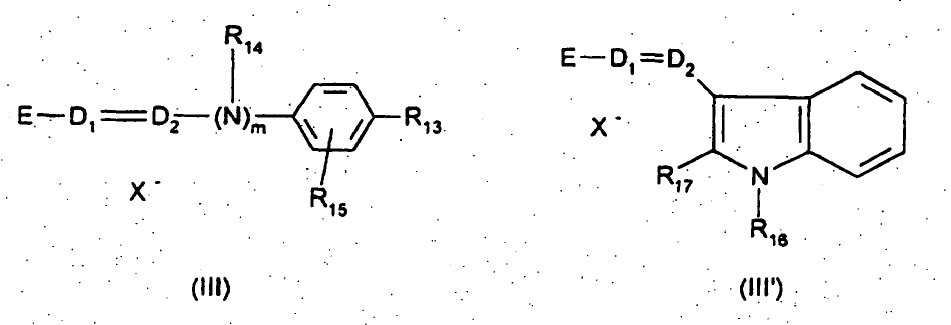 dans lesquelles :
      R₁₃ représente un atome d'hydrogène, un radical alcoxy en C₁-C₄, un atome d'halogène tel que le brome, le chlore, l'iode ou le fluor ou un radical amino,
      R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
      R₁₅ représente un atome d'hydrogène ou d'halogène tel que le brome, le chlore, l'iode ou le fluor,
      R₁₆ et R₁₇, identiques ou différents, représentent un atome d'hydrogène ou un radical alkyle en C₁-C₄,
      D₁ et D₂, identiques ou différents, représentent un atome d'azote ou le groupement -CH,
      m = 0 ou 1,
      étant entendu que lorsque R₁₃ représente un groupement amino non substitué, alors D₁ et D₂ représentent simultanément un groupement -CH et m = 0,
      X⁻ représente un anion de préférence choisi parmi le chlorure, le méthyl sulfate et l'acétate,
      E représente un groupement choisi par les structures E1 à E8 suivantes : 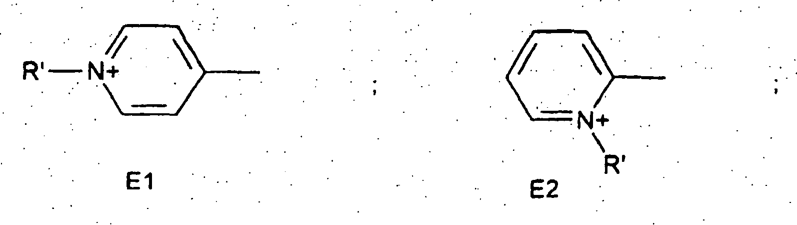 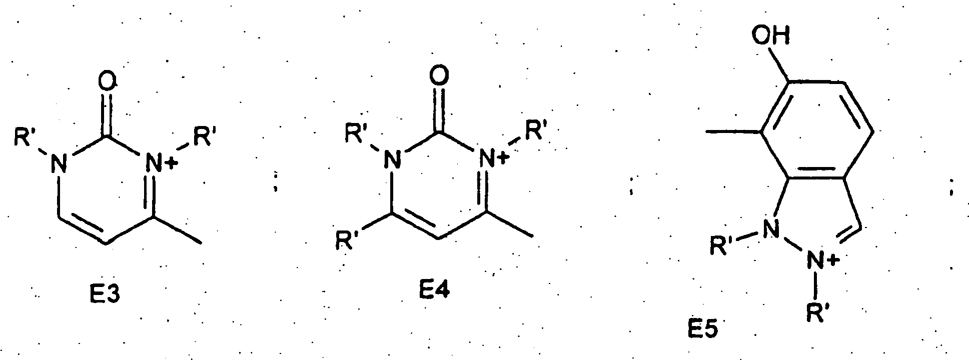 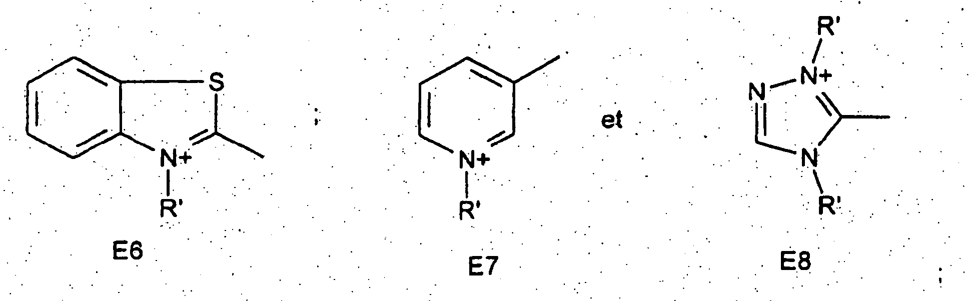 dans lesquelles R' représente un radical alkyle en C₁-C₄ ;
         lorsque m = 0 et que D, représente un atome d'azote, alors E peut également désigner un groupement de structure E9 suivante : 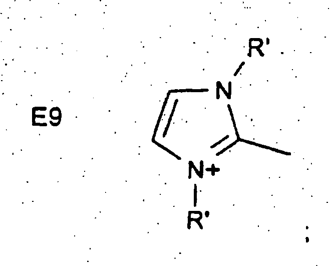 dans laquelle R' représente un radical alkyle en C₁-C₄.
   Les colorants directs cationiques de formules (I), (II), (III) et (III') utilisables dans les compositions tinctoriales conformes à l'invention, sont des composés connus et sont décrits par exemple dans les demandes de brevets WO 95/01772, WO 95/15144 et EP-A-0 714 954.
   Parmi les colorants directs cationiques de formule (I) utilisables dans les compositions tinctoriales conformes à l'invention, on peut plus particulièrement citer les composés répondant aux structures (I1) à (I54) suivantes : 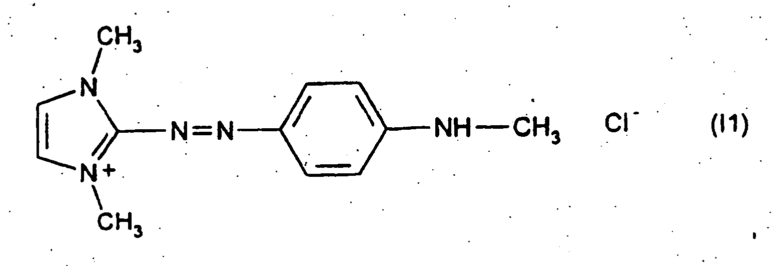 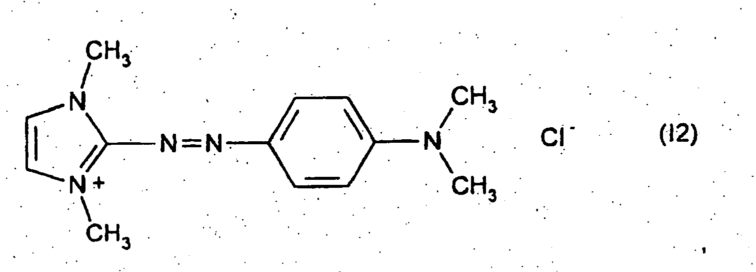 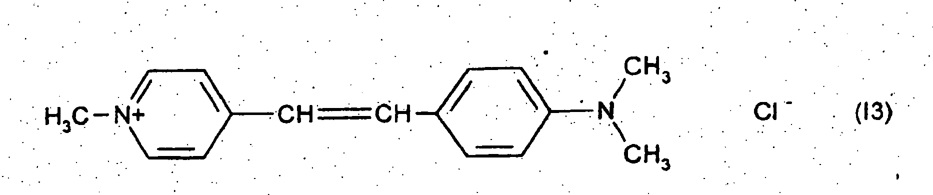 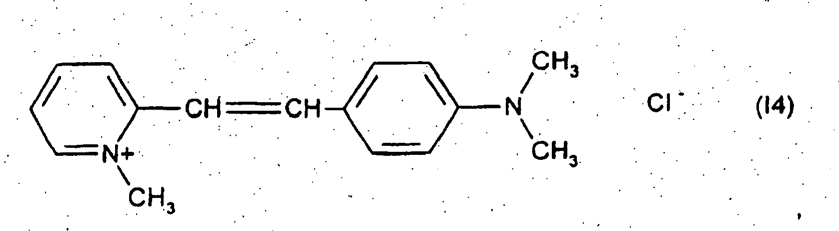 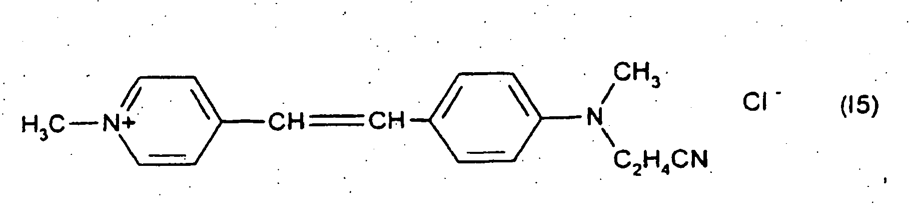 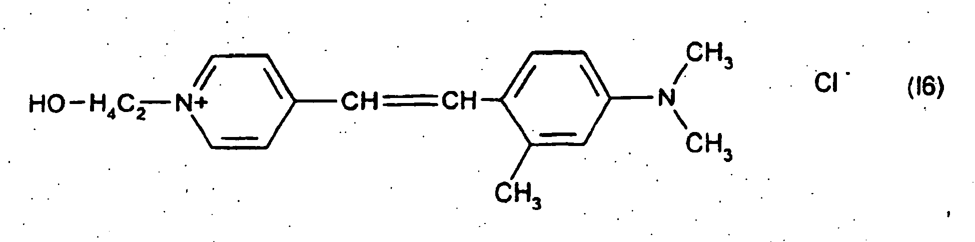 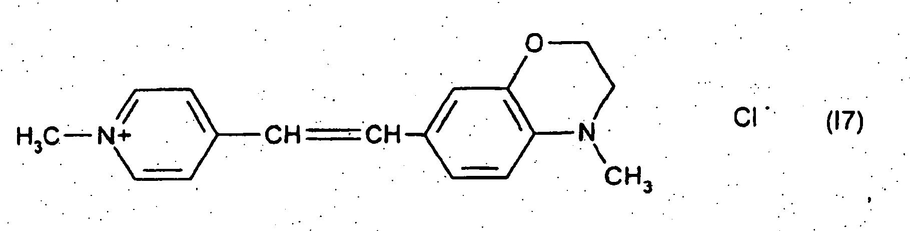 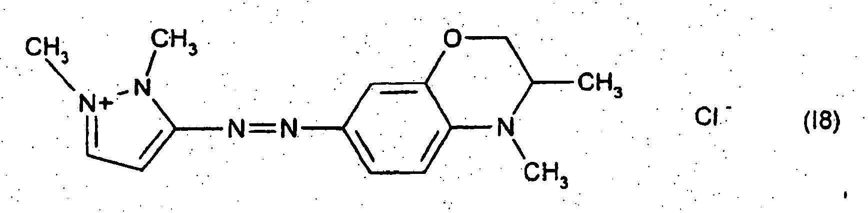 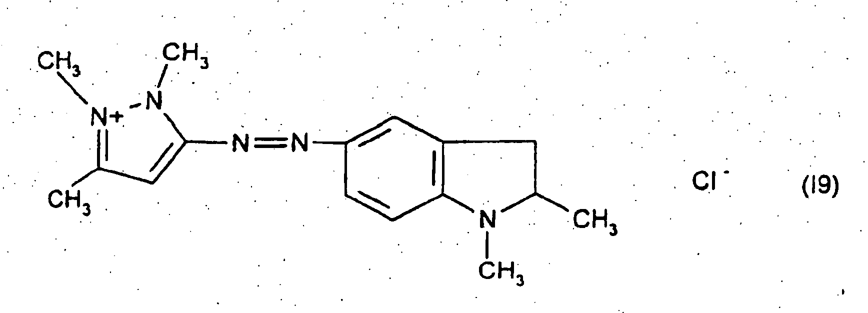 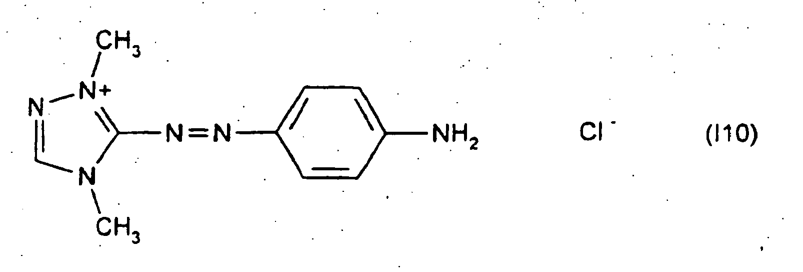 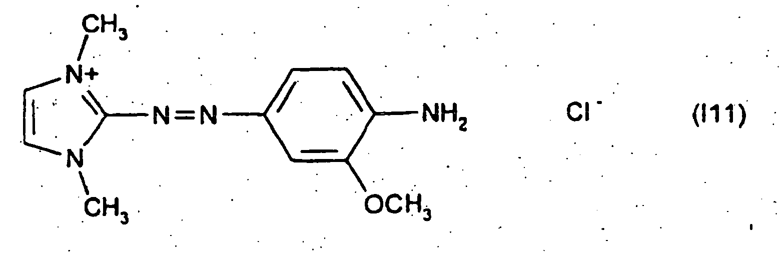 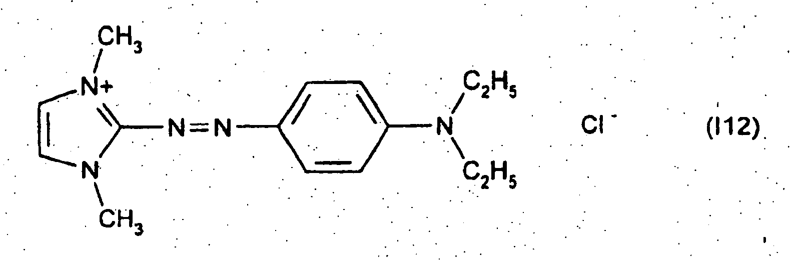 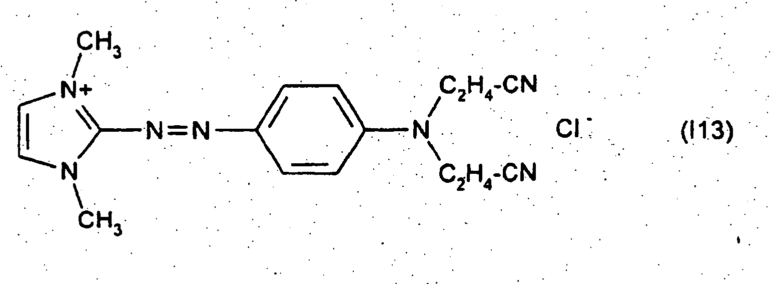 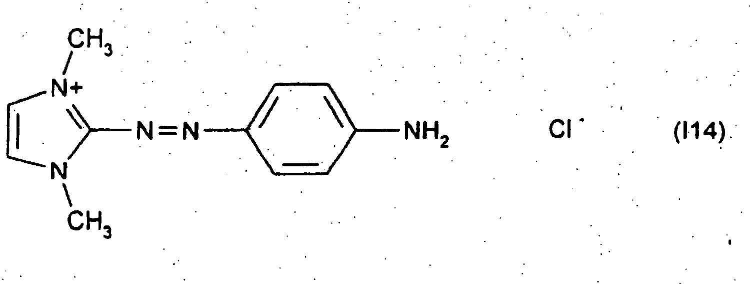 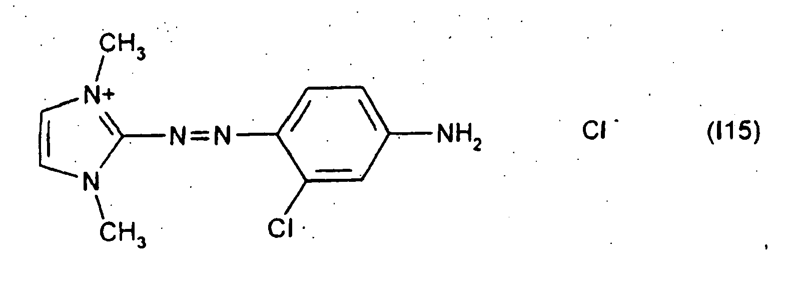 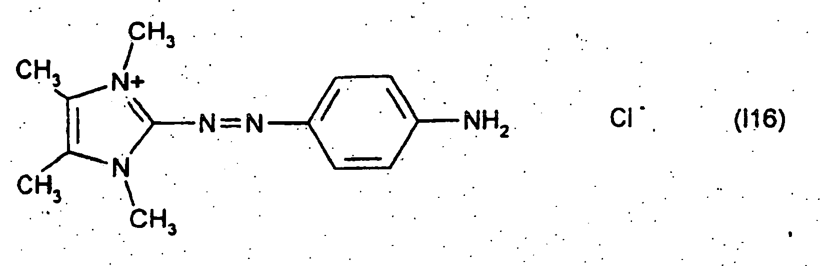 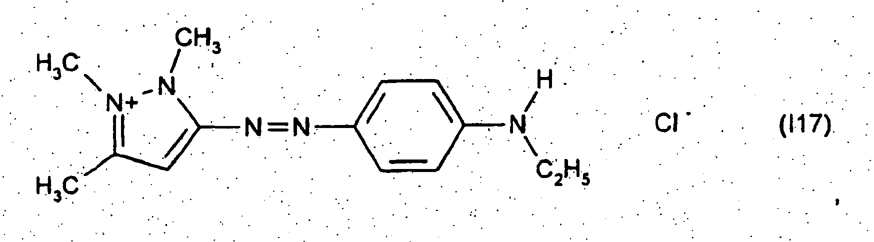 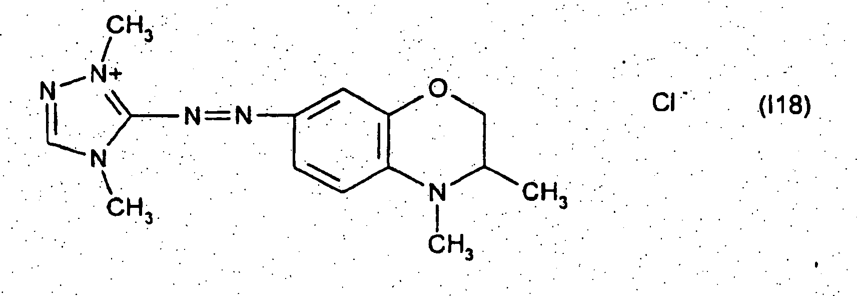 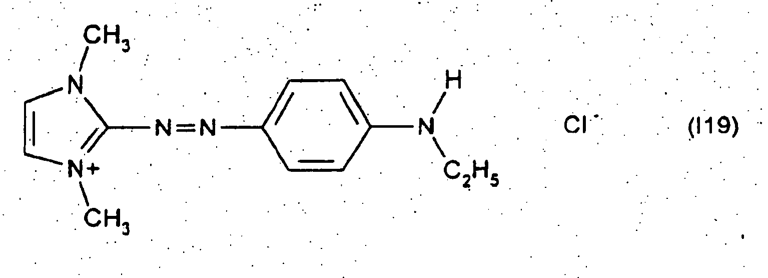 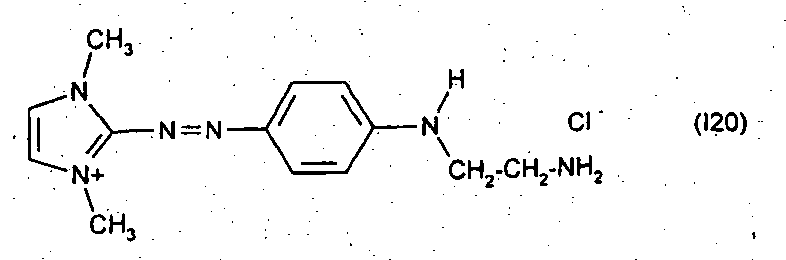 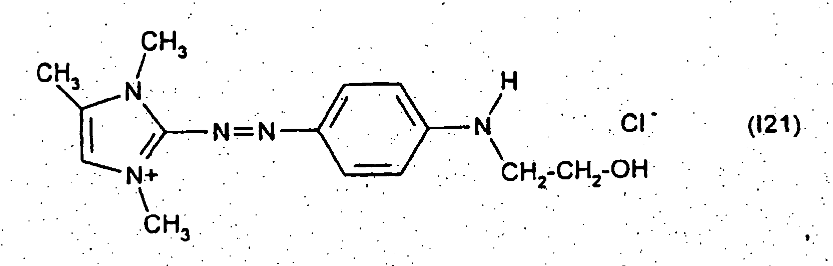 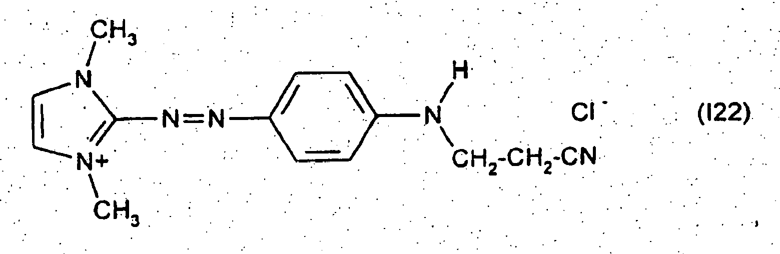 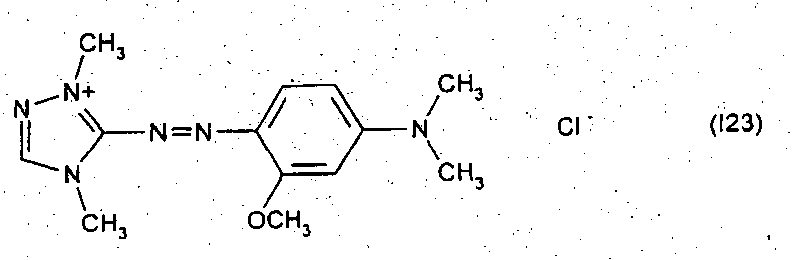 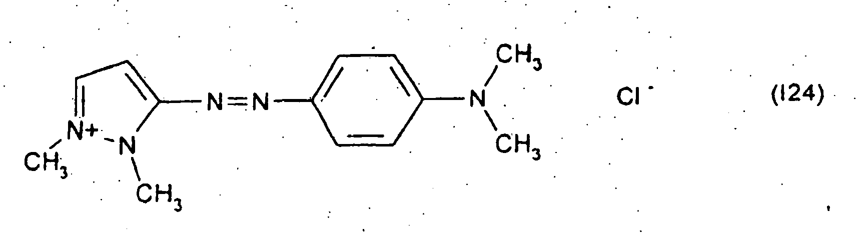 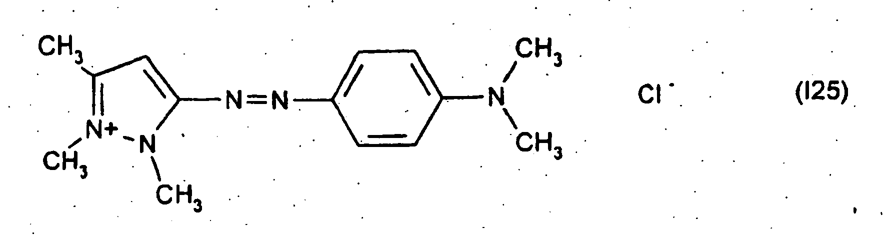 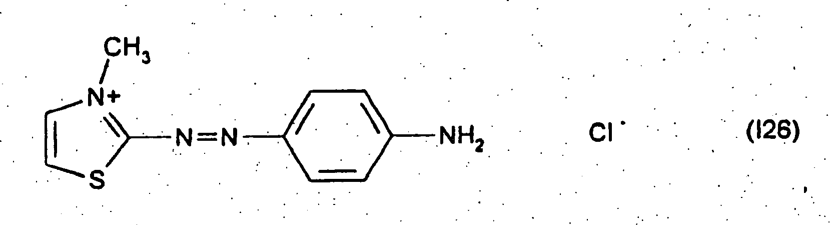 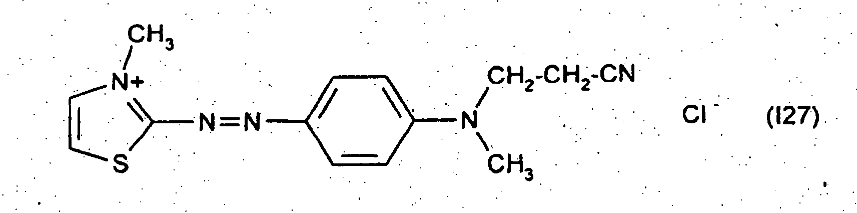 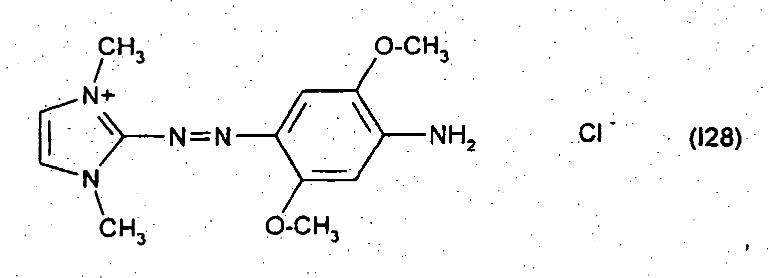 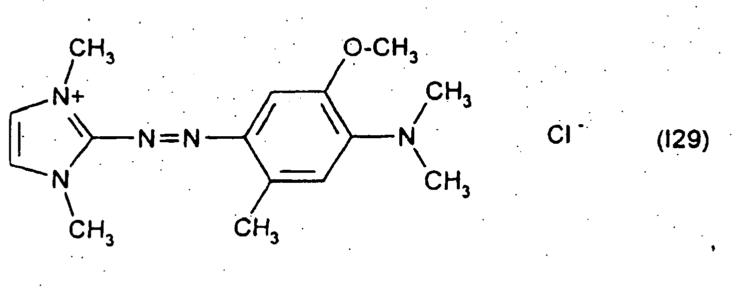 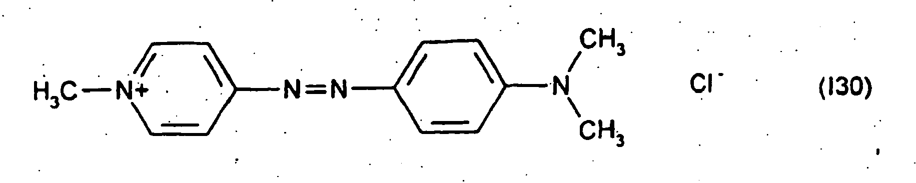 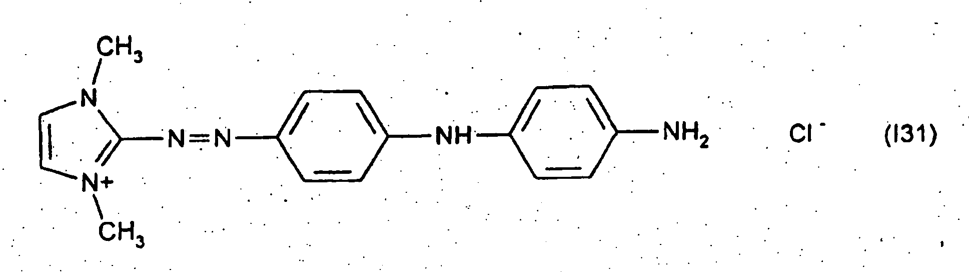 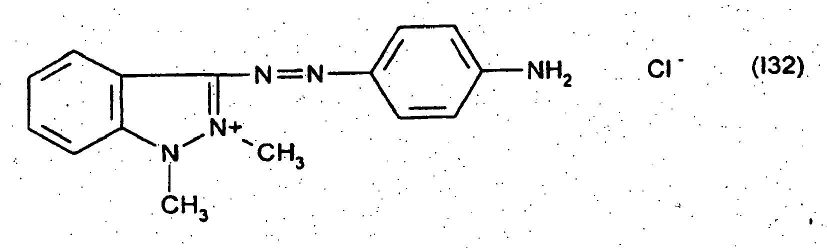 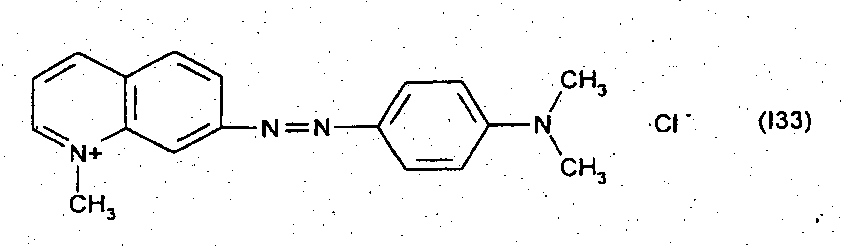 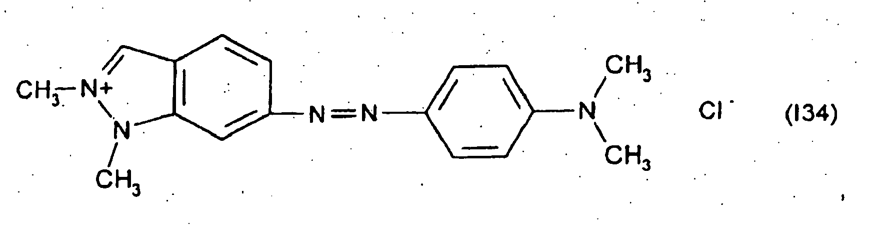 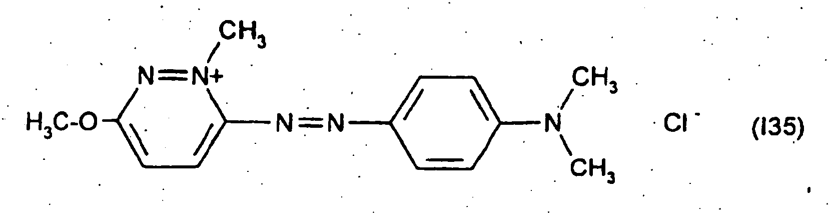 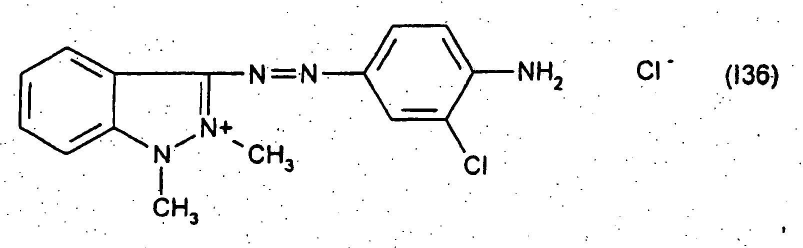 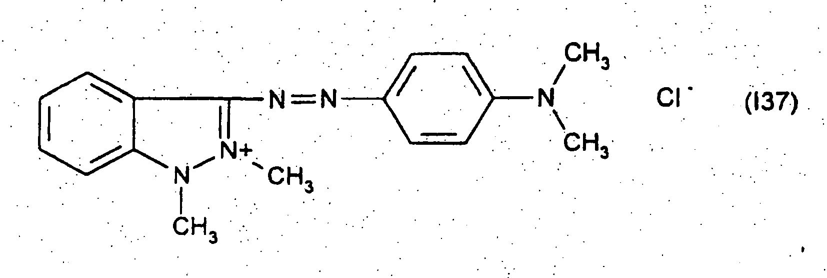 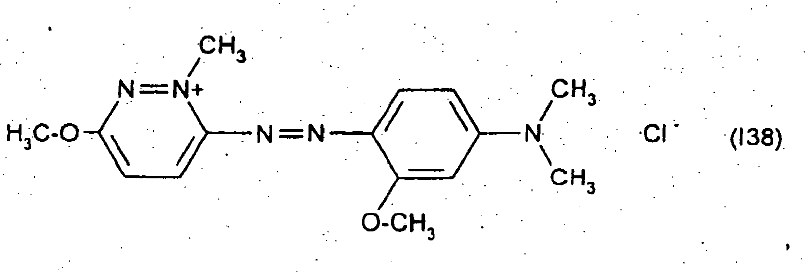 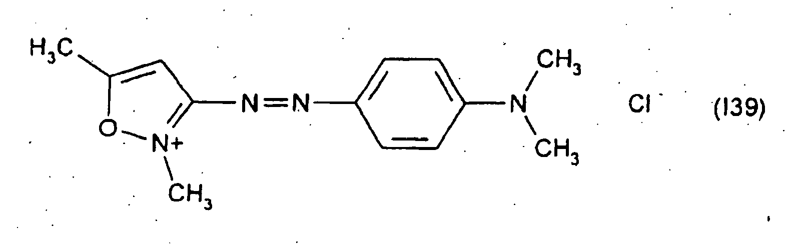 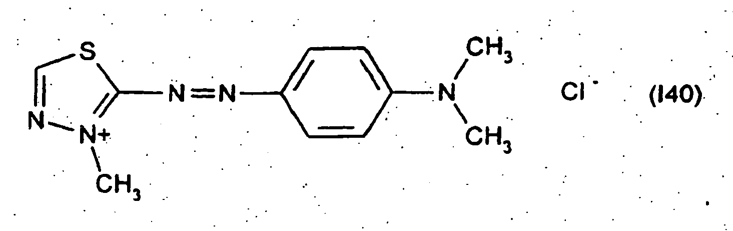 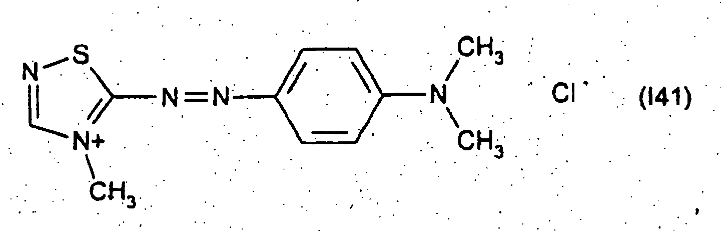 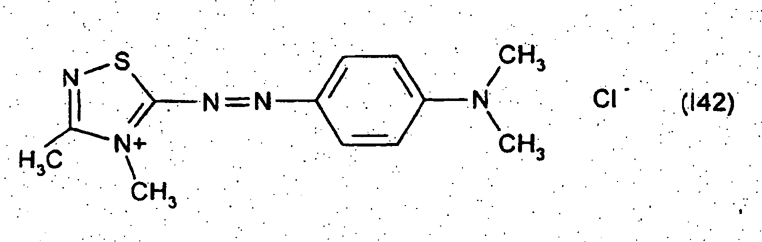 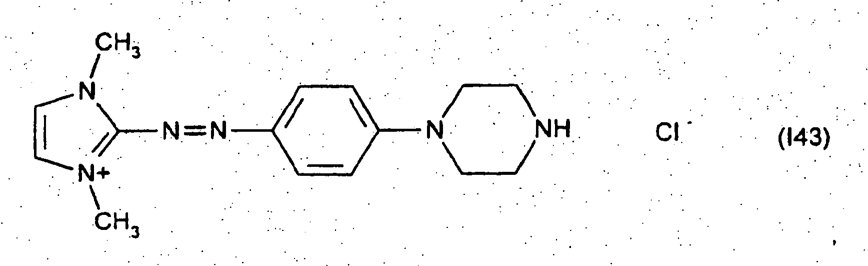 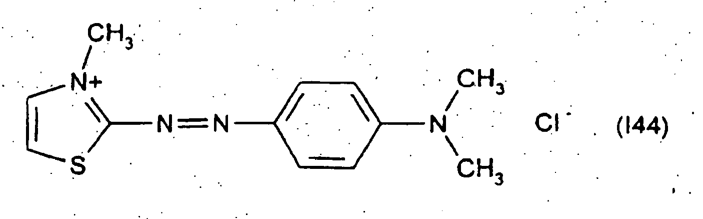 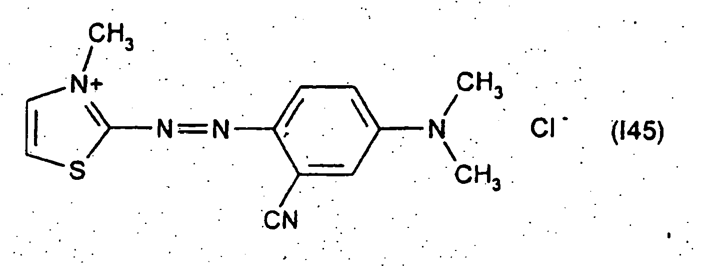 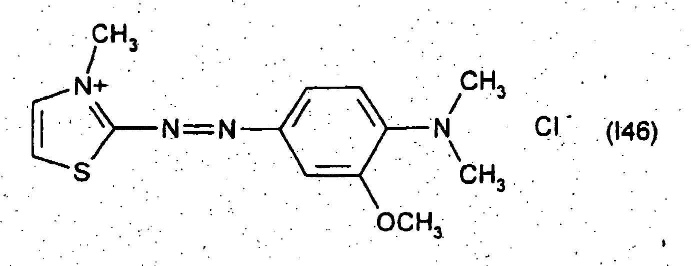 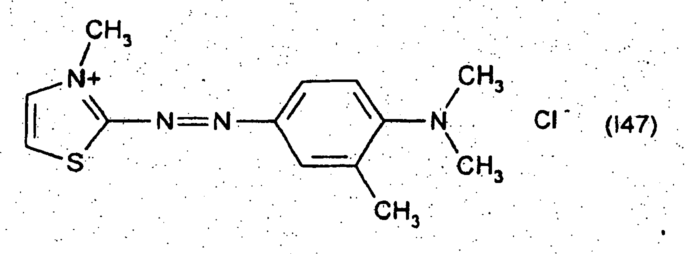 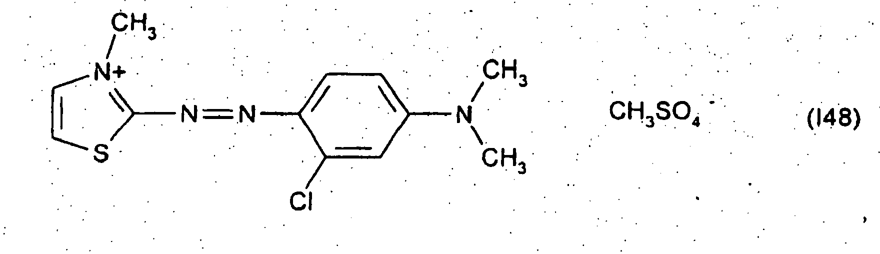 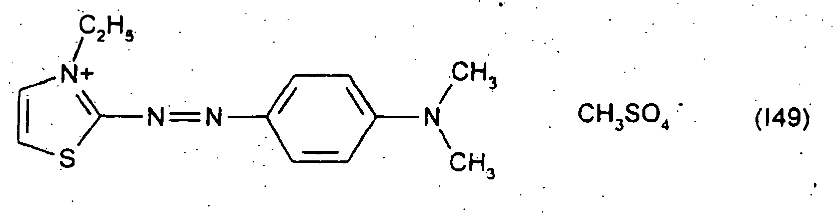 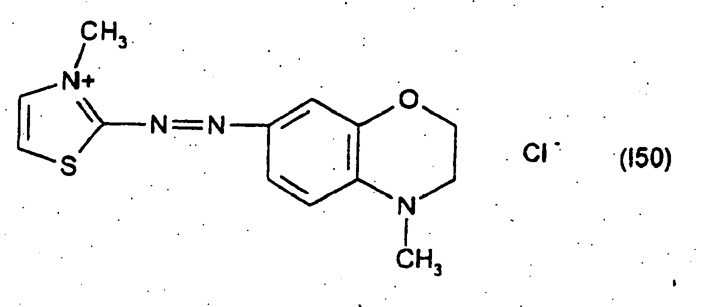 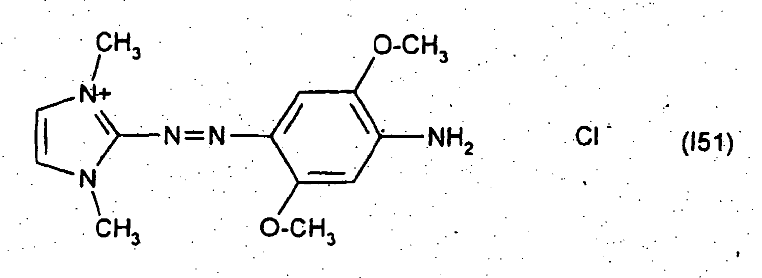 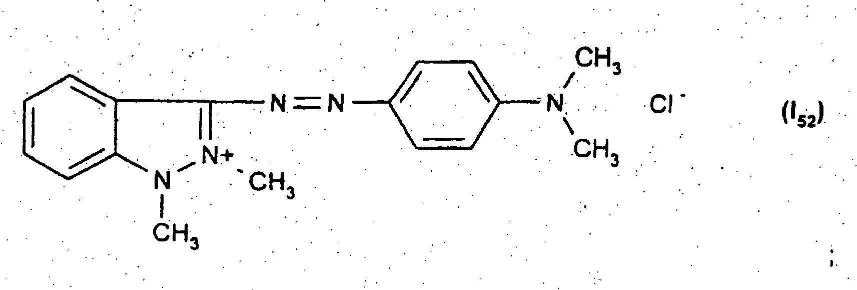 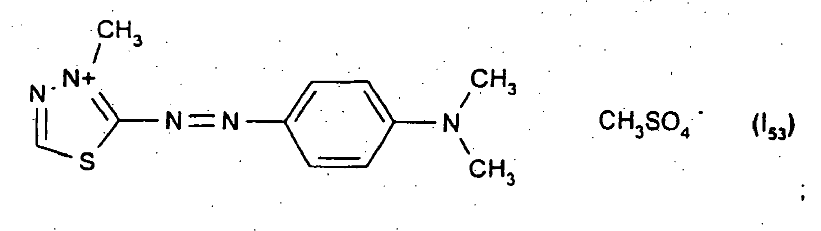 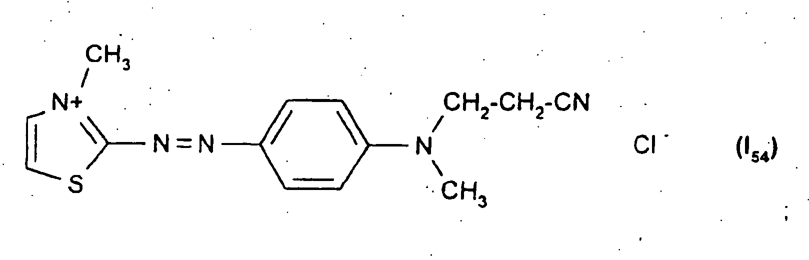 Parmi les composés de structures (I1) à (i54) décrits ci-dessus, on préfère tout particulièrement les composés répondant aux structures (I1), (I2), (I14) et (I31).
   Parmi les colorants directs cationiques de formule (II) utilisables dans les compositions tinctoriales conformes à l'invention, on peut plus particulièrement citer les composés répondant aux structures (I11) à (I19) suivantes : 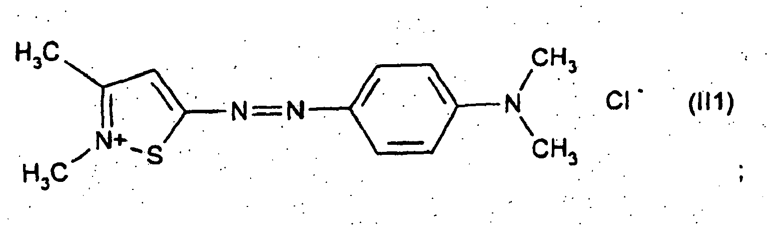 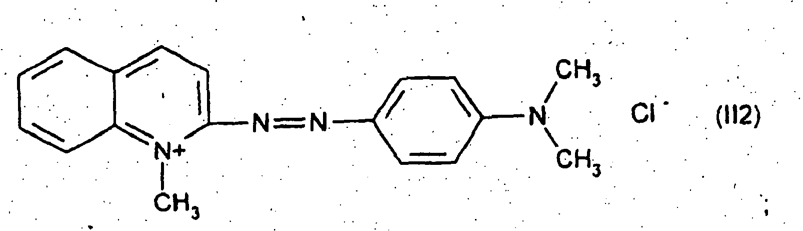 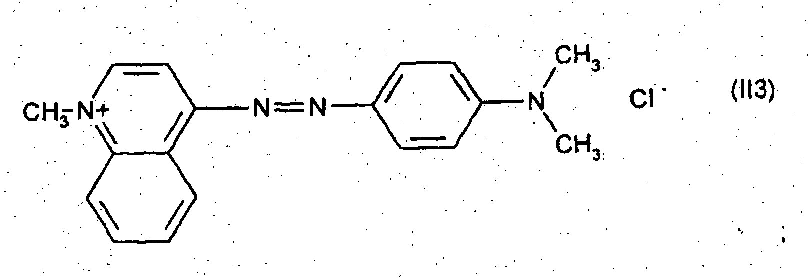 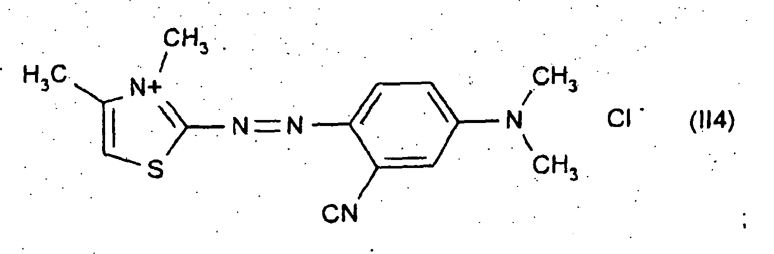 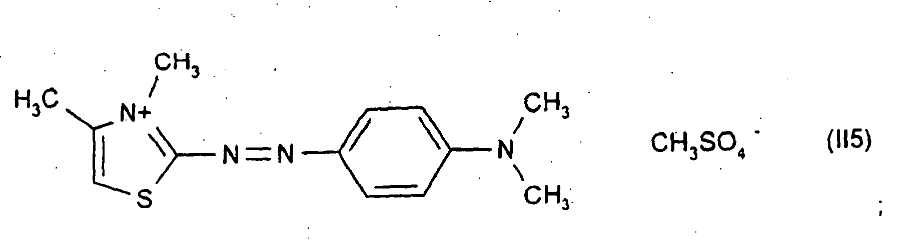 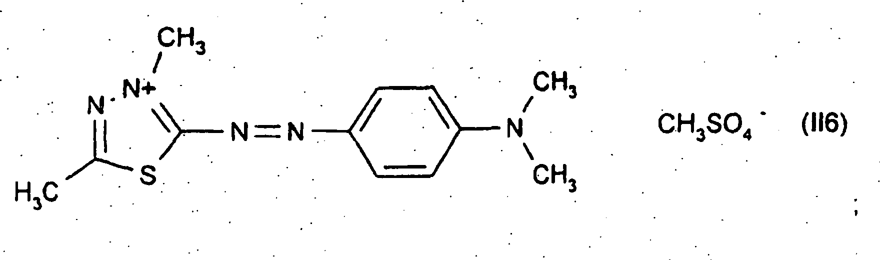 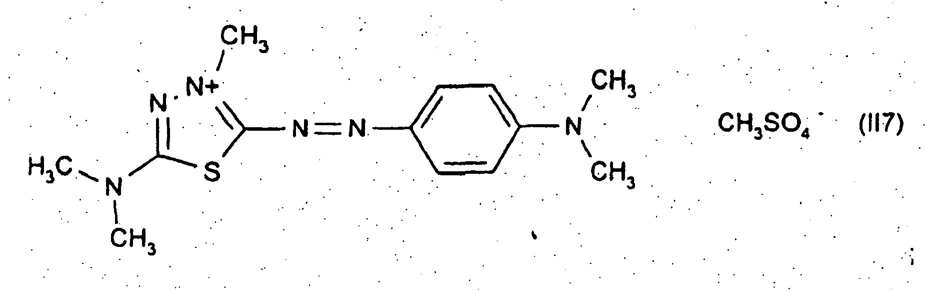 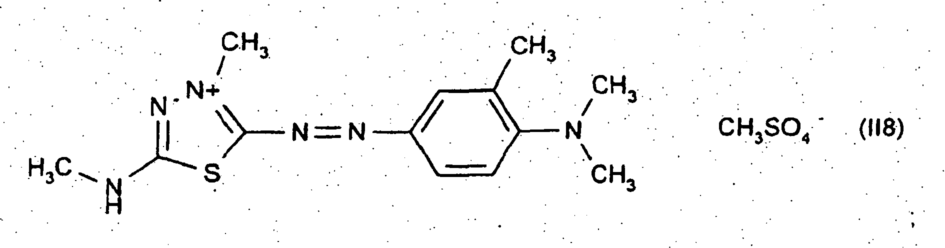 et 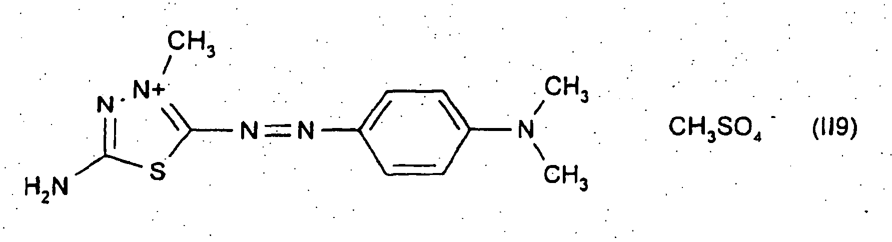 Parmi les colorants directs cationiques de formule (III), utilisables dans les compositions tinctoriales conformes à l'invention, on peut plus particulièrement citer les composés répondant aux structures (III1) à (III18) suivantes : 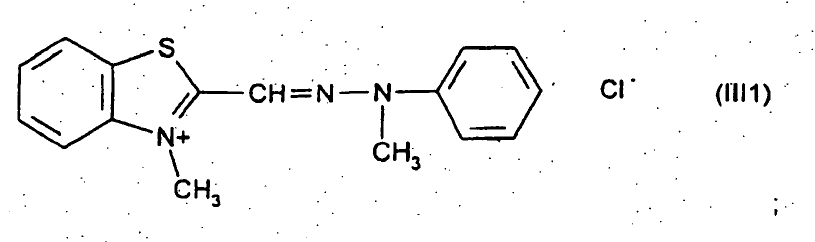 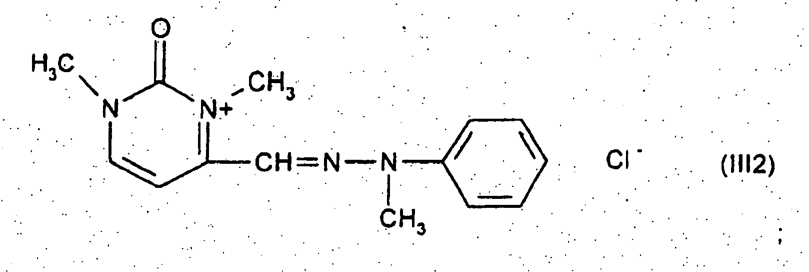 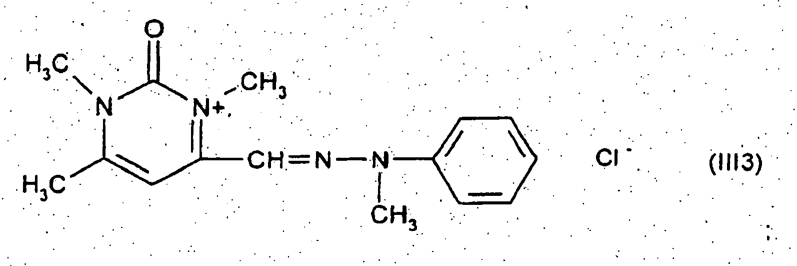 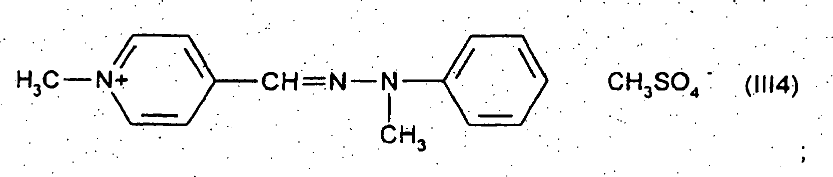 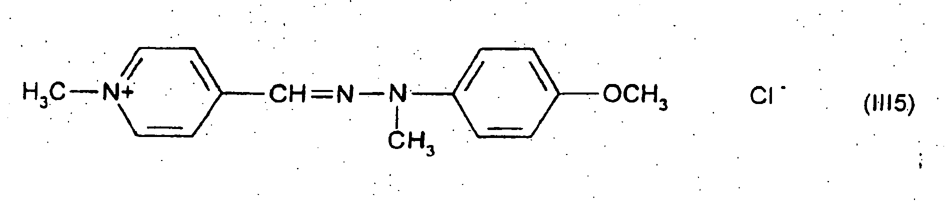 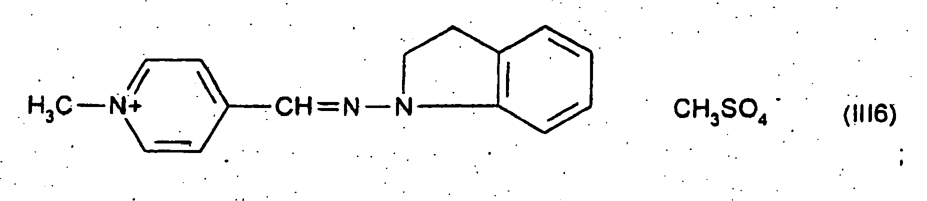 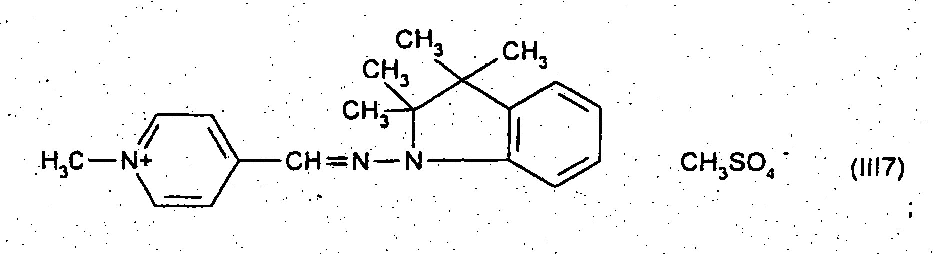 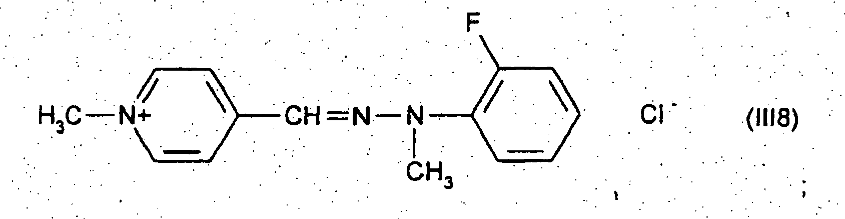 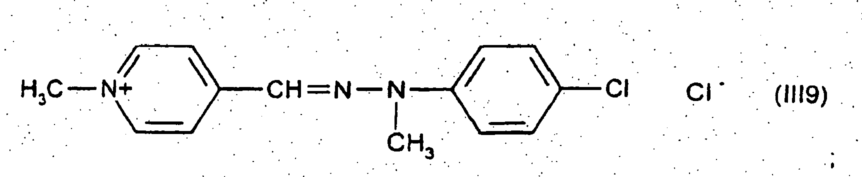 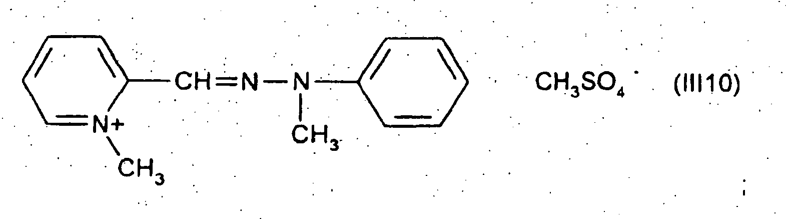 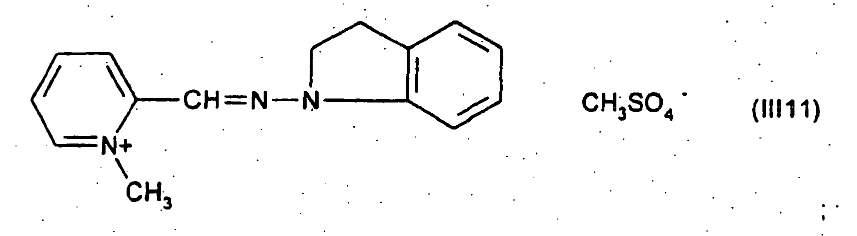 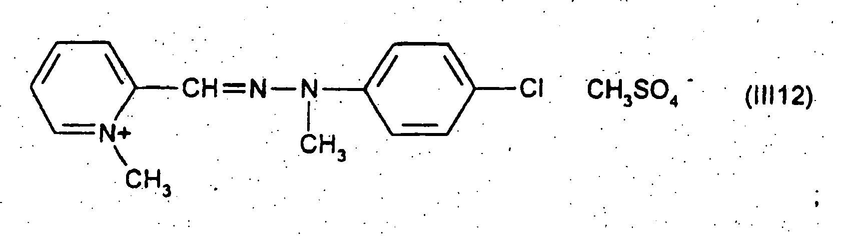 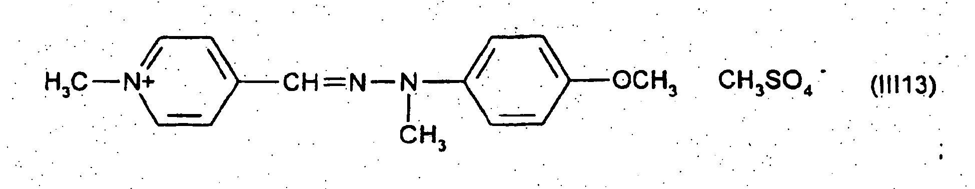 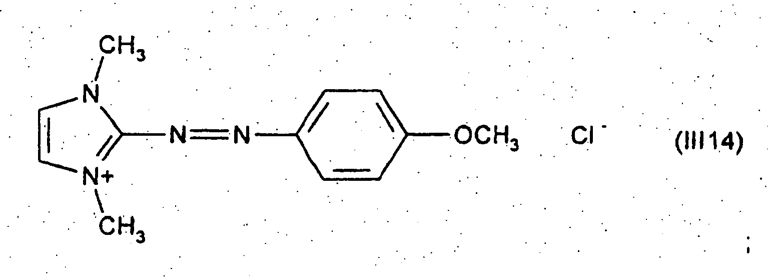 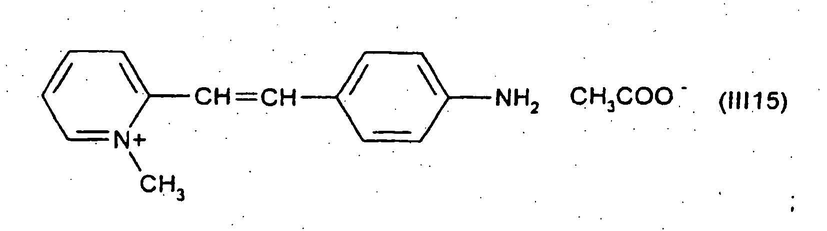 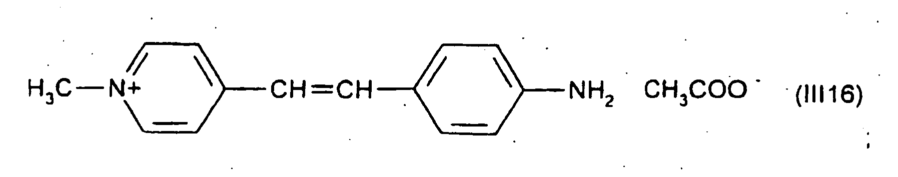 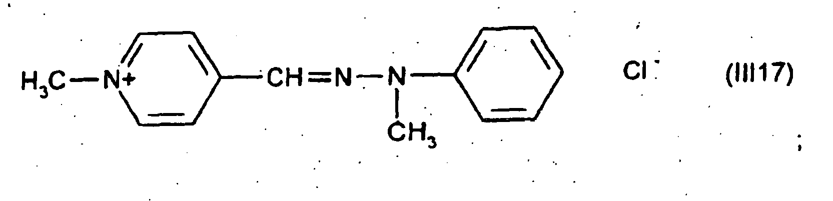 et 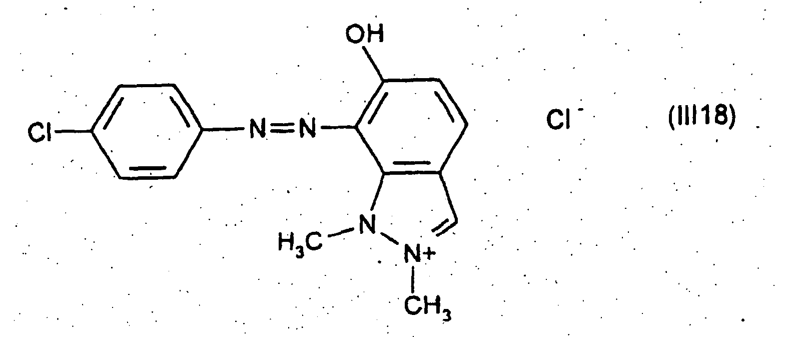 Parmi les composés particuliers de structures (III1) à (III18) décrits ci-dessus, on préfère tout particulièrement les composés répondant aux structures (III4), (III5) et (III13).
   Parmi les colorants directs cationiques de formule (III'), utilisables dans les compositions tinctoriales conformes à l'invention, on peut plus particulièrement citer les composés répondant aux structures (III'1) à (III'3) suivantes : 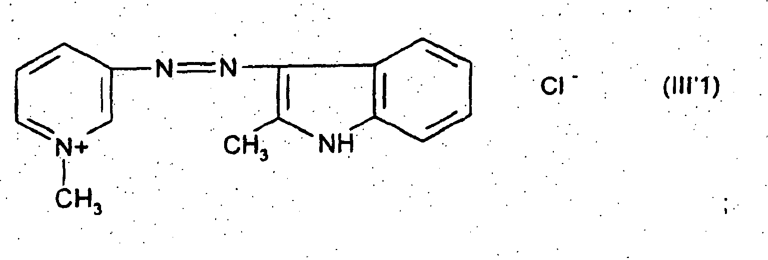 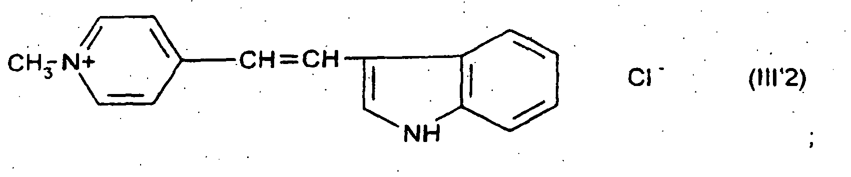 et 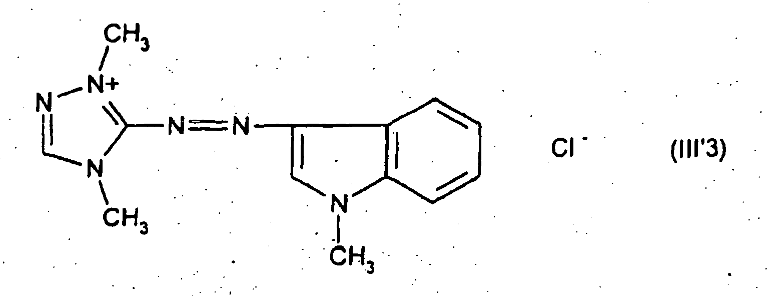 Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.
(ii) Par polyol, on désigne au sens de l'invention, un composé de type alkyle, linéaire, ramifié ou cyclique, saturé ou insaturé, portant au moins deux fonctions -OH sur la chaîne alkyle, ainsi que les polymères (polyéthers) de ces composés alkyle polyhydroxylés.
   De préférence, le composé alkyle comporte de 2 à 12 atomes de carbone, et encore plus préférentiellement de 2 à 9.

Les polyols utilisés selon l'invention sont choisis parmi les polyols en C₂-C₉, choisis parmi le 1,3-propanediol, le 2-butène-1,4-diol, le pentane-1,5-diol, le 2,2-diméthyl-propane-1,3-diol, le 3-méthyl-pentane-1,5-diol, le pentane-1,2-diol, le 2,2,4-triméthyl-pentane-1,3-diol, le 2-méthyl-propane-1,3-diol, l'hexylèneglycol, le 1,3-butylèneglycol, le diéthylèneglycol, le triéthylèneglycol, et parmi les éthers aliphatiques en C₁-C₈ de polyols en C₃-C₉ et les éthers aromatiques en C₆-C₈ de polyols en C₂-C₉ choisis parmi le monoéthyléther de propylèneglycol, le diméthyléther d'isopropylèneglycol, le monométhyléther de dipropylèneglycol, le monométhyléther de tripropylèneglycol et le diméthyléther de diéthylèneglycol ; le monophényléther d'éthylèneglycol ou le monobenzyléther d'éthylèneglycol, le monophényléther de propylèneglycol, le monobenzyléther de propylèneglycol, le monophényléther de diéthylèneglycol et le monobenzyléther de diéthylèneglycol.

Le ou les polyols et/ou le ou les éthers de polyols décrits au sens de l'inventicn sont présents dans la composition tinctoriale conforme à l'invention dans des proportions généralement comprises entre 0,1 et 40% en poids, et encore plus particulièrement de 0,5 à 20% en poids par rapport au poids total de la composition.

Le milieu approprié pour la teinture (ou support) est généralement constitué par un mélange d'eau et d'au moins un polyol et/ou un éther de polyol tel que défini ci-dessus. Il peut contenir en outre un ou des solvants organiques différents du ou des polyols et/ou du ou des éthers de polyols utilisés conformément à l'invention, pour solubiliser les composés qui ne seraient pas suffisamment solubles dans le milieu. A titre de solvant organique, on peut par exemple citer les alcanols en C₁-C₄, tels que l'éthanol et l'isopropanol ainsi que les alcools aromatiques comme l'alcool benzylique, les produits analogues et leurs mélanges.

Lesdits solvants organiques additionnels peuvent être présents dans des proportions de préférence comprises entre 0,5 et 40 % en poids environ par rapport au poids total de la composition tinctoriale, et encore plus préférentiellement entre 1 et 20 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture directe des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 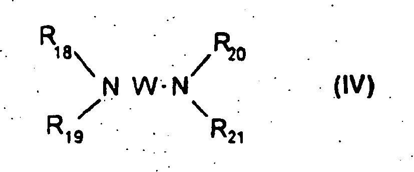 dans laquelle W est un reste propylène éventuellement substitué par un groupement hydroxyle ou un radical alkyle en C₁-C₆ ; R₁₈, R_{19'} R₂₀ et R₂₁, identiques ou différents, représentent un atome d'hydrogène, un radical alkyle en C₁-C₆ ou hydroxyalkyle en C₁-C₆.

La composition tinctoriale conforme à l'invention peut contenir, en plus du ou des colorants directs cationiques (i) définis précédemment, un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

La composition tinctoriale conforme à l'invention peut également renfermer divers adjuvants utilisés classiquement dans les compositions pour la teinture directe des cheveux, tels que des agents antioxydants, des agents de pénétration, des agents séquestrants, des parfums, des tampons, des agents dispersants, des agents tensioactifs, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition de teinture directe conforme à l'invention ne soient pas, ou substantiellement pas, altérées par la ou les adjonctions envisagées.

La composition de teinture directe selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant le ou les polyols et/ou le ou les éthers de polyols particuliers.

Lorsque l'association du colorant direct cationique (i) et du polyol et/ou de l'éther de polyol selon l'invention (ii) est utilisée dans une composition destinée à la teinture directe éclaircissante, alors la composition tinctoriale conforme à l'invention renferme en outre au moins un agent oxydant différent d'une enzyme telle que les oxydases et les oxydoréductases mais choisi notamment parmi le peroxyde d'hydrogène, le peroxyde d'urée, les bromates de métaux alcalins, les persels tels que les perborates et persulfates. L'utilisation du peroxyde d'hydrogène est particulièrement préférée.

Un autre objet de l'invention est un procédé de teinture directe des fibres kératiniques et en particulier des fibres kératiniques humaines telles que les cheveux mettant en oeuvre la composition tinctoriale telle que définie précédemment.

Selon une première variante de ce procédé de teinture directe conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture directe conforme à l'invention, on applique sur les fibres au moins une composition tinctoriale telle que définie précédemment, pendant un temps suffisant pour développer la coloration désirée, sans rinçage final.

Selon une autre forme de réalisation particulière de ce procédé de teinture directe, et lorsque la composition tinctoriale conforme à l'invention renferme au moins un agent oxydant, le procédé de teinture comporte une étape préliminaire consistant à stocker sous forme séparée, d'une part, une composition (A) comprenant, dans un milieu approprié pour la teinture, au moins un colorant direct cationique tel que défini précédemment et, d'autre part, une composition (B) renfermant, dans un milieu approprié pour la teinture, au moins un agent oxydant, puis à procéder à leur mélange au moment de l'emploi avant d'appliquer ce mélange sur les fibres kératiniques, la composition (A) ou la composition (B) contenant au moins un polyol et/ou un éther de polyol tel que défini précédemment.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

### EXEMPLE 1 :

On a préparé la composition de teinture suivante :

| | |
|---|---|
| Colorant direct cationique de formule I(14) | 0,2 g |
| Propylène glycol | 10,0 g |
| 2-amino-2-méthyl-propanol q.s. | pH 9 |
| Eau déminéralisée q.s.p | 100 g |
| M.A.*: Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance orangé puissant.

### EXEMPLE 2 :

On a préparé la composition de teinture suivante :

| | |
|---|---|
| Colorant direct cationique de formule I(1) | 0,2 g |
| Monométhyléther de propylène glycol | 10,0 g |
| 2-amino-2-méthyl-propanol q.s. | pH 9 |
| Eau déminéralisée q.s.p | 100 g |
| M.A.*: Matière Active | |

La composition ci-dessus a été appliquée pendant 30 minutes sur des mèches de cheveux gris naturels à 90 % de blancs. Les mèches de cheveux ont ensuite été rincées, lavées avec un shampooing standard puis séchées.

Elles ont été teintes dans une nuance rouge puissant.

## Revendications

1. Composition pour la teinture directe des fibres kératiniques et en particulier des fibres kératiniques humaines telles que les cheveux renfermant, dans un milieu approprié pour la teinture exempt d'oxydases ou d'oxydoréductases, (i) au moins un colorant direct cationique de formules suivantes (I), (II), (III), (III') : 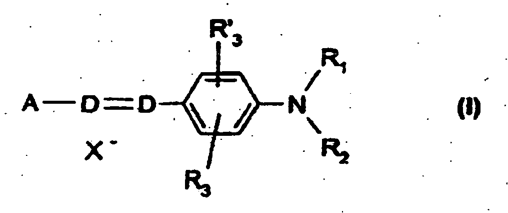 **formule (I) dans laquelle :**
D représente un atome d'azote ou le groupement -CH,
**R₁** et **R₂**, identiques ou différents, représentent un atome d'hydrogène ; un radical alkyle en C₁-C₄ pouvant être substitué par un radical -CN, -OH ou -NH₂ ou forment avec un atome de carbone du cycle benzénique un tiétérocycle éventuellement oxygéné ou azoté, pouvant être substitué par un ou plusieurs radicaux alkyle en C₁-C₄ ; un radical 4'-aminophényle,
**R₃** et **R'₃**, identiques ou différents, représentent un atome d'hydrogène ou d'halogène choisi parmi le chlore, le brome, l'iode et le fluor, un radical cyano, alkyl en C₁-C₄ alcoxy en C₁-C₄ ou acétyloxy,
**X ⁻** représente un anion de préférence choisi parmi le chlorure, le méthyl sulfate et l'acétate,
**A** représente un groupement choisi par les structures A1 à A18 suivantes : 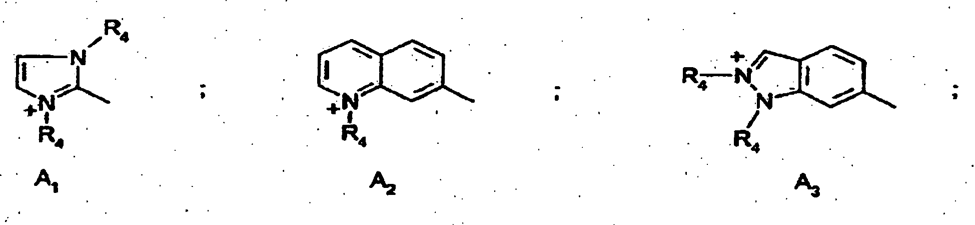 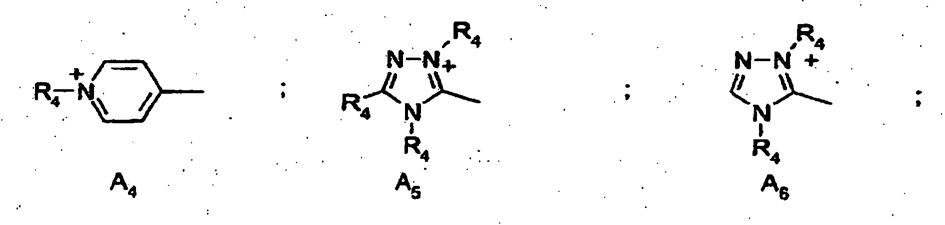 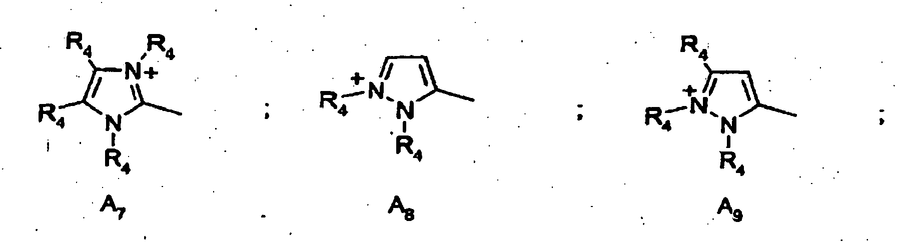 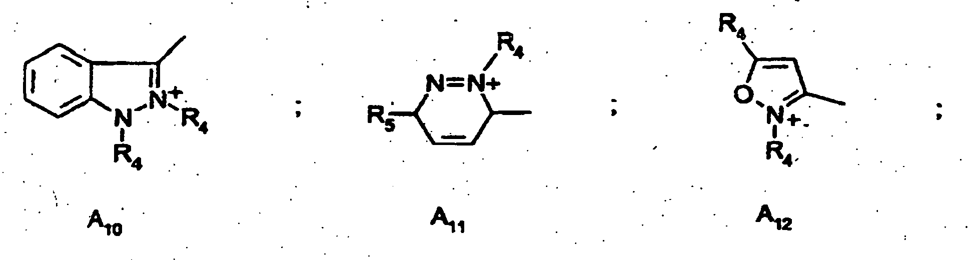 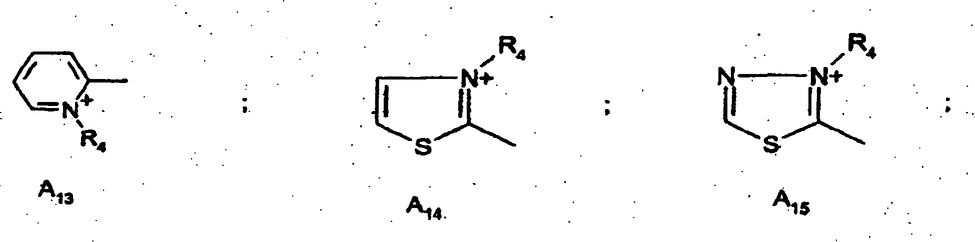 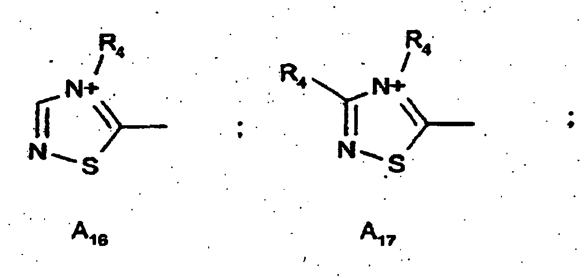 et 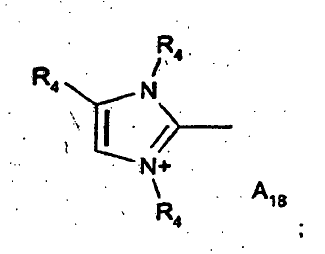
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ; 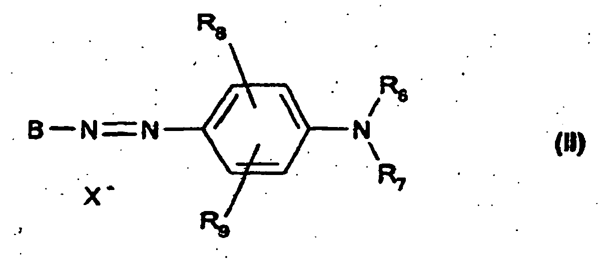 **formule (II) dans laquelle :**
**R₆** représente un atome d'hydrogène ou un radical alkyle en C₁-C₄,
**R₇** représente un atome d'hydrogène, un radical alkyle pouvant être substitué par un radical -CN ou par un groupement amino, un radical 4'-aminophényle ou forme avec **R₆** un hétérocycle éventuellement oxygéné et/ou azoté pouvant être substitué par un radical alkyle en C₁-C₄,
**R₈** et **R₉,** identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
**X⁻** représente un anion de préférence choisi parmi le chlorure, le méthyl sulfate et l'acétate,
**B** représente un groupement choisi par les structures B1 à B6 suivantes : 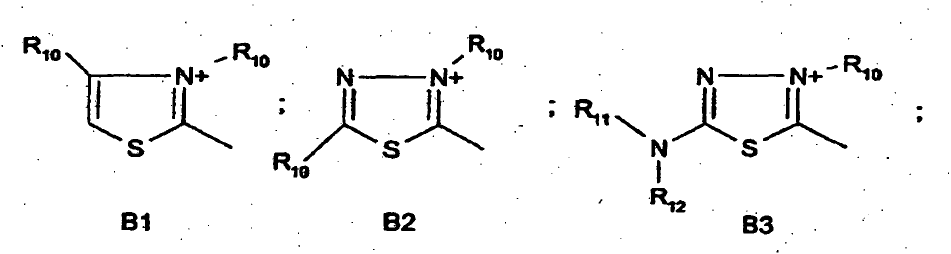 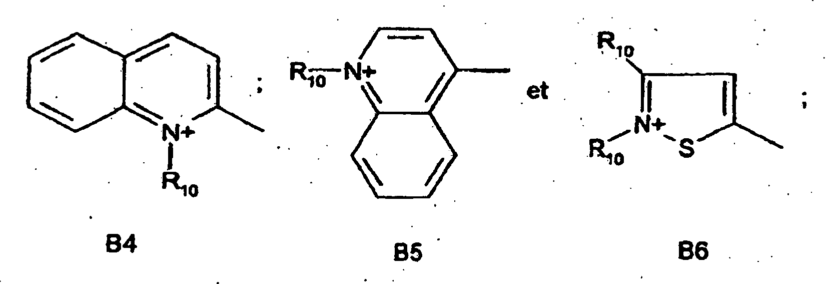 dans lesquelles R₁₀ représente un radical alkyle en C₁-C₄, R₁₁ et R₁₂, identiques ou différents, représentent un atome d'hydrogène ou un radical alkyle en C₁-C₄ ; 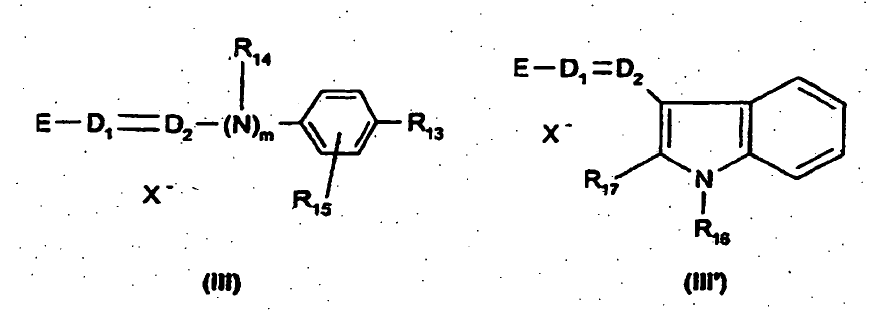
**formules (III) et (III') dans lesquelles :**
**R₁₃** représente un atome d'hydrogène, un radical alcoxy en C₁-C₄, un atome d'halogène tel que le brome, le chlore, l'iode ou le fluor ou un radical amino,
**R₁₄** représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
**R₁₅** représente un atome d'hydrogène ou d'halogène tel que le brome, le chlore, l'iode ou le fluor,
**R₁₆** et **R₁₇**, identiques ou différents, représentent un atome d'hydrogène ou un radical alkyle en C₁-C₄,
**D₁** et **D₂**, identiques ou différents, représentent un atome d'azote ou le groupement -CH,
**m** = 0 ou 1,
étant entendu que lorsque R₁₃ représente un groupement amino non substitué, alors D₁ et D₂ représentent simultanément un groupement -CH et m = 0,
**X⁻** représente un anion de préférence choisi parmi le chlorure, le méthyl sulfate et l'acétate,
**E** représente un groupement choisi par les structures E1 à E8 suivantes : 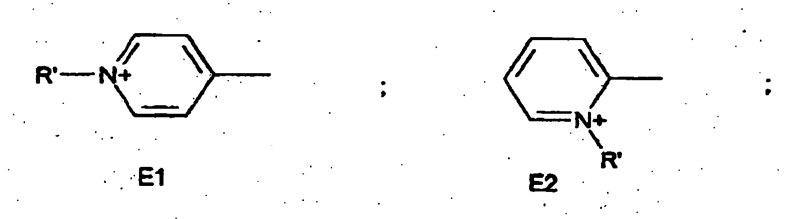 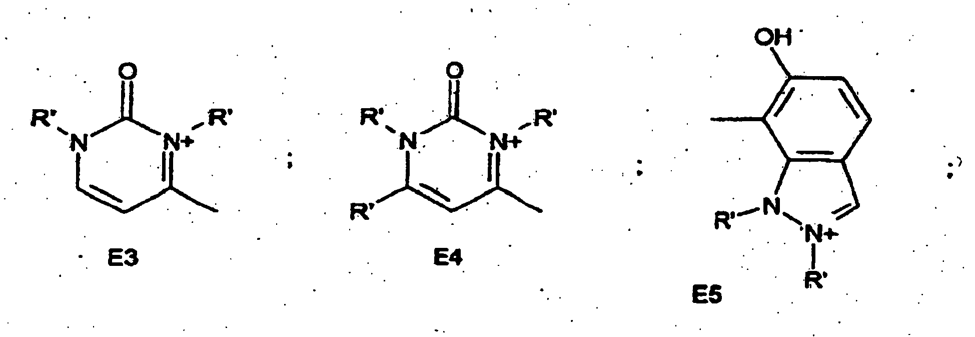 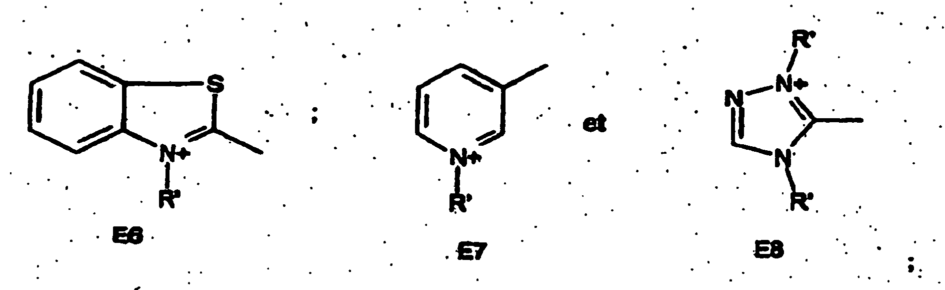 dans lesquelles R' représente un radical alkyle en C₁-C₄;
lorsque m = 0 et que D, représente un atome d'azote, alors E peut également désigner un groupement de structrue E9 suivante : 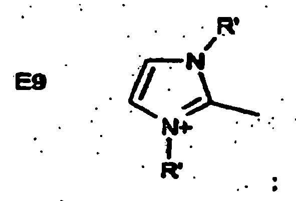
dans laquelle R' représente un radical alkyle en C₁-C₄;
ladite composition étant **caractérisée par le fait qu'**elle contient (ii) au moins un polyol et/ou un éther aliphatique en C₁-C₆ de polyol en C₃-C₉ et/ou un éther aromatique en C₆-C₈ de polyol en C₂-C₉ choisi parmi le 1,3-propanediol, le 2-butène-1,4-diol, le pentane-1,5-diol, le 2,2-diméthyl-propane-1,3 diol, le 3-méthyl-pantane-1,5-diol, le pentane-1,2-diol, le 2,2,4-triméthyl-pentane-1,3-diol, le 2-méthyl-propane-1,3-diol, l'hexylèneglycol, le 1,3-butylèneglycol, le diéthylèneglycol, le triéthylèneglycol, le monoéthyléther de propylèneglycol, le diméthyléther d'isopropylèneglycol, le monométhyléther de dipropylèneglycol, le monométhyléther de tripropylèneglycol, le deméthytether de diéthylèneglycol, le monophényléther d'éthylèneglycol, le monobanzyléther d'éthylèneglycol, le monophényléther de propylèneglycol, le monobenzyléther de propylèneglycol, le monophényléther de diéthylèneglycol et le monobenzyléther de diéthylèneglycol.

2. Composition selon la revendication 1, **caractérisée par le fait que** les colorants directs cationiques de formuls (I) sont choisis parmi les composés répondant aux structures (I1) à (I54) suivantes :

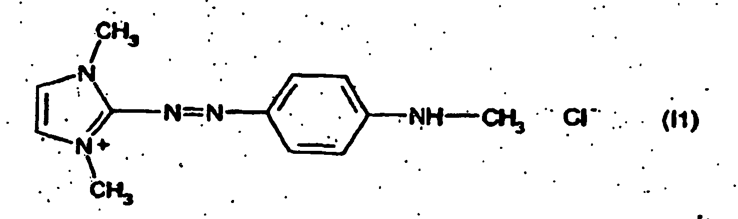

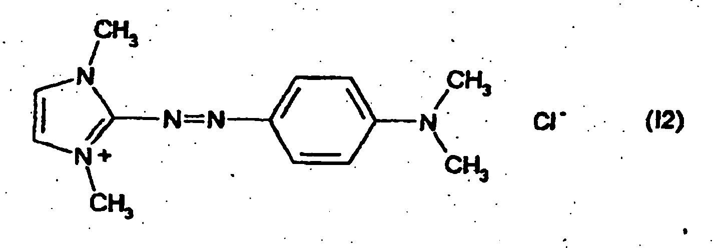

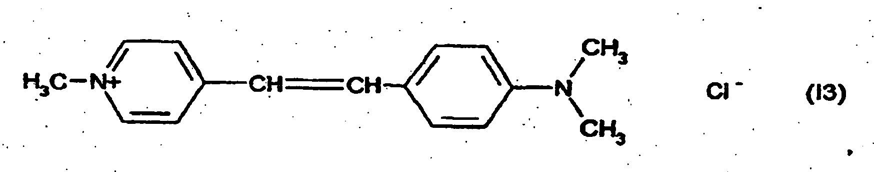

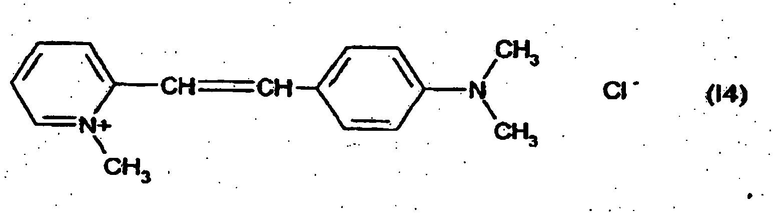

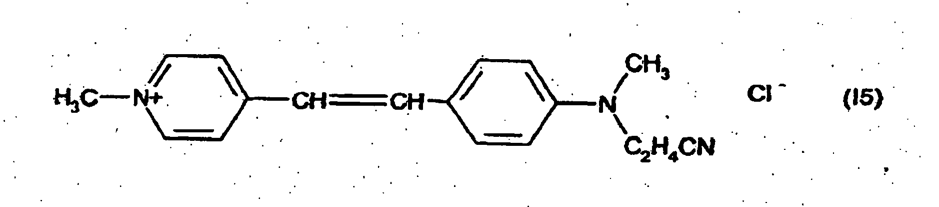

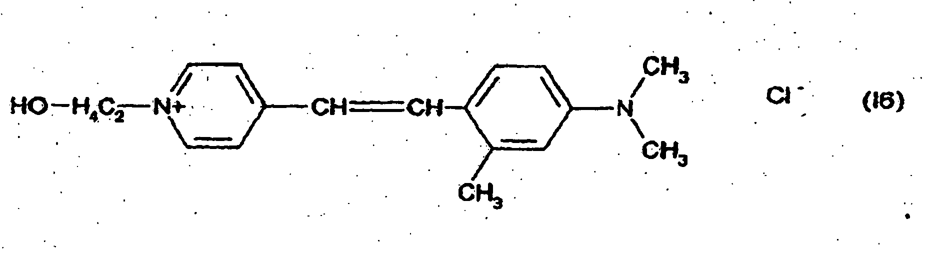

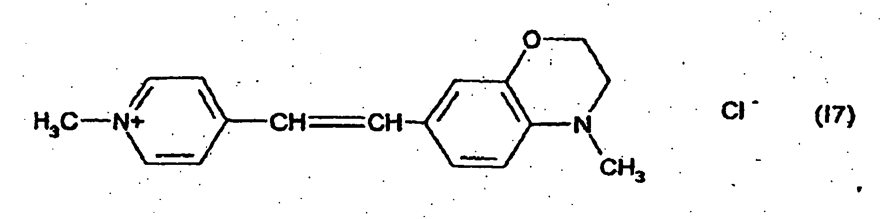

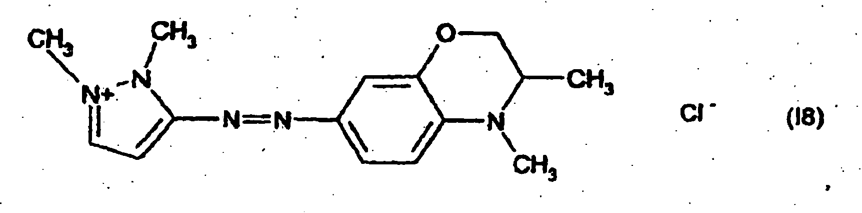

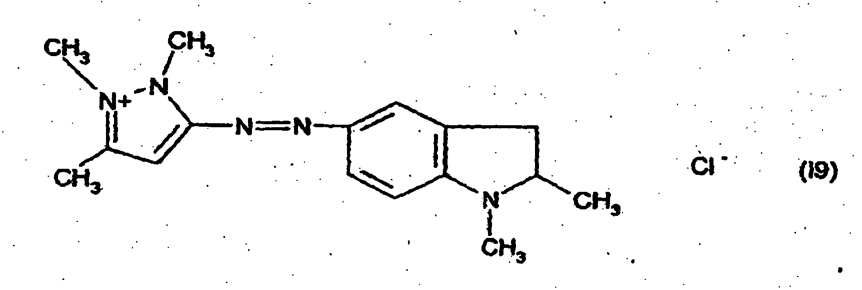

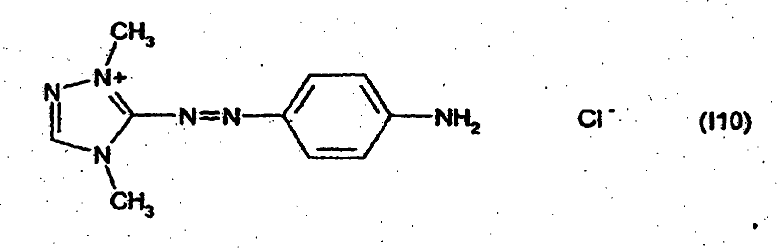

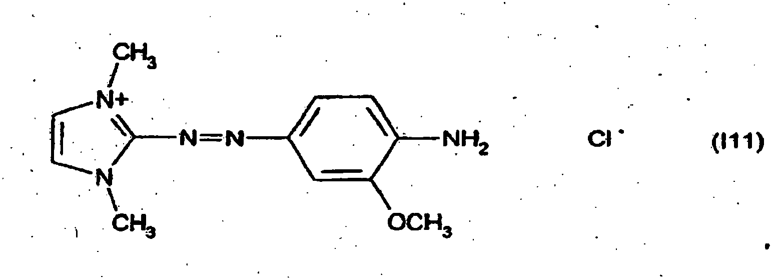

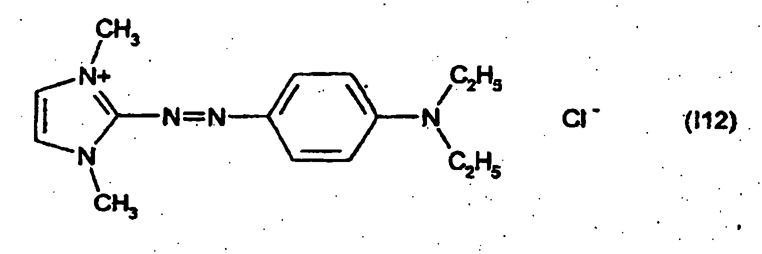

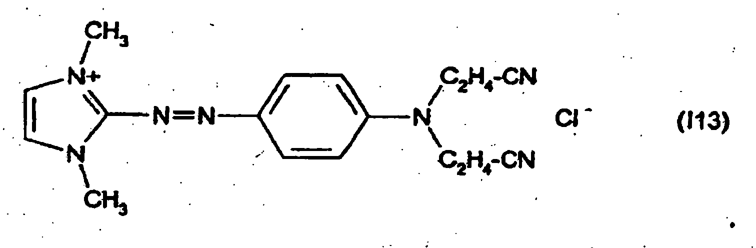

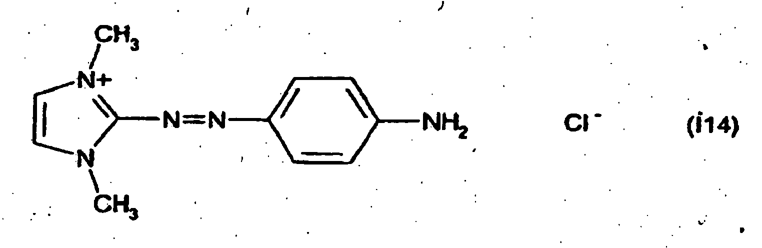

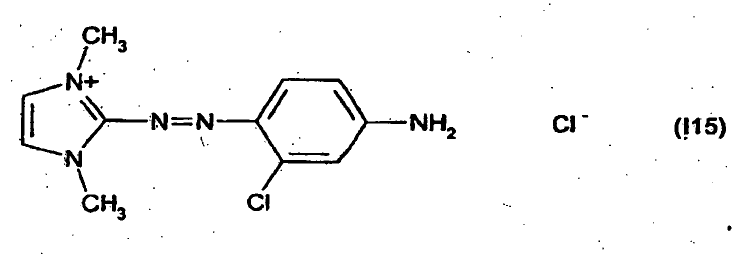

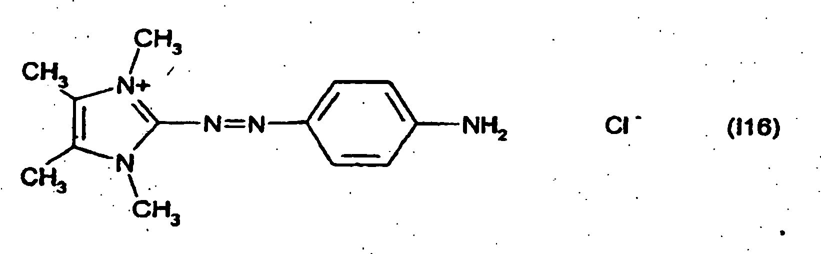

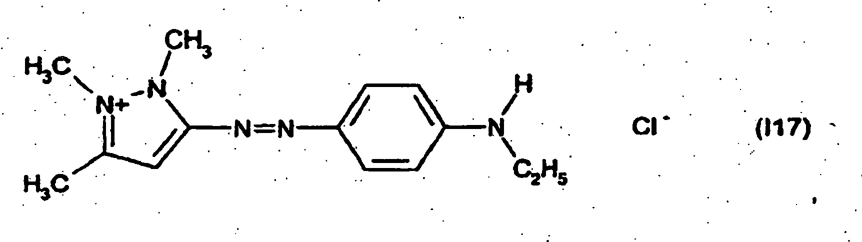

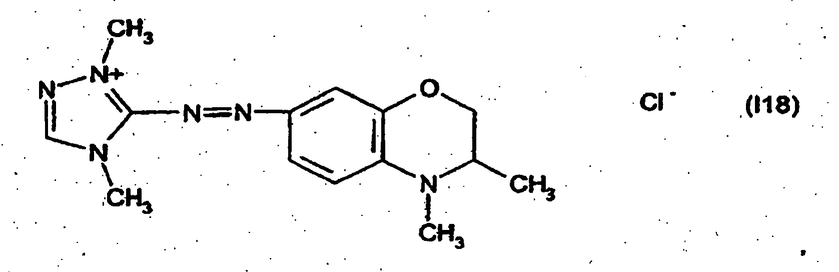

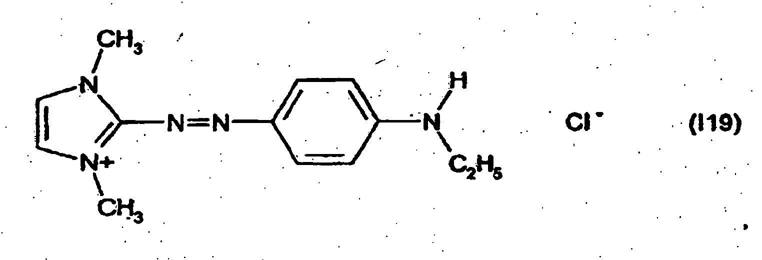

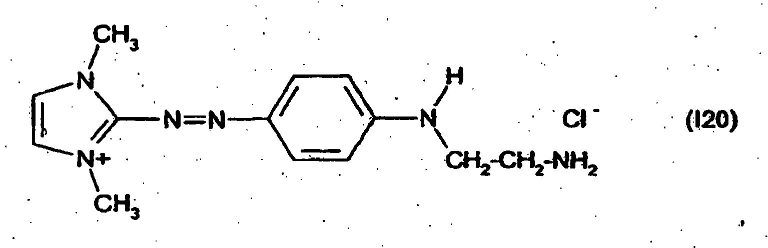

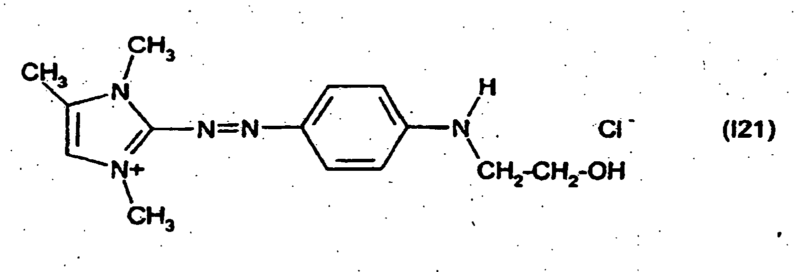

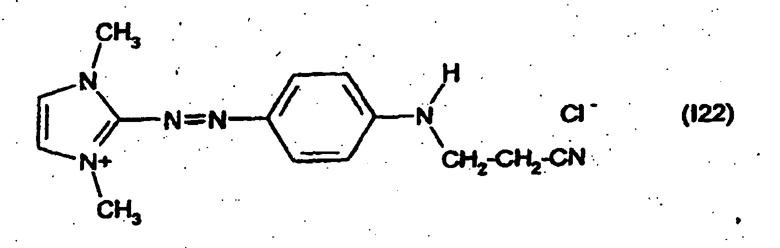

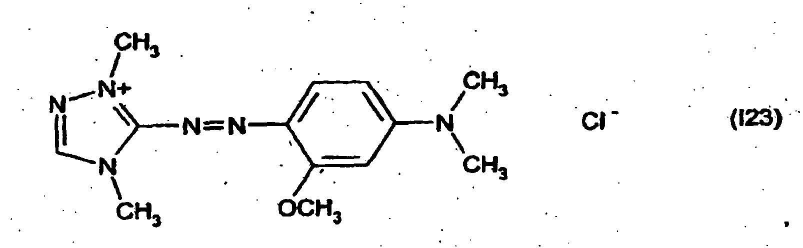

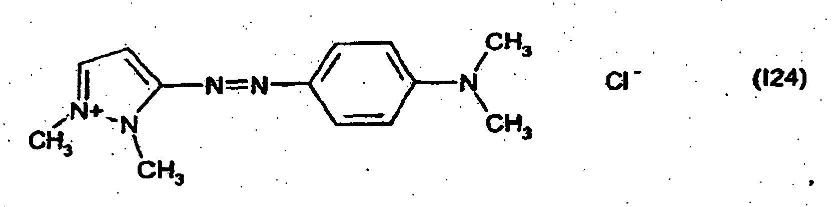

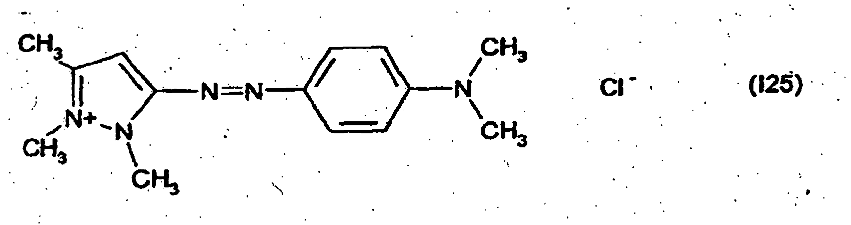

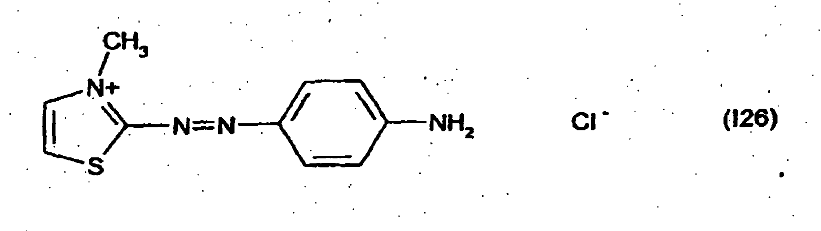

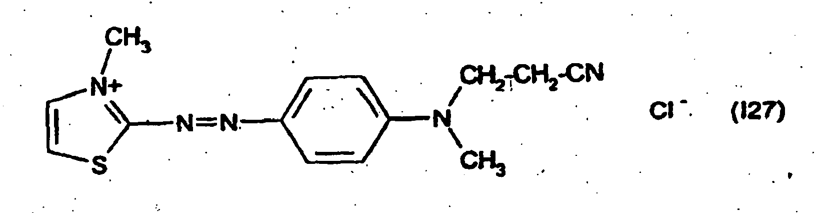

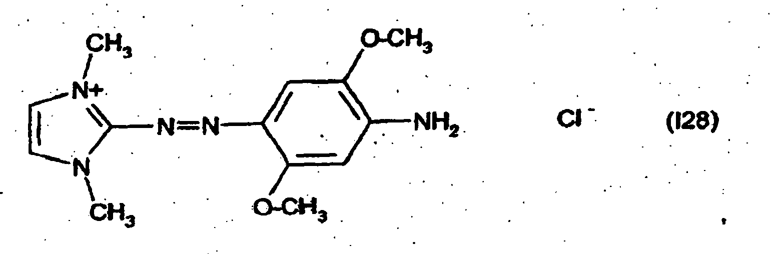

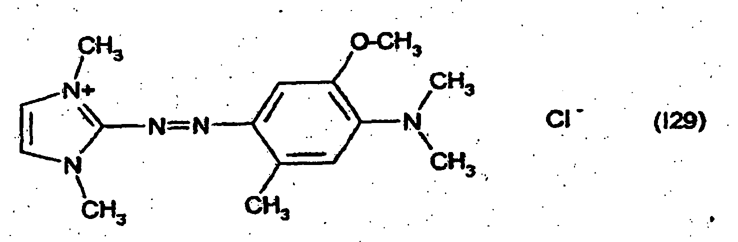

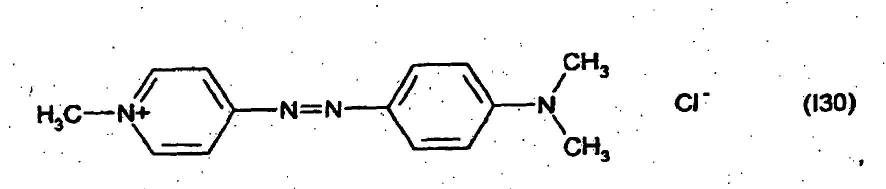

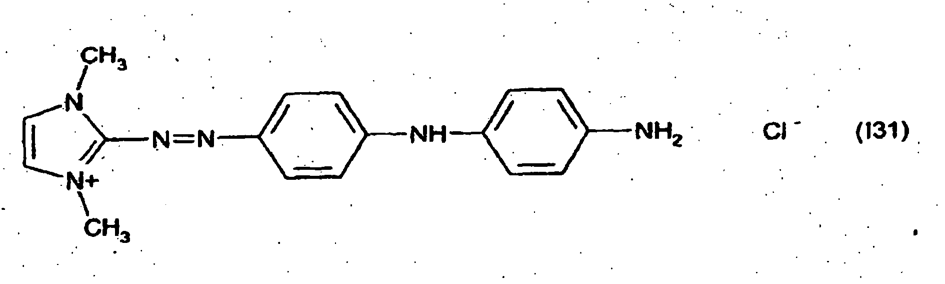

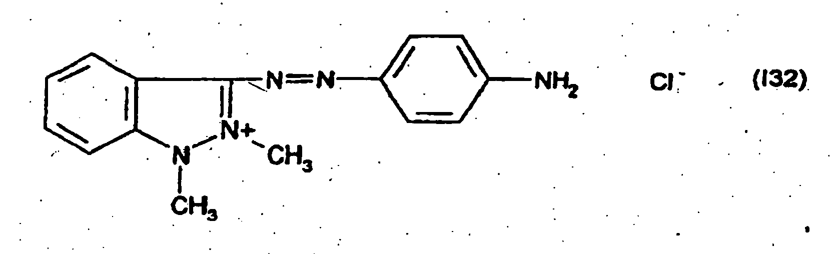

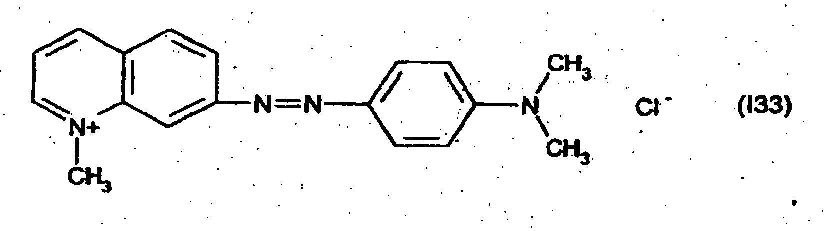

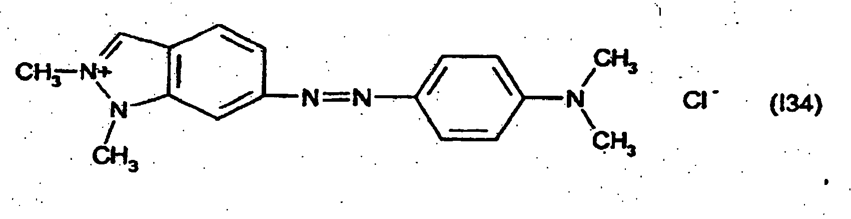

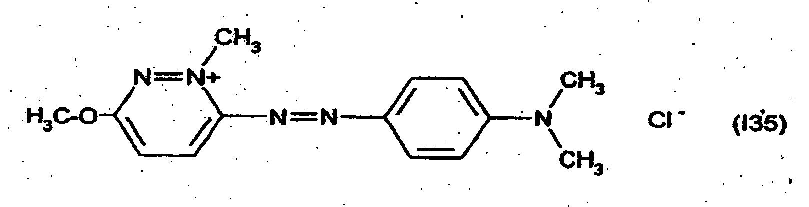

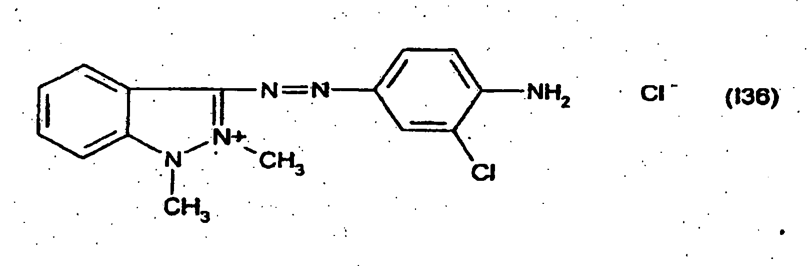

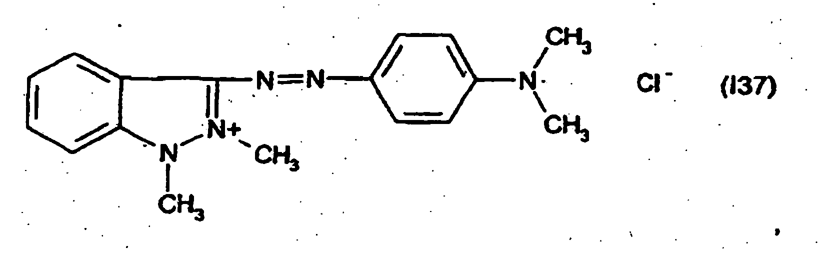

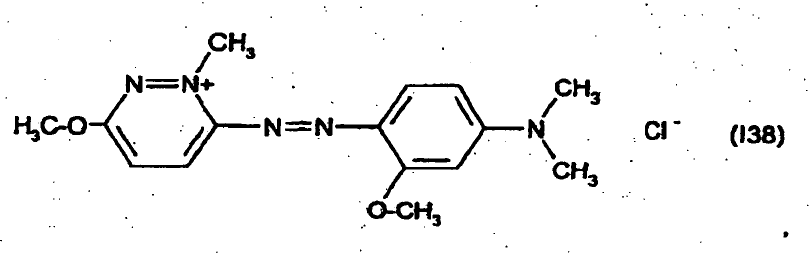

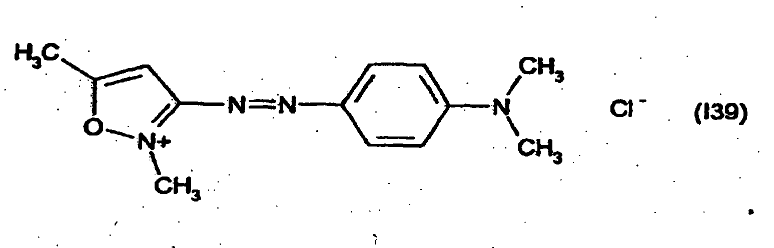

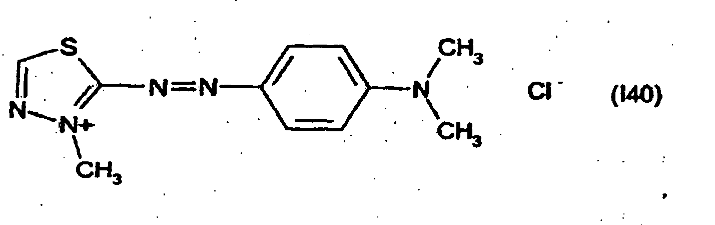

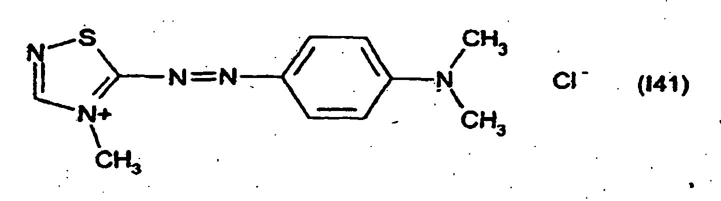

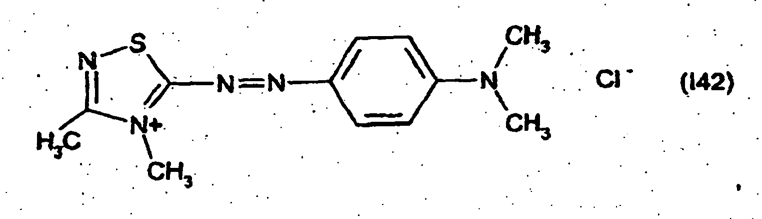

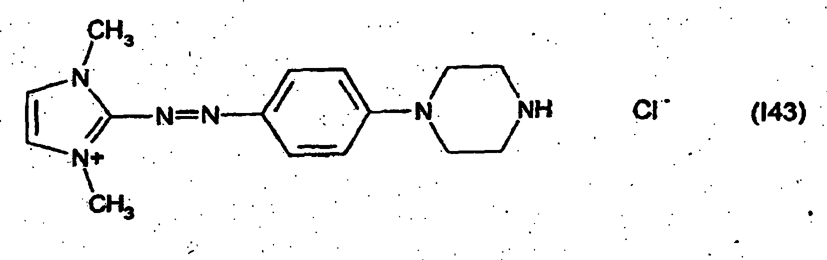

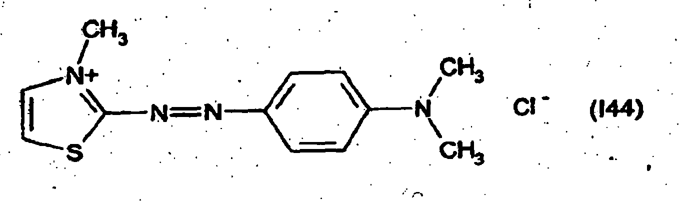

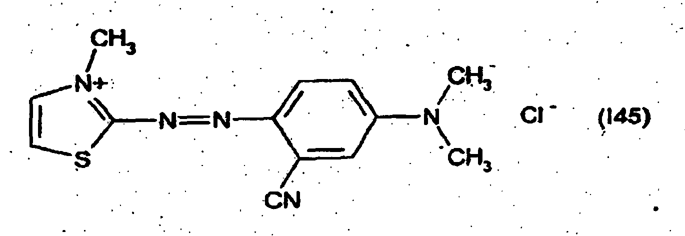

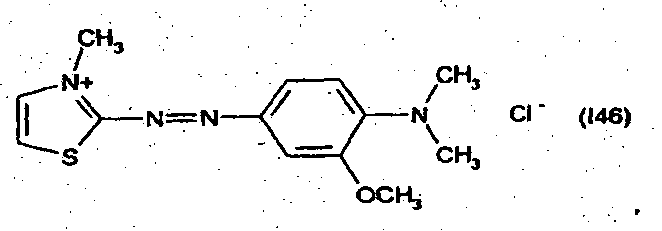

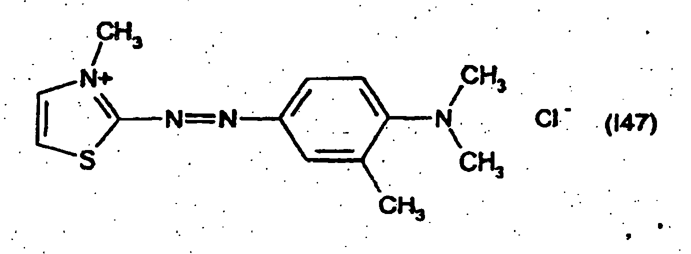

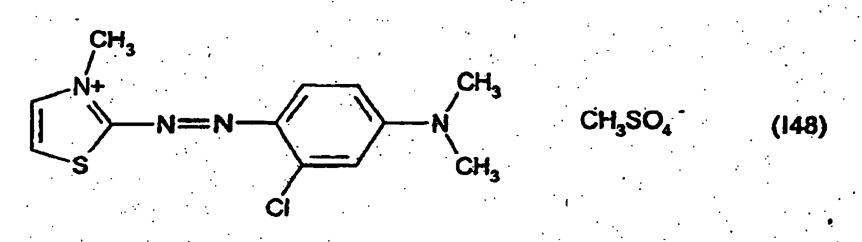

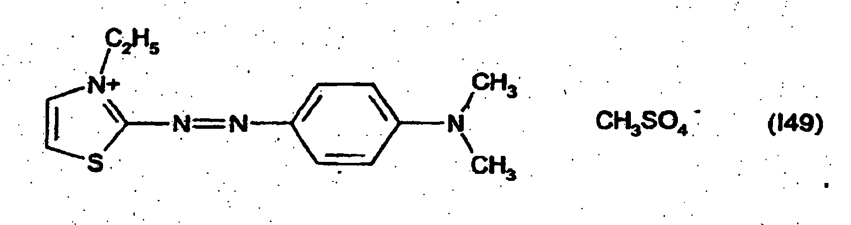

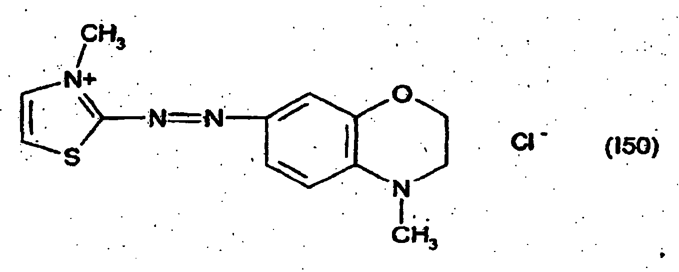

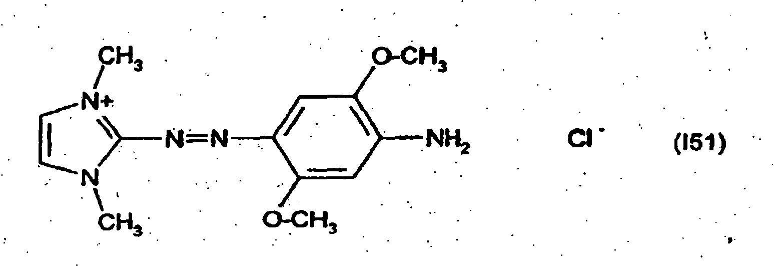

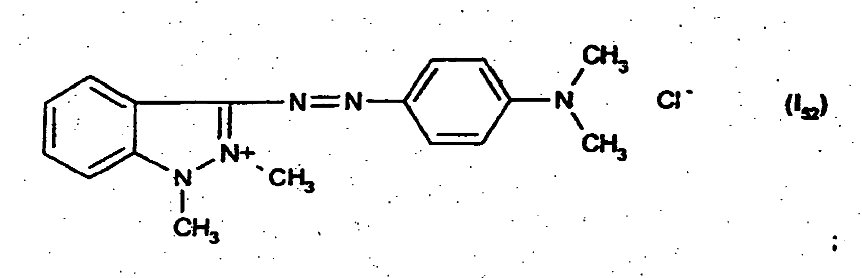

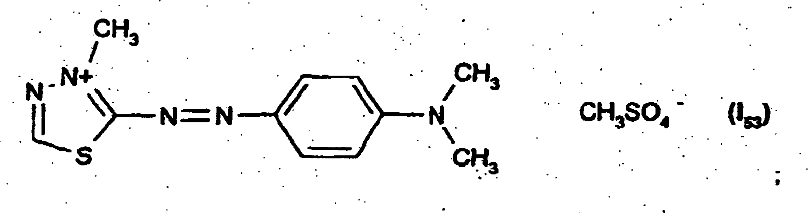

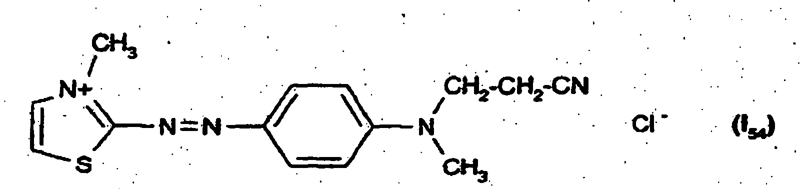

3. Composition selon la revendication 2, **caractérisée par le fait que** les colorants directs cationiques répondent aux structures (I1), (I2), (I14), et (I31).

4. Composition selon la revendication 1, **caractérisée par le fait que** les colorants directs cationiques de formule (II) sont choisis parmi les composés répondant aux structures (II1) à (II9) suivantes : 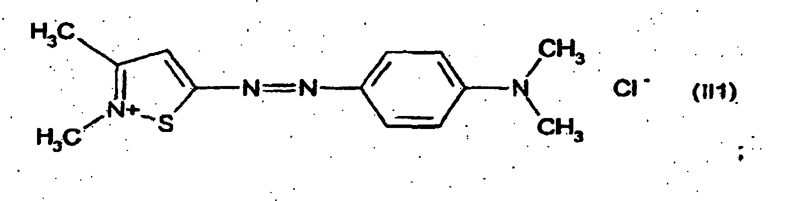 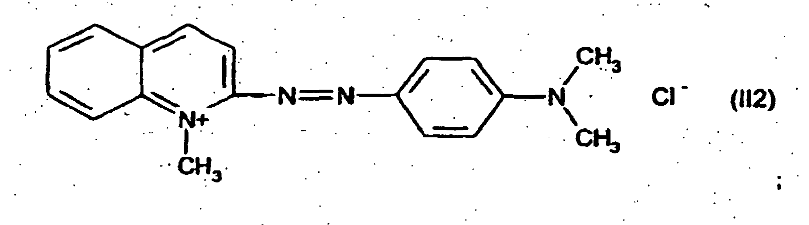 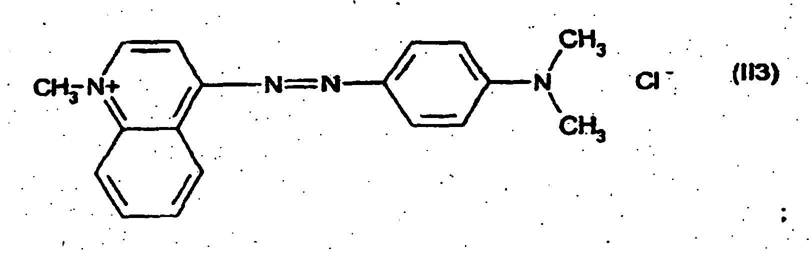 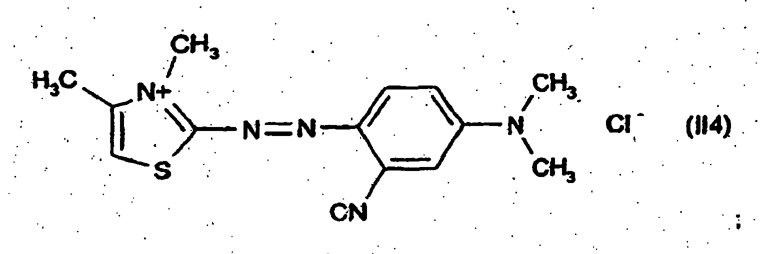 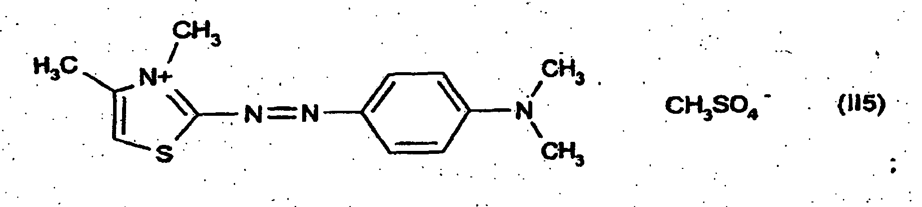 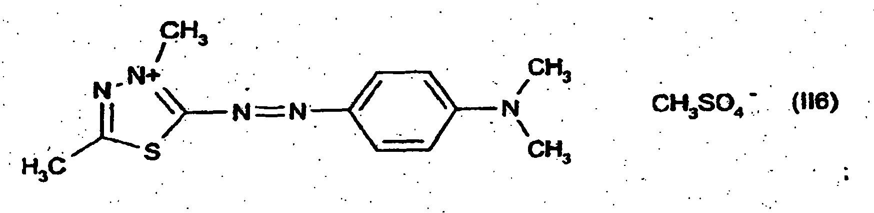 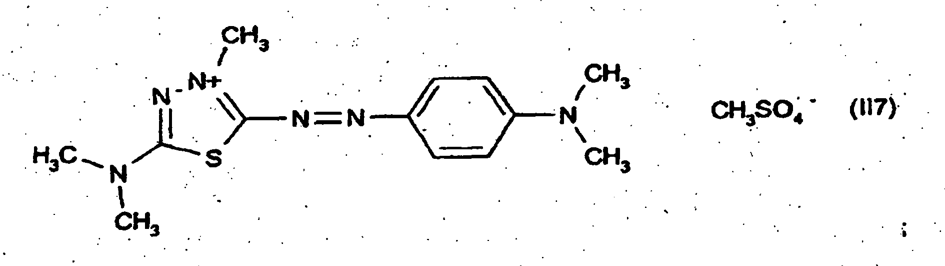 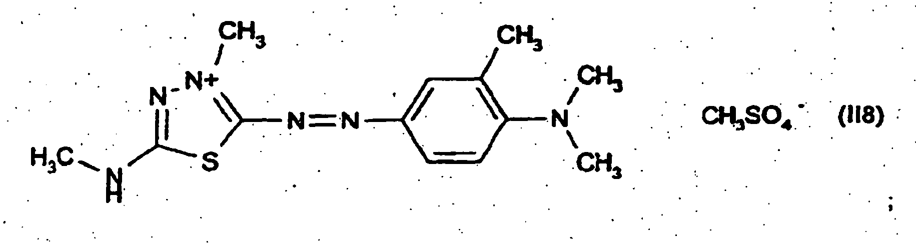 et

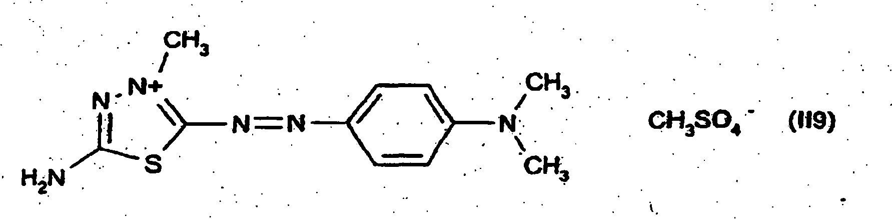

5. Composition selon la revendication 1, **caractérisée par le fait que** les colorants directs cationiques de formule (III) sont choisis parmi les composés répondant aux structures (III1) à (III18) suivantes : 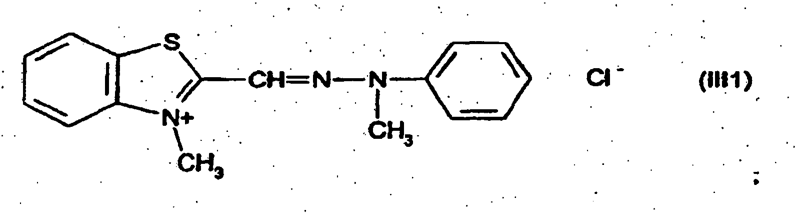 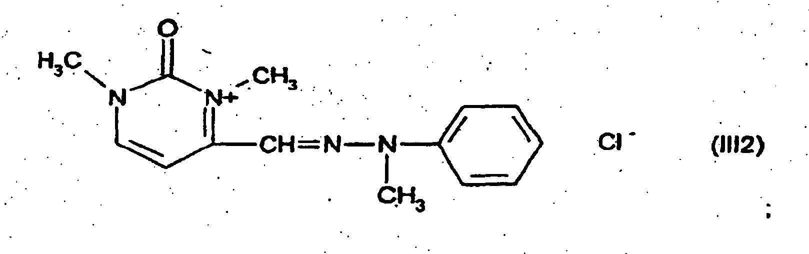 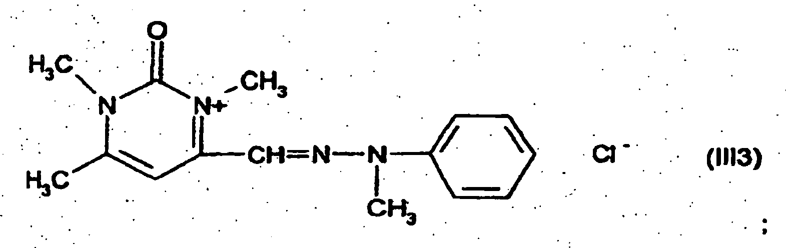 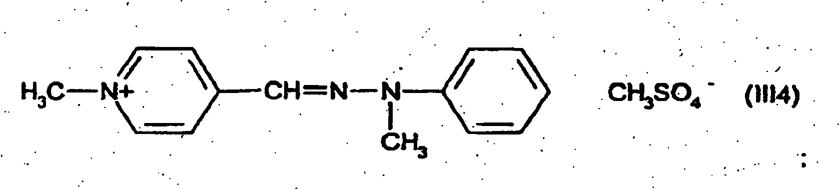 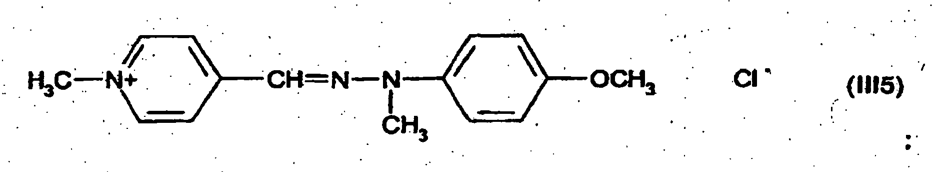 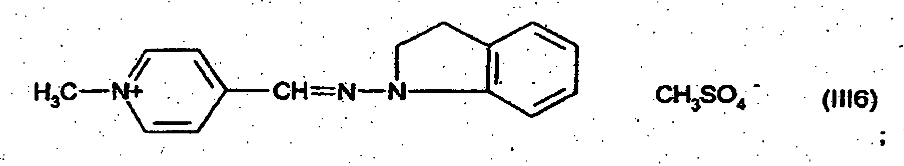 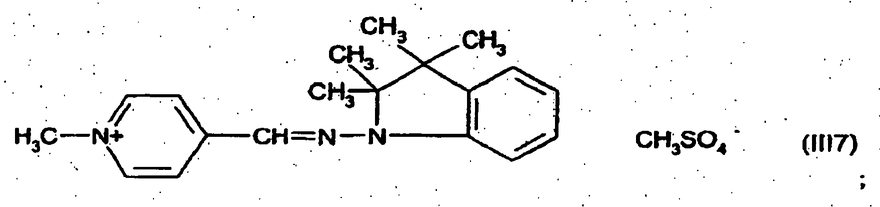 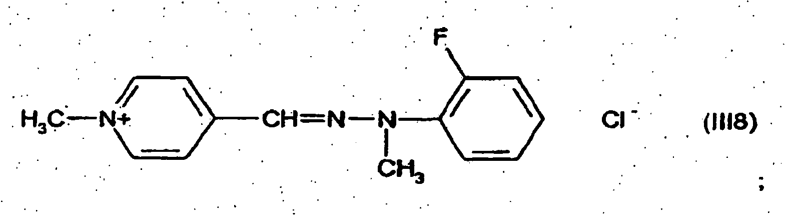 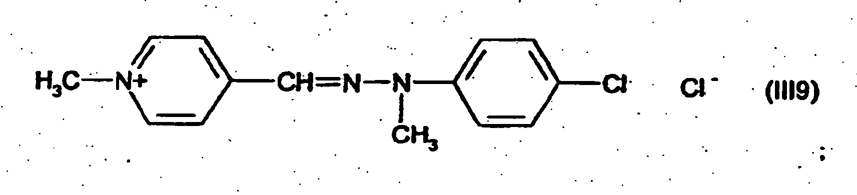 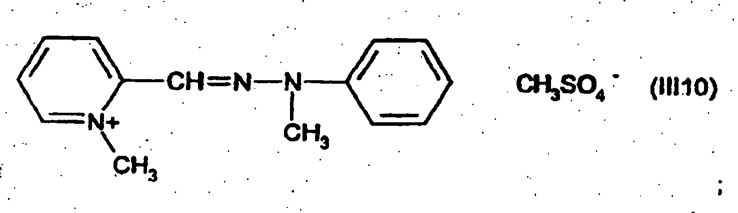 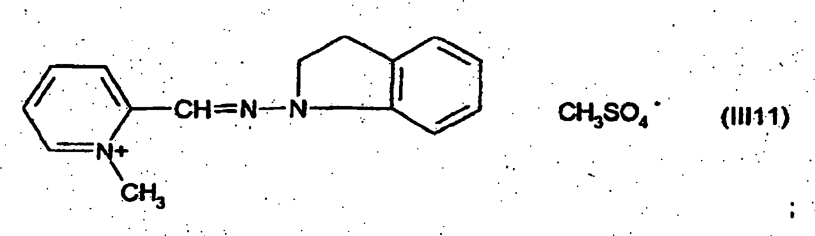 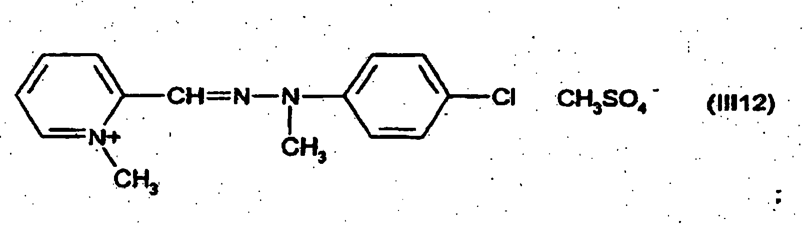 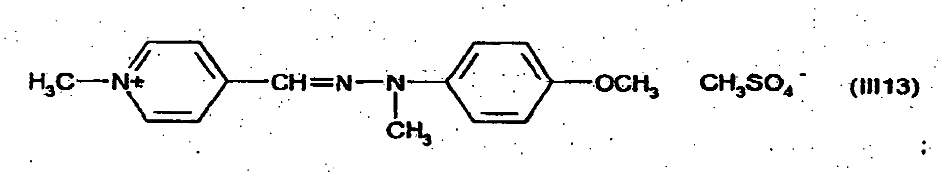 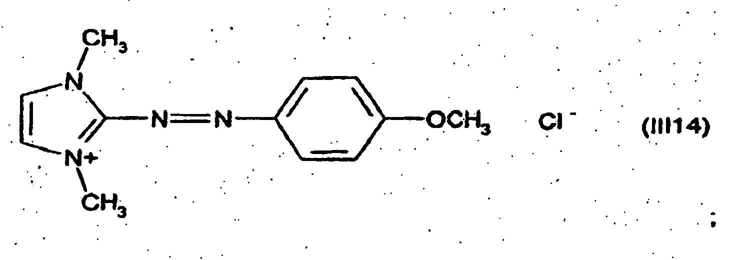 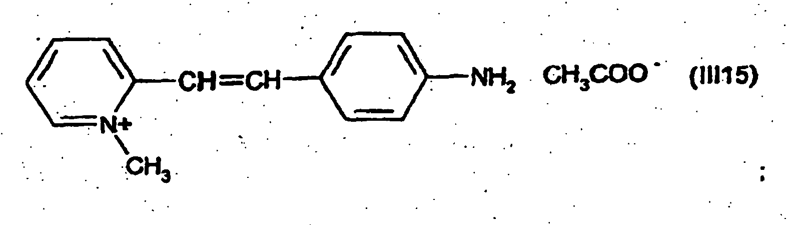 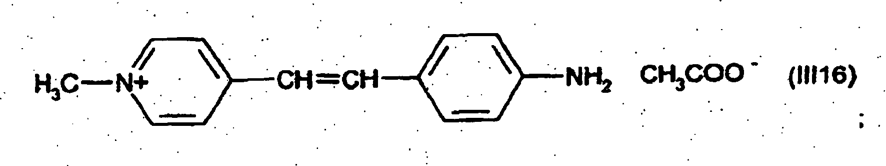 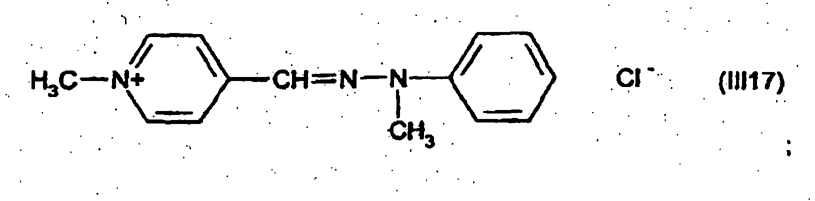 et

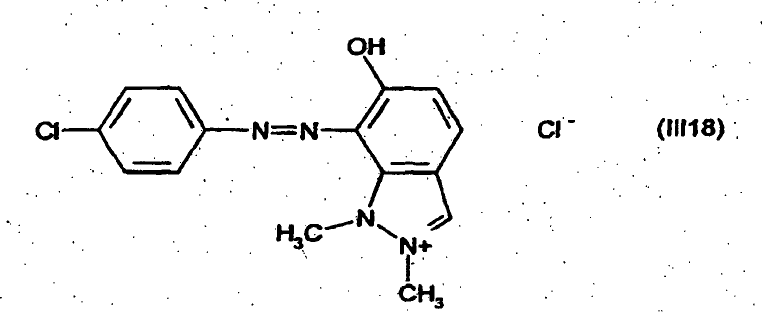

6. Composition selon la revendication 5, **caractérisée par le fait que** les colorants directs cationiques de formule (III) sont choisis parmi les composés répondant aux structures (III4), (III5) et (III13).

7. Composition selon la revendication 1, **caractérisée par le fait que** les colorants directs cationiques de formule (III') sont choisis parmi les composés répondant aux structures (III'1 ) à (I11'3) suivantes : 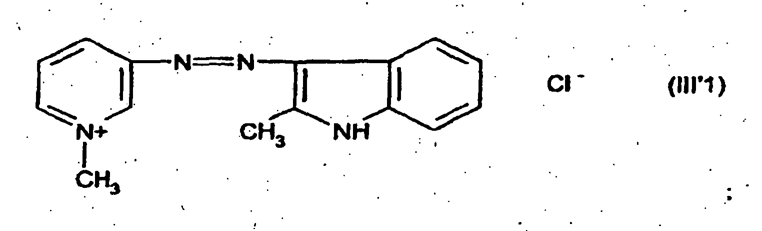 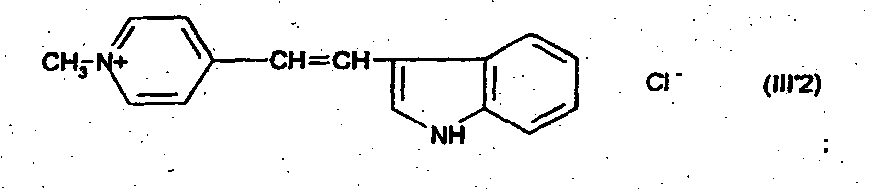 et

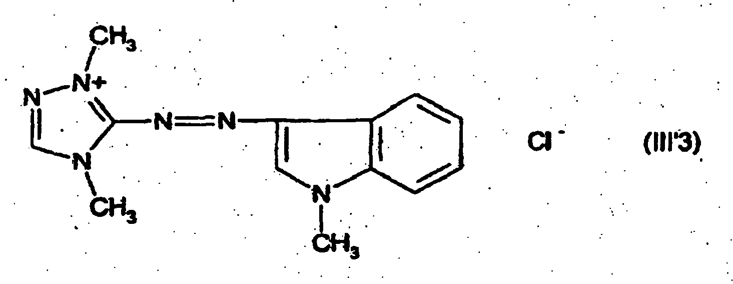

8. Composition selon l'une quelconque des revendications précédentes, **caractérisée par le fait que** le ou les colorants directs cationiques de formules (I), (II), (III) ou (III') représentent de 0,001 à 10 % en poids du poids total de la composition.

9. Composition selon la revendication 8, **caractérisée par le fait que** le ou les colorants directs cationiques de formules (I), (II), (III) ou (III') représentent de 0,005 à 5 % en poids du poids total de la composition.

10. Composition selon l'une quelconque des revendications précédentes, **caractérisée par le fait que** le polyol et/ou l'éther aliphatique en C₁-C₈ de polyol en C₃-C₉ et/ou l'éther aromatique en C₆-C₈ de polyol en C₂-C₉ représentent de 0,1 à 40% en poids du poids total de la composition.

11. Composition selon la revendication 10, **caractérisée par le fait que** le polyol et/ou l'éther aliphatique en C₁-C₈ de polyol en C₃-C₉ et/ou l'éther aromatique en C₆-C₈ de polyol en C₂-C₉ représentent de 0,5 à 20% en poids du poids total de la composition.

12. Composition selon l'une quelconque des revendications précédentes, **caractérisée par le fait qu'**elle contient en outre des colorants directs additionnels.

13. Composition selon l'une quelconque des revendications précédentes, **caractérisée par le fait que** le milieu approprié, pour les fibres kératiniques (ou support) est constitué par un mélange d'eau et d'au moins un solvant choisi parmi les polyols et/ou les éthers de polyols définis dans la revendication 1.

14. Composition selon la revendication 13, **caractérisée par le fait que** le milieu comprend en outre au moins un solvant organique.

15. Composition selon l'une quelconque des revendications précédentes, **caractérisée par le fait qu'**elle présente un pH compris entre 2 et 11, et de préférence entre 5 et 10.

16. Composition selon l'une quelconque des revendications précédentes, **caractérisée par le fait qu'**elle est destinée à la teinture directe éclaircissante et qu'elle renferme au moins un agent oxydant.

17. Procédé de teinture directe des fibres kératiniques et en particulier des fibres kératiniques humaines telles que les cheveux, **caractérisé par le fait que** qu'on applique sur les fibres au moins une composition tinctoriale comprenant, dans un milieu approprié pour la teinture exempt d'oxydases ou d'oxydoréductases, (i) au moins un colorant direct cationique de formules suivantes (I), (II), (III), (III') : 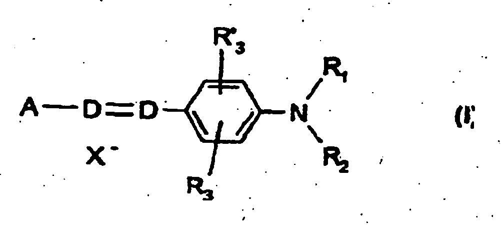 **formule (I) dans laquelle :**
D représente un atome d'azote ou le groupement -CH,
**R₁** et **R₂,** identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
**R₃** et **R'₃**, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
**X⁻** représente un anion de préférence choisi parmi le chlorure, le méthyl sulfate et l'acétate,
**A** représente un groupement choisi par les structures A1 à A18 suivantes : 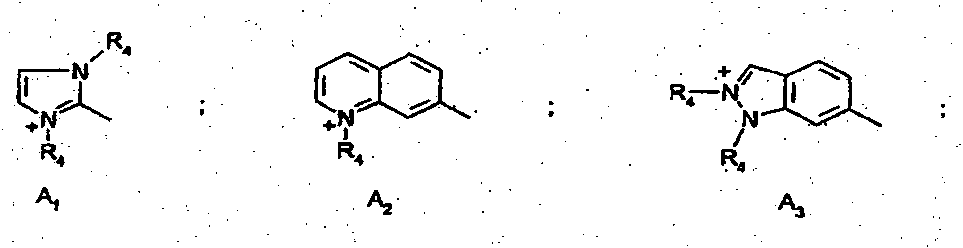 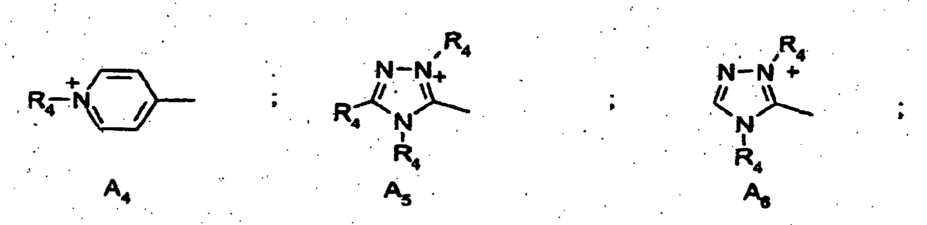 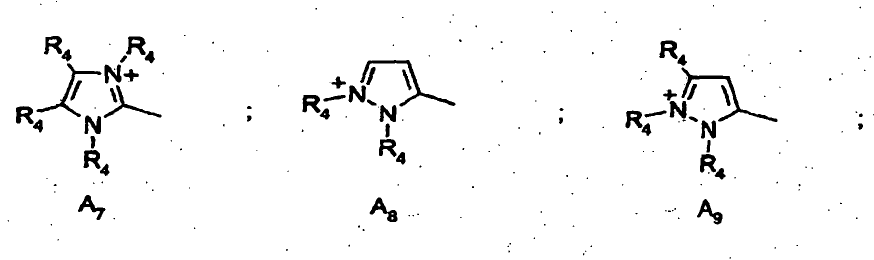 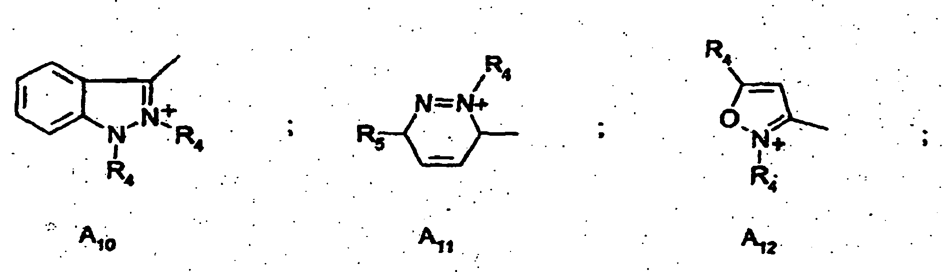 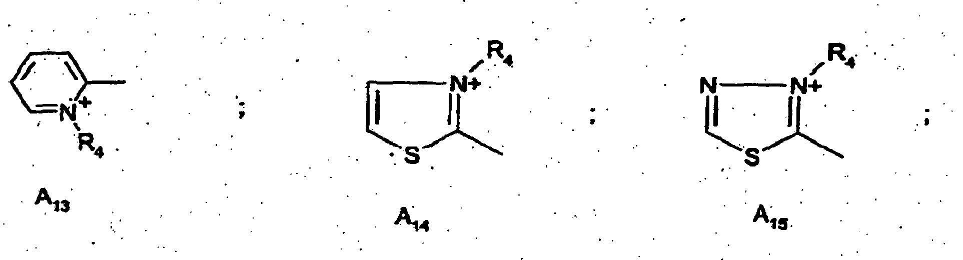 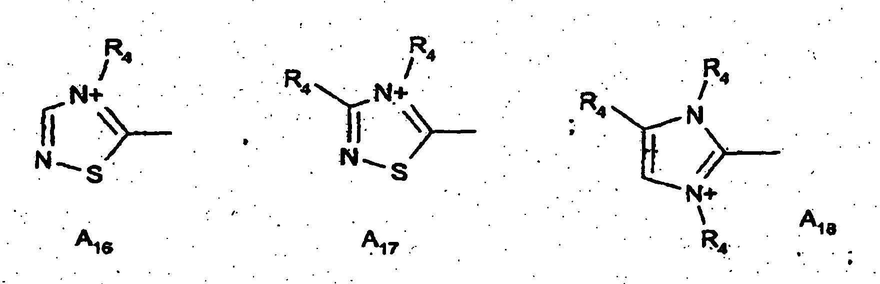 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ; 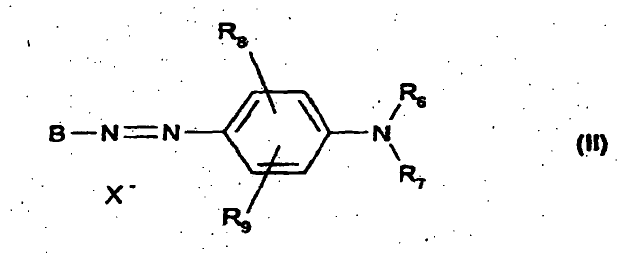
**formule (II) dans laquelle :**
**R₆** représente un atome d'hydrogène ou un radical alkyle en C₁-C₄,
**R₇** représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical-alkyle en C₁-C₄.
**R₆** et **R₉,** identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
**X**⁻ représente un anion de préférence choisi parmi le chlorure, le méthyl sulfate et l'acétate,
**B** représente un groupement choisi par les structures B1 à B6 suivantes : 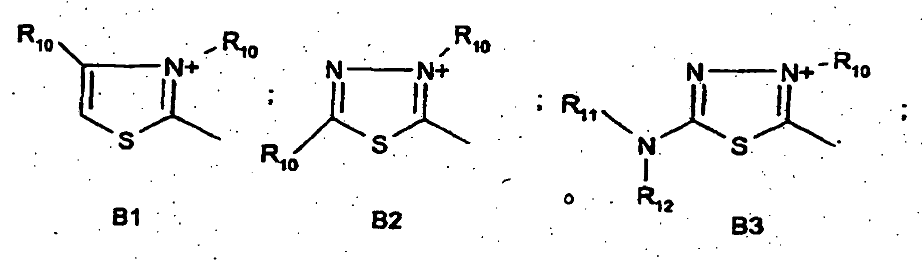 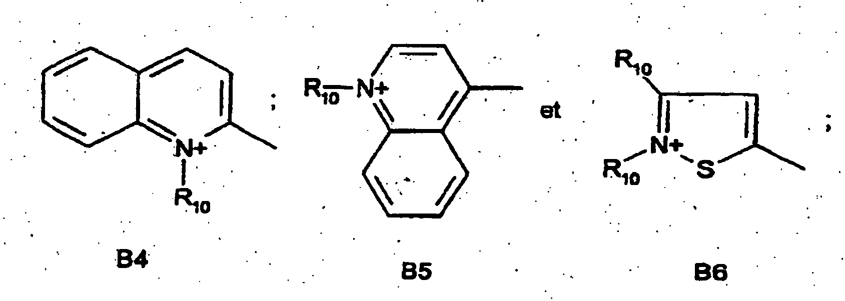 dans lesquelles R₁₀ représente un radical alkyle en C₁-C₄, R₁₁, et R₁₂, identiques ou différents, représentent un atome d'hydrogène ou un radical alkyle en C₁-C₄ ; 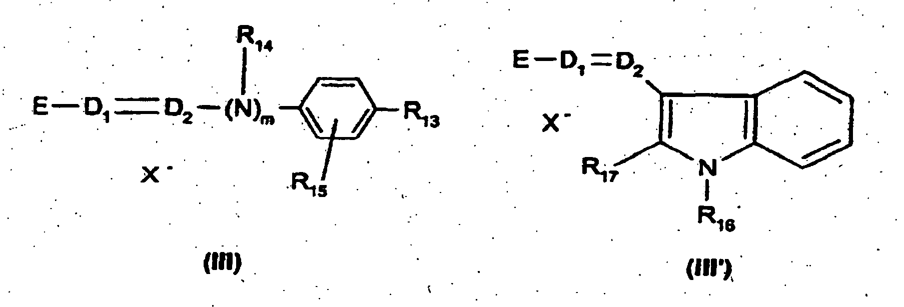
**formules (III) et (III') dans desquelles :**
**R₁₃** représente un atome d'hydrogène, un radical alcoxy en C₁-C₄, un atome d'halogène tel que le brome, le chlore, l'iode ou le fluor ou un radical amino,
**R₁₄** représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
**R₁₅** représente un atome d'hydrogène ou d'halogène tel que le brome, le chlore, l'iode ou le fluor,
**R₁₆** et **R₁₇,** identiques ou différents, représentent un atome d'hydrogène ou un radical alkyle en C₁-C₄,
**D₁** et **D₂,** identiques ou différents, représentent un atome d'azote ou le groupement -CH, m = 0 ou 1,
étant entendu que lorsque R₁₃ représente un groupement amino non substitué, alors D₁ et
**D₂** représentent simultanément un groupement -CH et m = 0,
**X⁻** représente un anion de préférence choisi parmi le chlorure, le méthyl sulfate et l'acétate,
**E** représente un groupement choisi par les structures E1 à E8 suivantes : 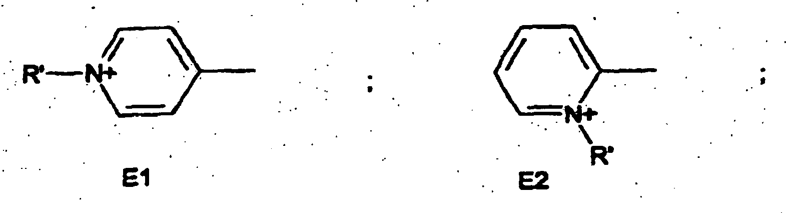 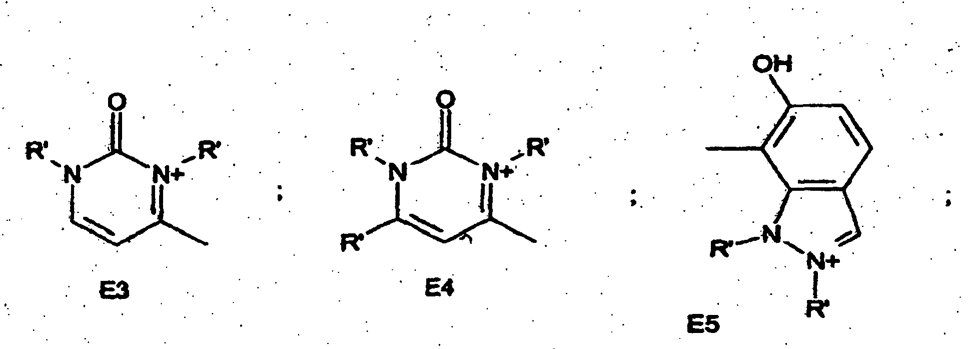 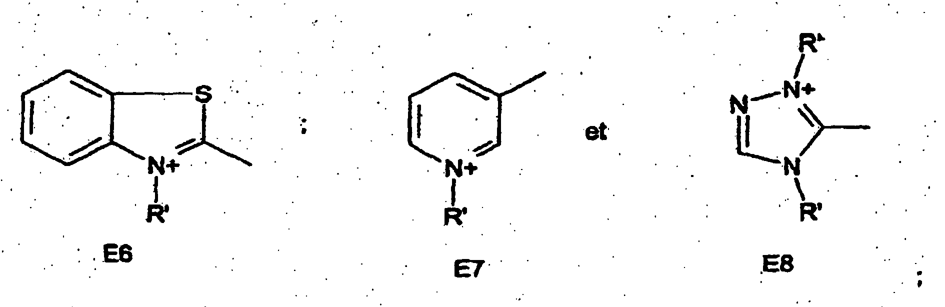 dans lesquelles R' représente un radical alkyle en C₁-C₄ ;
lorsque m = 0 et que D₁ représente un atome d'azote, alors E peut également désigner un groupement de structure E9 suivante : 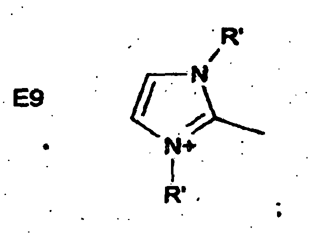 dans laquelle R' représente un radical alkyle en C₁-C₄ ;
ladite composition étant **caractérisée par le fait qu'**elle contient (ii) au moins un polyol et/ou un éther aliphatique en C₁-C₈ de polyol en C₃-C₉ et/ou un éther aromatique en C₆-C₈ de polyol en C₂-C₉, et telle que définie à l'une quelconque des revendications 2 à 16, pendant un temps suffisant pour développer la coloration désirée, après quoi on rince, on lave éventuellement au shampooing, on rince à nouveau et on sèche.

18. Procédé de teinture directe des fibres kératiniques et en particulier des fibres kératiniques humaines telles que les cheveux, **caractérisé par le fait que** qu'on applique sur les fibres au moins une composition tinctoriale comprennant, dans un milieu approprié pour la teinture exempt d'oxydases ou d'oxydoréductases, (i) au moins un colorant direct cationique de formules suivantes (I), (II), (III), (III') : 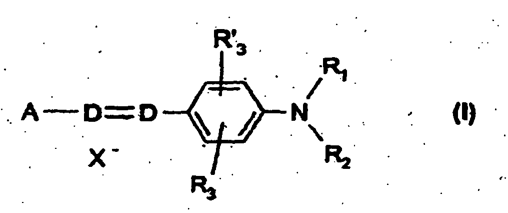 **formule (I) dans laquelle :**
D représente un atome d'azote ou le groupement -CH,
**R₁** et **R₂,** identiques ou différents, représentent un atome d'hydrogène ; un radical alkyle en
C₁-C₄ pouvant être substitué par un radical -CN, -OH ou -NH₂ ou forment avec un atome de carbone du cycle benzénique un hétérocycle éventuellement oxygéné ou azoté. pouvant être substitué par un ou plusieurs radicaux alkyle en C₁-C₄ ; un radical 4'-aminophényle,
**R₃** et **R'₃,** identiques ou différents, représentent un atome d'hydrogène ou d'halogène choisi parmi le chlore, le brome, l'iode et le fluor, un radical cyano, alkyl en C₁-C₄ alcoxy en C₁-C₄ ou acétyloxy,
**X⁻** représente un anion de préférence choisi parmi le chlorure, le méthyl sulfate et l'acétate,
**A** représente un groupement choisi par les structures A1 à A18 suivantes : 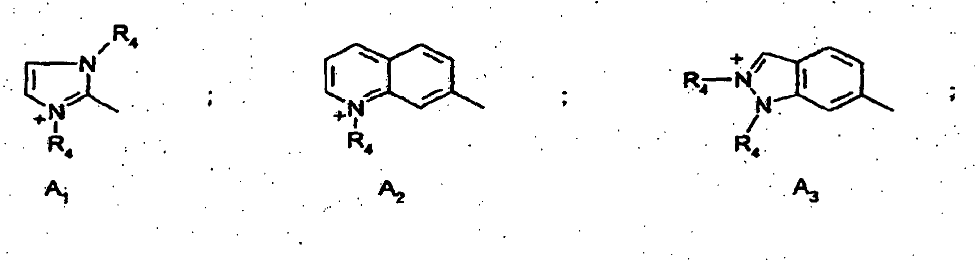 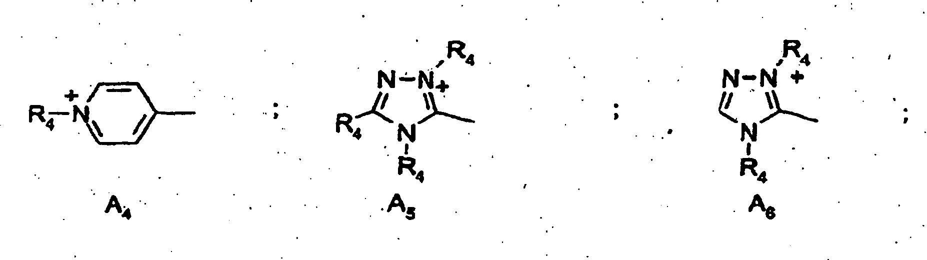 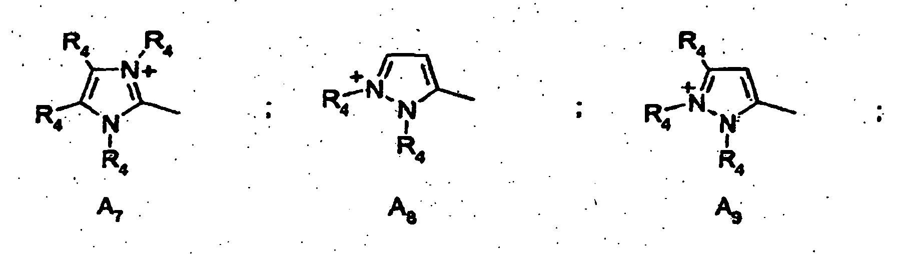 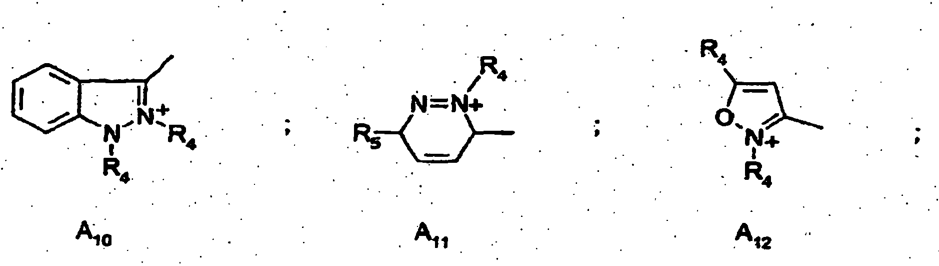 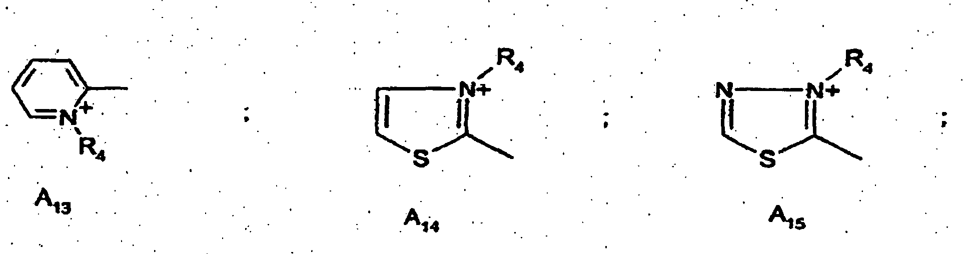 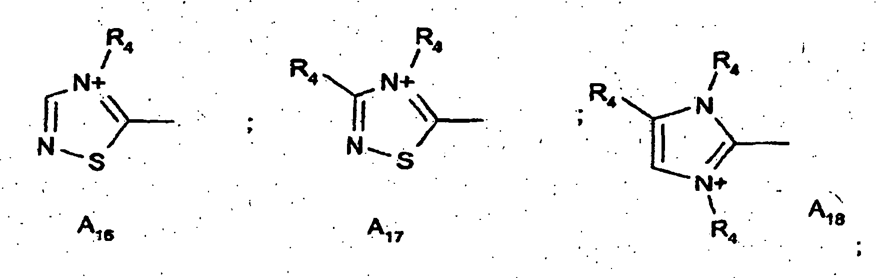 dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ; 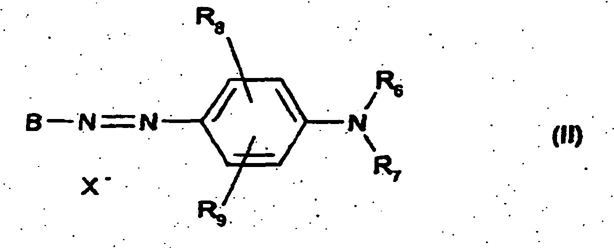
**formule (II) dans laquelle :**
**R₆** représente un atome d'hydrogène ou un radical alkyle en C₁-C₄,
**R₇** représente un atome d'hydrogène, un radical alkyle pouvant être substitué par un radical -CN ou par un groupement amino, un radical 4'-aminophényle ou forme avec **R₆** un hétérocycle éventuellement oxygéné et/ou azoté pouvant être substitué par un radical alkyle en C₁-C₄,
**R₈** et **R₉,** identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
**X⁻** représente un anion de préférence choisi parmi le chlorure, le méthyl sulfate et l'acétate,
B représente un groupement choisi par les structures B1 à B6 suivantes : 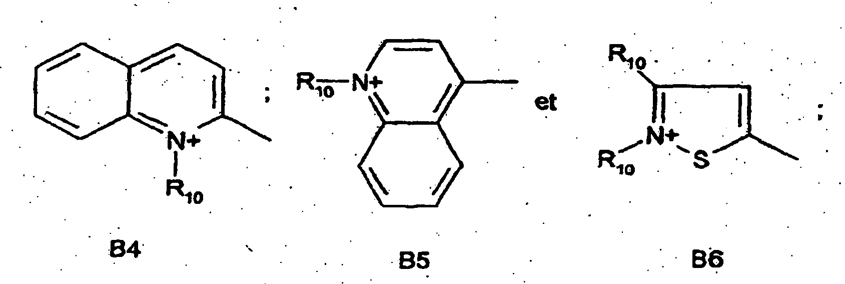 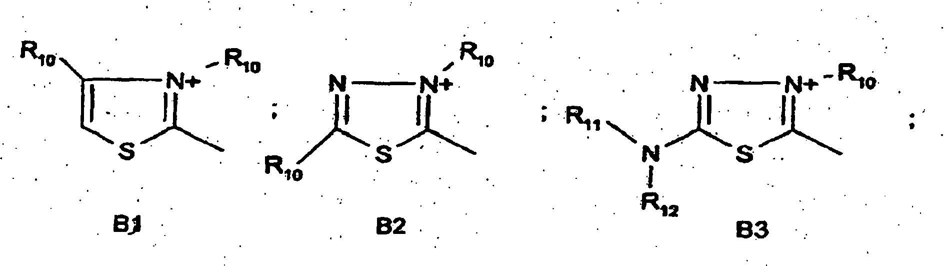 dans lesquelles R₁₀ représente un radical alkyle en C₁-C₄, R₁₁ et R₁₂, identiques ou différents, représentent un atome d'hydrogène ou un radical alkyle en C₁-C₄ ; 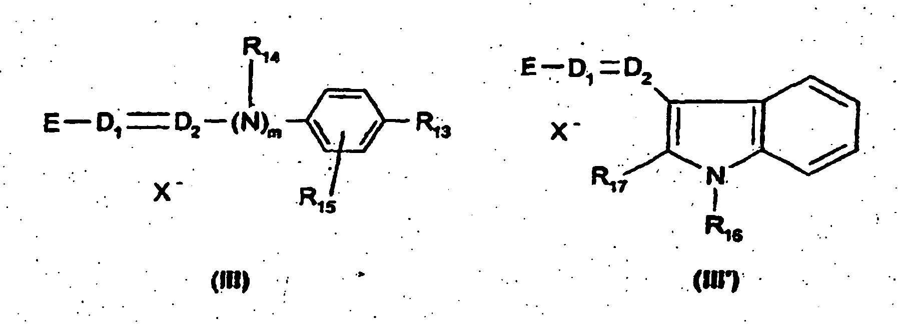
**formules (III) et (III') dans lesquelles :**
**R₁₃** représente un atome d'hydrogène, un radical alcoxy en C₁-C₄, un atome d'halogène tel que le brome, le chlore, l'iode ou le fluor ou un radical amino,
**R₁₄** représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
**R₁₅** représente un atome d'hydrogène ou d'halogène tel que le brome,. le chlore, l'iode ou le fluor,
**R₁₆** et **R₁₇,** identiques ou différents, représentent un atome d'hydrogène ou un radical alkyle en C₁-C₄,
**D₁** et **D₂**, identiques ou différents, représentent un atome d'azote ou le groupement -CH, m=0 ou 1,
étant entendu que lorsque **R₁₃** représente un groupement amino non substitué, alors D₁ et
D₂ représentent simultanément un groupement -CH et m = 0,
**X⁻** représente un anion de préférence choisi parmi le chlorure, le méthyl sulfate et l'acétate,
**E** représente un groupement choisi par les structures E1 à E8 suivantes : 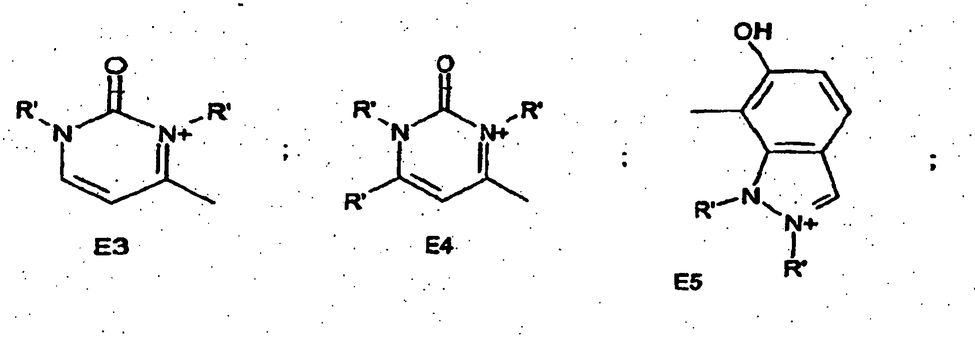 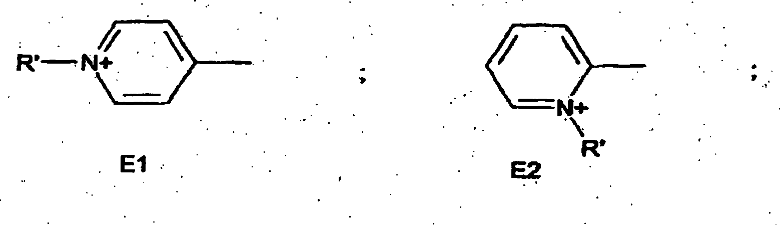 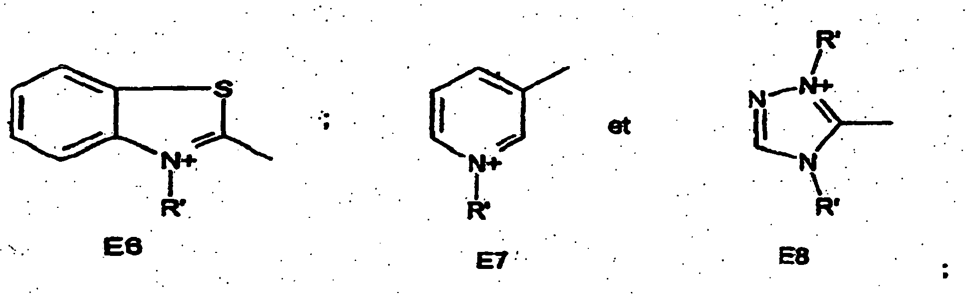 dans lesquelles R' représente un radical alkyle en C₁-C₄ :
lorsque m = 0 et que D₁ représente un atome d'azote, alors E peut également désigner un groupement de structure E9 suivante : 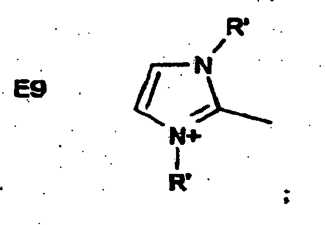 dans laquelle R' représente un radical alkyle en C₁-C₄ ;
ladite composition étant **caractérisée par le fait qu'**elle contient (ii) au moins un polyol et/ou un éther aliphatique en C₁-C₈ de polyol en C₃-C₉ et/ou un éther aromatique en C₆-C₈ de polyol en C₂-C₉, et telle que définie à l'une quelconque des revendications 1 à 16, pendant un temps suffisant pour développer la coloration désirée, sans rinçage final.

19. Procédé de teinture directe des fibres kératiniques et en particulier des fibres kératiniques humaines telles que les cheveux, selon l'une des revendications 17 ou 18, **caractérisé par le fait qu'**il comporte une étape préliminaire consistant à stocker sous forme séparée, d'une part, une composition (A) comprenant, dans un milieu approprié pour la teinture, au moins un colorant direct cationique tel que défini dans les revendications précédentes et, d'autre part, une composition (B) renfermant, dans un milieu approprié pour la teinture, au moins un agent oxydant différant des oxydases et/ou des oxydoréductases, puis à procéder à leur mélange au moment de l'emploi avant d'appliquer ce mélange sur les fibres kératiniques, la composition (A) ou la composition (B) contenant au moins le polyol et/ ou l'éther de polyol tel que défini dans la revendication 1.

20. Dispositif à plusieurs compartiments ou "kit" de teinture à plusieurs compartiments, **caractérisé par le fait qu'**un premier compartiment renferme la composition (A) telle que définie à la revendication 19 et un second compartiment renferme la composition (B) telle que définie à la revendication 19.

## Claims

1. Composition for the direct dyeing of keratin fibres, and in particular human keratin fibres such as the hair, containing, in a medium which is suitable for dyeing and which is free of oxidases or oxidoreductases, **(i)** at least one cationic direct dye of formulae (I), (II), (III) and (III') below: 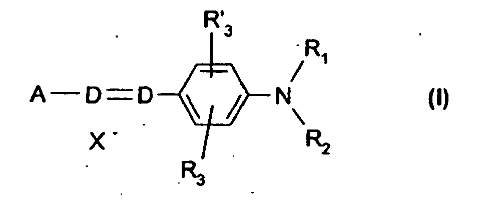 **in which formula (I):**
**D** represents a nitrogen atom or a -CH group,
**R**₁ and **R**₂, which may be identical or different, represent a hydrogen atom; a C₁-C₄ alkyl radical which can be substituted with a -CN, -OH or -NH₂ radical; or form, with a carbon atom of the benzene ring, an optionally oxygenated or nitrogenous heterocycle which can be substituted with one or more C₁-C₄ alkyl radicals; a 4'-aminophenyl radical,
**R**₃ and **R**'₃, which may be identical or different, represent a hydrogen or halogen atom chosen from chlorine, bromine, iodine and fluorine, or a cyano, C₁-C₄ alkyl, C₁-C₄ alkoxy or acetyloxy radical,
**X⁻** represents an anion preferably chosen from chloride, methyl sulphate and acetate,
**A** represents a group chosen from structures A1 to A18 below: 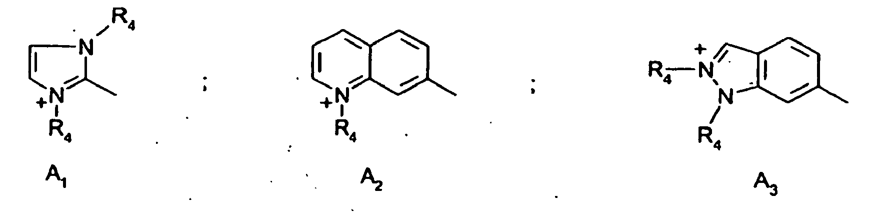 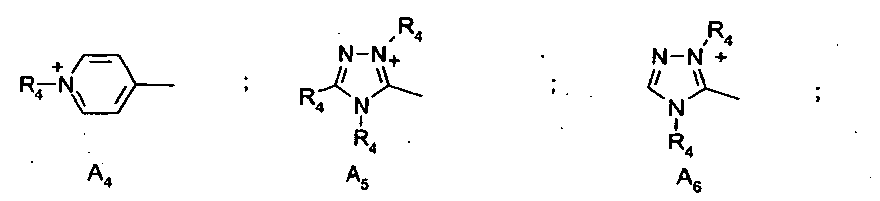 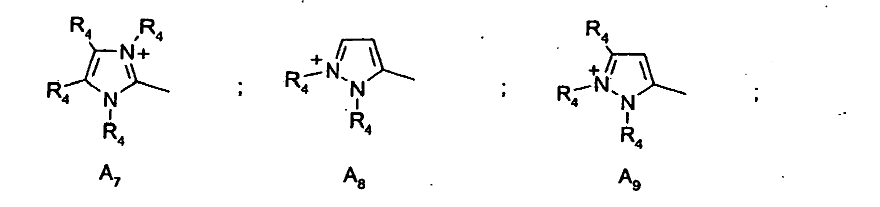 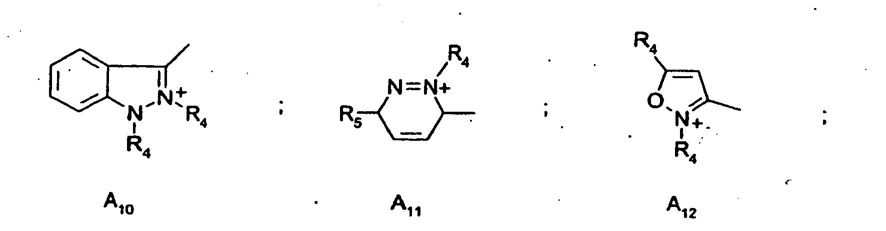 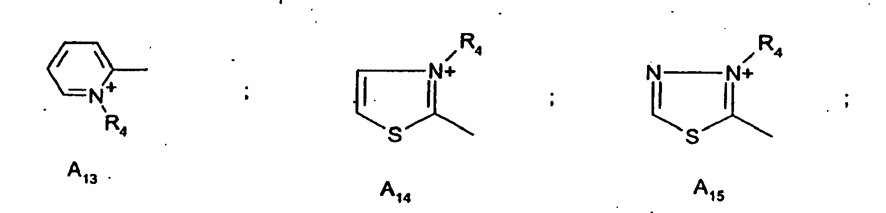 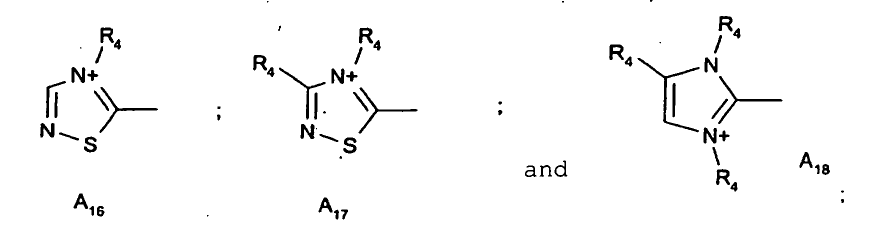
in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom; 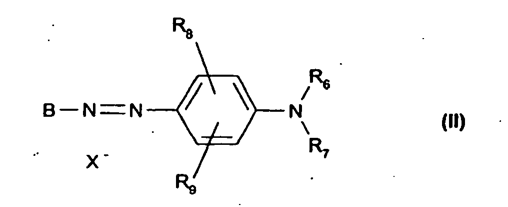 **in which formula (II):**
**R**₆ represents a hydrogen atom or a C₁-C₄ alkyl radical,
**R**₇ represents a hydrogen atom, an alkyl radical which can be substituted with a -CN radical or with an amino group, a 4'-aminophenyl radical, or forms, with R₆, an optionally oxygenated and/or nitrogenous heterocycle which can be substituted with a C₁-C₄ alkyl radical,
**R**₈ and **R**₉, which may be identical or different,
represent a hydrogen atom, a halogen atom such as bromine, chlorine, iodine or fluorine, a C₁-C₄ alkyl or
C₁-C₄ alkoxy radical or a -CN radical,
**X⁻** represents an anion preferably chosen from chloride,
methyl sulphate and acetate,
**B** represents a group chosen from structures B1 to B6 below: 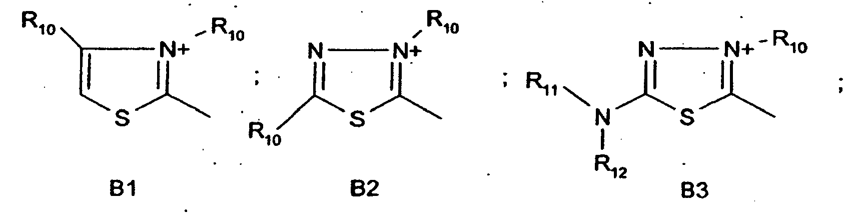 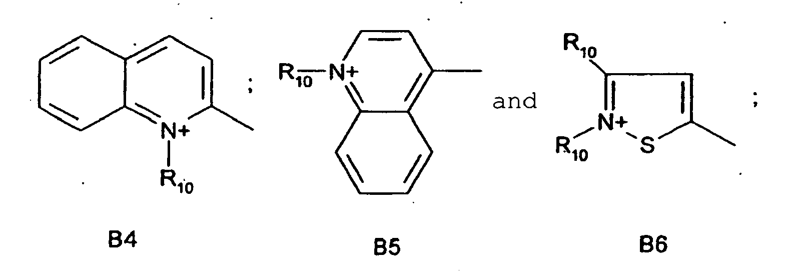
in which R₁₀ represents a C₁-C₄ alkyl radical, R₁₁ and R₁₂, which may be identical or different, represent a hydrogen atom or a C₁-C₄ alkyl radical; 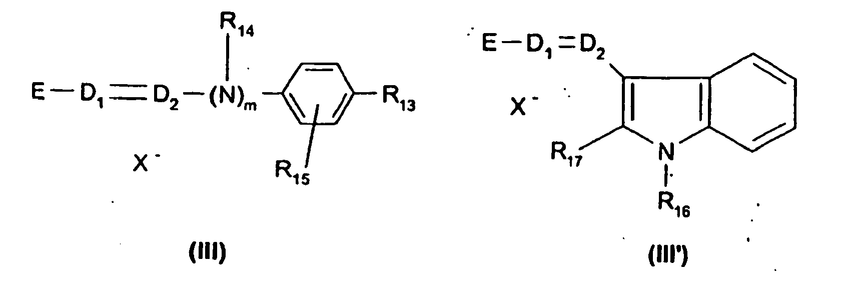 **in which formulae (III) and (III'):**
**R**₁₃ represents a hydrogen atom, a C₁-C₄ alkoxy radical, a halogen atom such as bromine, chlorine, iodine or fluorine or an amino radical,
**R**₁₄ represents a hydrogen atom, a C₁-C₄ alkyl radical or forms, with a carbon atom of the benzene ring, a heterocycle which is optionally oxygenated and/or substituted with one or more C₁-C₄ alkyl groups,
**R**₁₅ represents a hydrogen or halogen atom such as bromine, chlorine, iodine or fluorine,
**R**₁₆ and **R**₁₇, which may be identical or different, represent. a hydrogen atom or a C₁-C₄ alkyl radical,
**D**₁ and **D**₂, which may be identical or different, represent a nitrogen atom or a -CH group,
**m** = 0 or 1,
it being understood that when R₁₃ represents an unsubstituted amino group, then D₁ and D₂ simultaneously represent a -CH group and m = 0,
**X⁻** represents an anion preferably chosen from chloride,
methyl sulphate and acetate,
**E** represents a group chosen from structures E1 to E8 below: 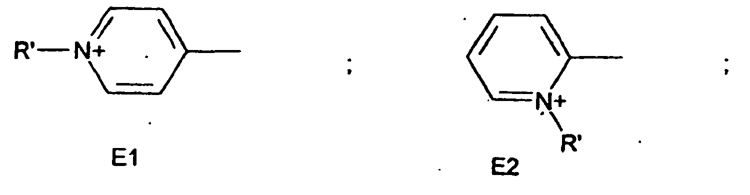 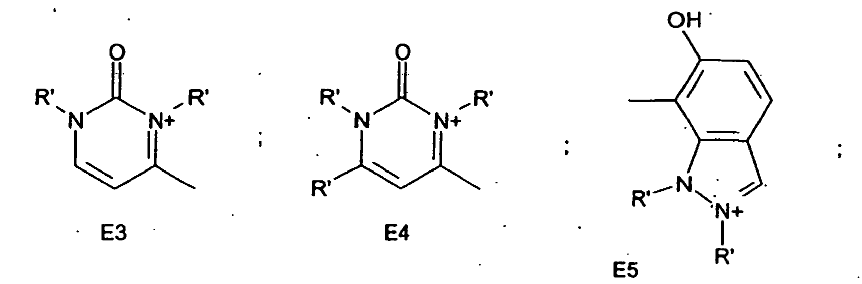 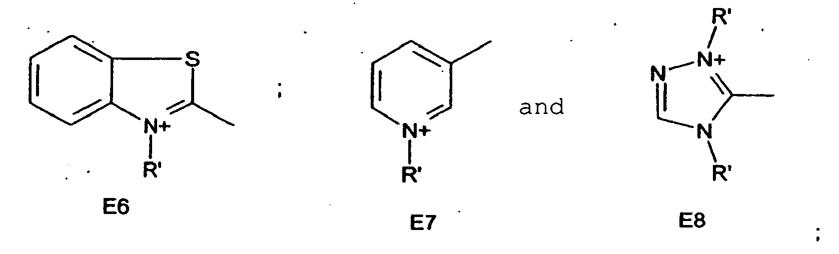
in which R' represents a C₁-C₄ alkyl radical;
when m = 0 and when D₁ represents a nitrogen atom, then E can also denote a group of structure E9 below: 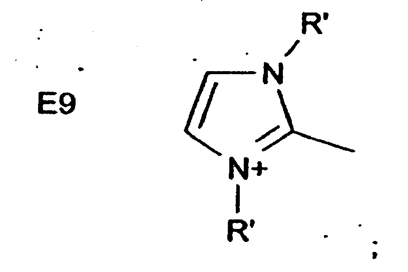 in which R' represents a C₁-C₄ alkyl radical;
the said composition being **characterized in that** it contains **(ii)** at least one polyol and/or C₁-C₈ aliphatic ether of a C₃-C₉ polyol and/or a C₆-C₈ aromatic ether of a C₂-C₉ polyol, chosen from 1,3-propanediol, 2-butene-1,4-diol, pentane-1,5-diol, 2,2-dimethylpropane-1,3-diol, 3-methylpentane-1,5-diol, pentane-1,2-diol, 2,2,4-trimethylpentane-1,3-diol, 2-methylpropane-1,3-diol, hexylene glycol, 1,3-butylene glycol, diethylene glycol, triethylene glycol, propylene glycol monoethyl ether, isopropylene glycol dimethyl ether, dipropylene glycol monomethyl ether, tripropylene glycol monomethyl ether, diethylene glycol dimethyl ether, ethylene glycol monophenyl ether, ethylene glycol monobenzyl ether, propylene glycol monophenyl ether, propylene glycol monobenzyl ether, diethylene glycol monophenyl ether and diethylene glycol monobenzyl ether.

2. Composition according to Claim 1, **characterized in that** the cationic direct dyes of formula (I) are chosen from the compounds corresponding to structures (I1) to (I54) below:

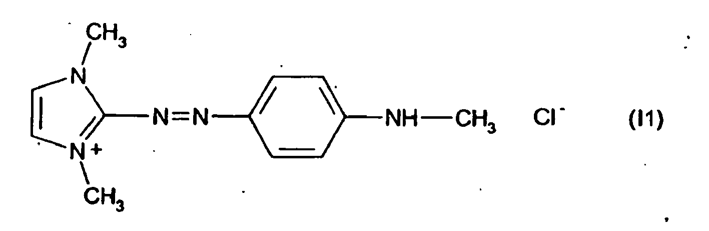

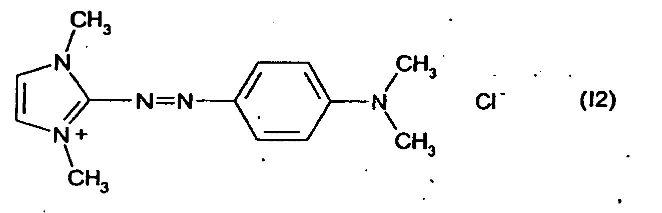

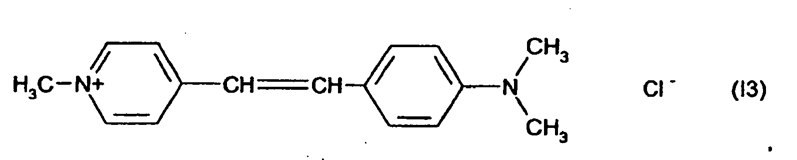

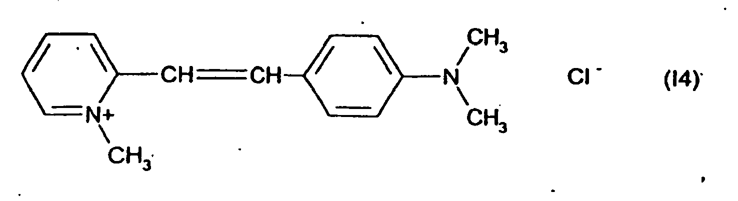

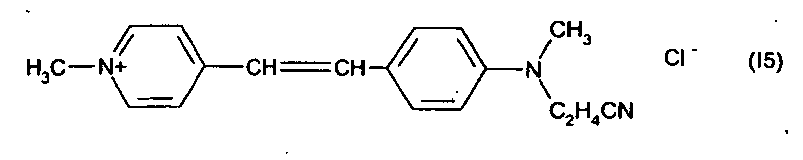

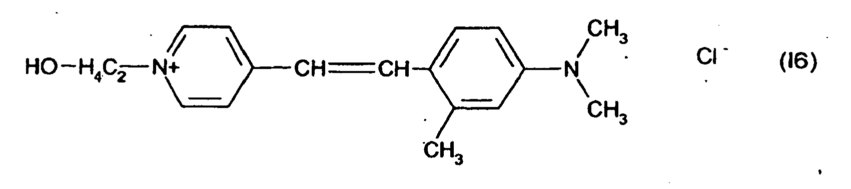

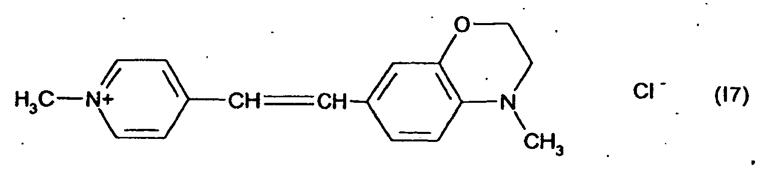

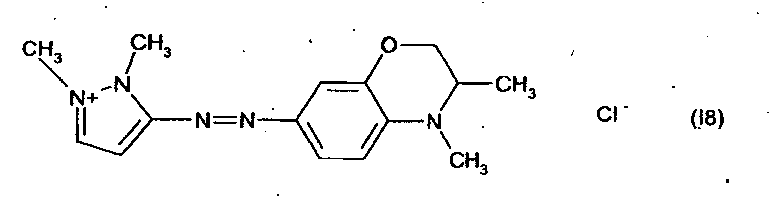

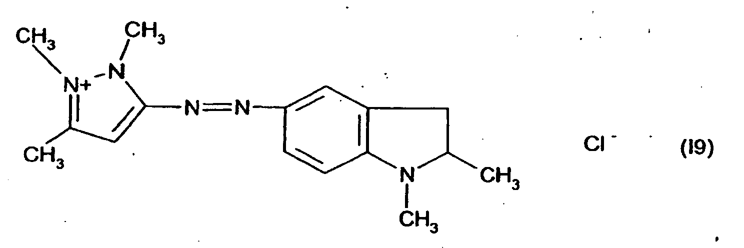

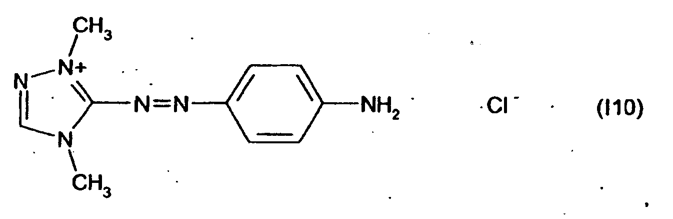

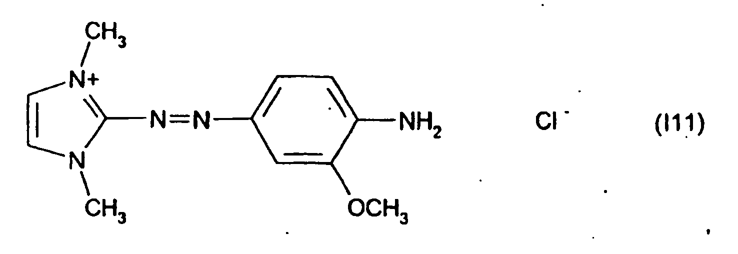

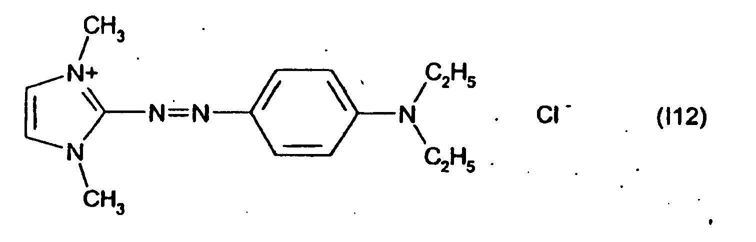

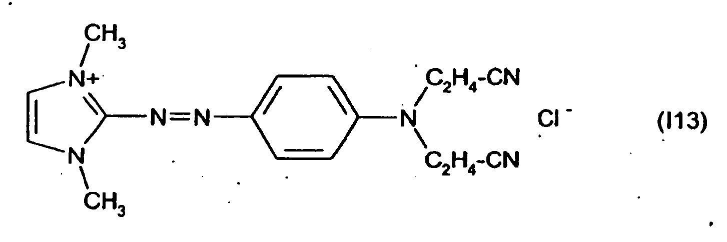

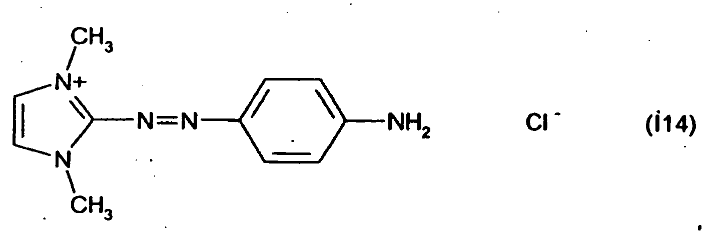

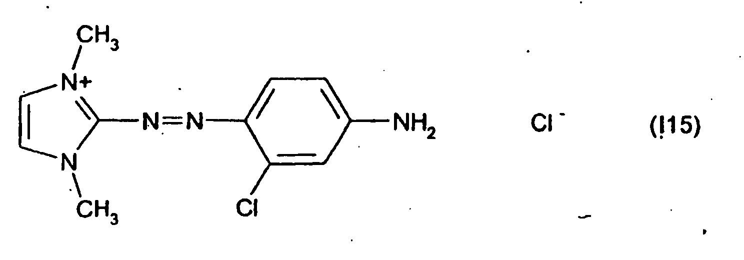

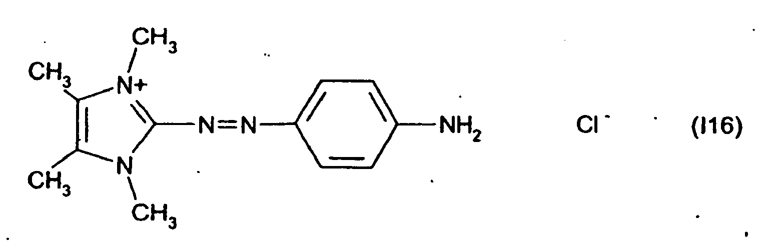

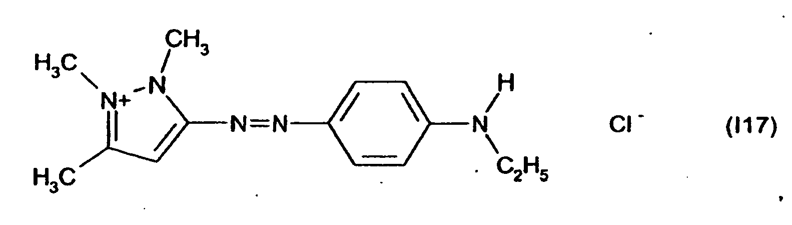

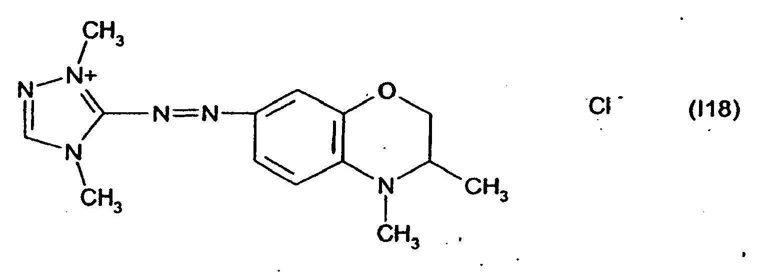

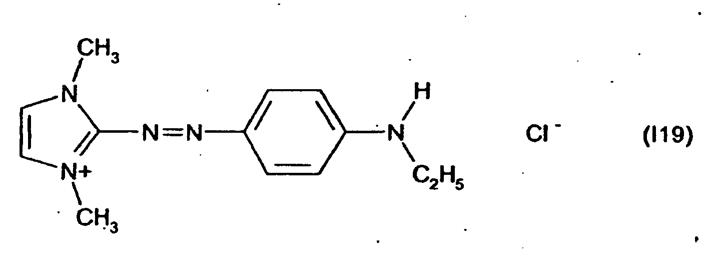

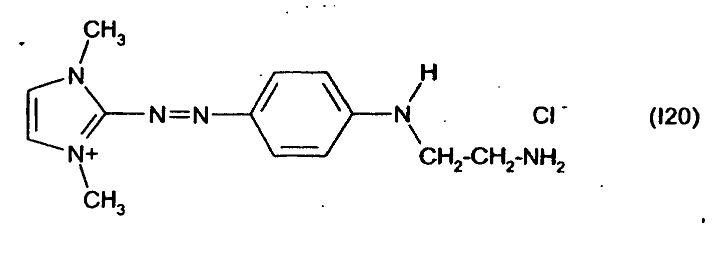

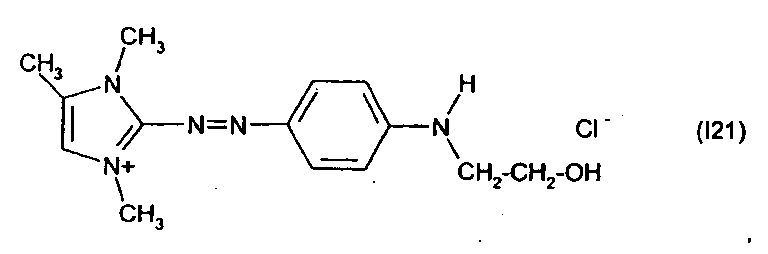

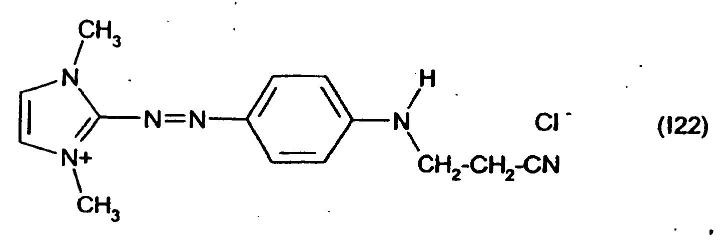

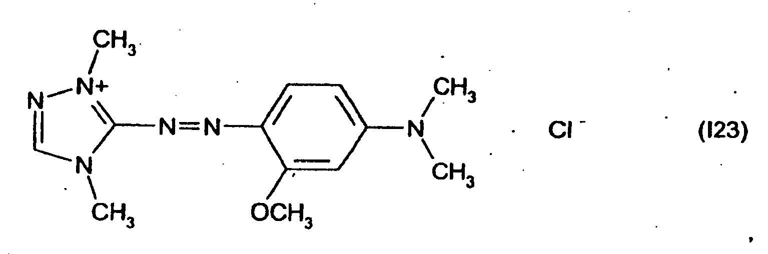

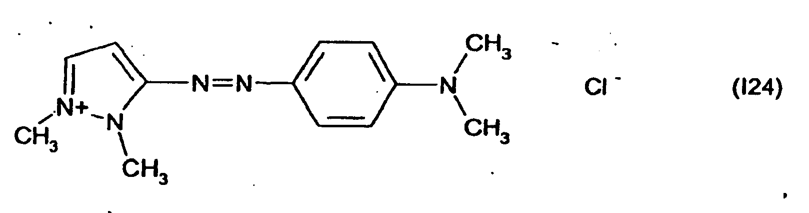

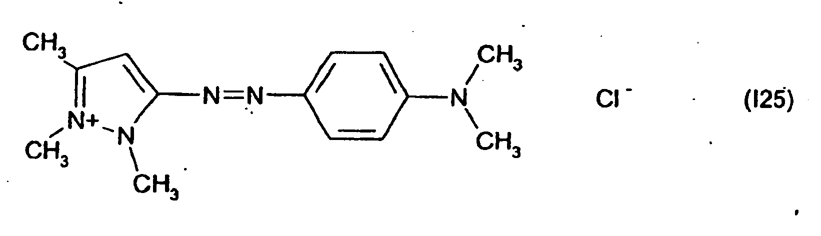

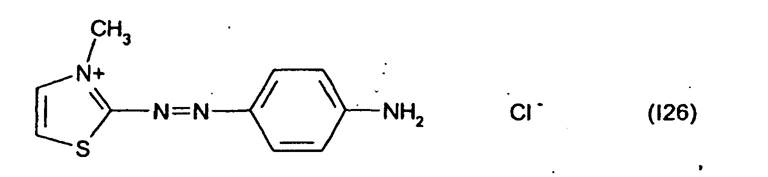

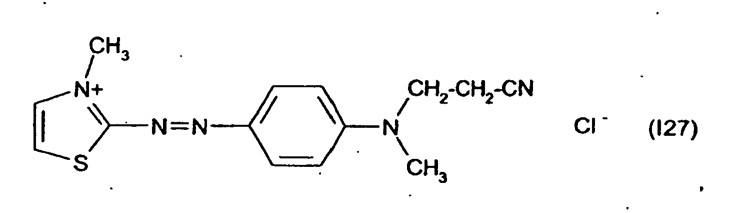

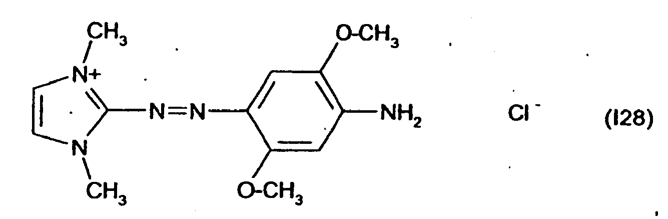

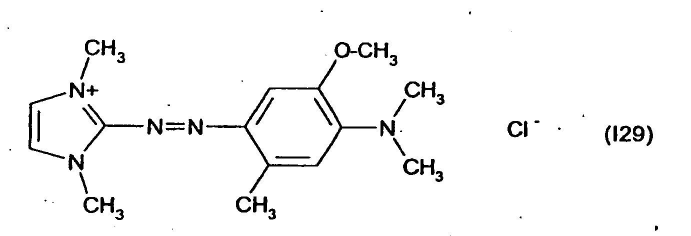

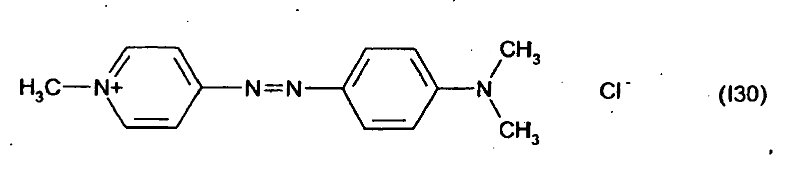

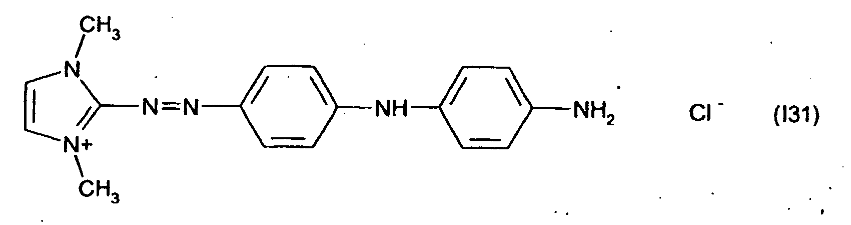

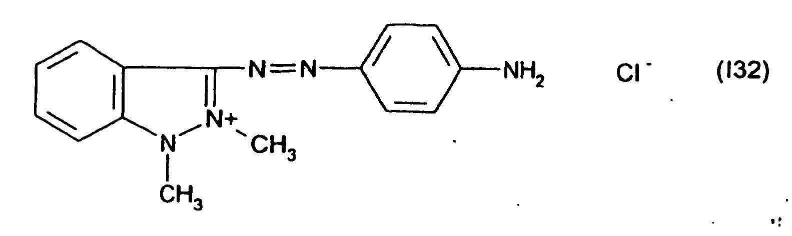

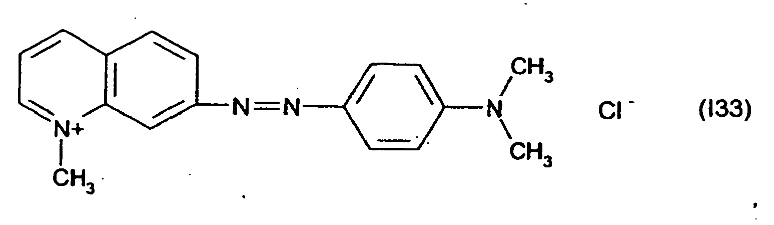

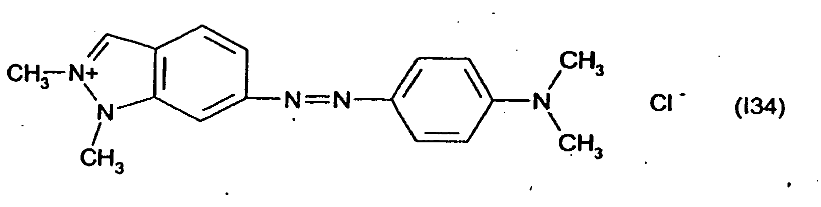

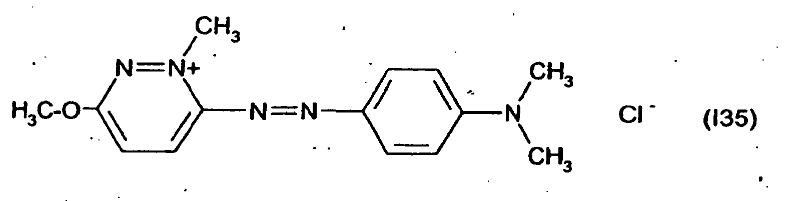

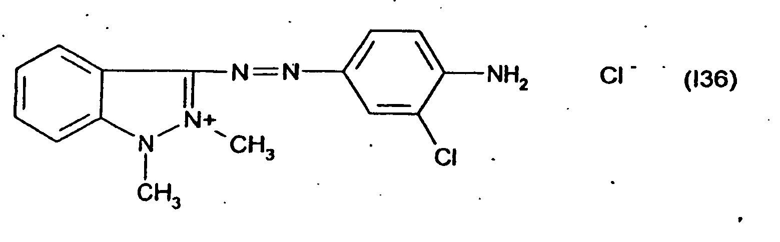

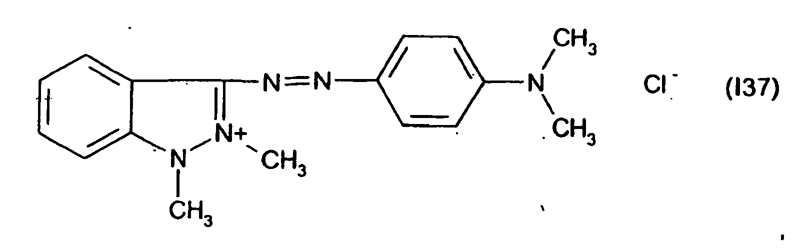

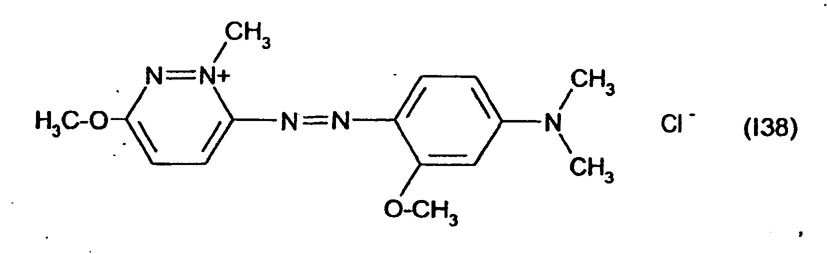

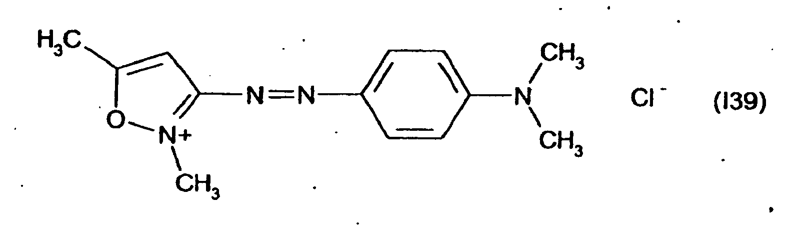

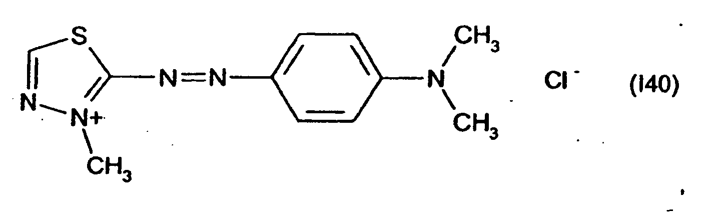

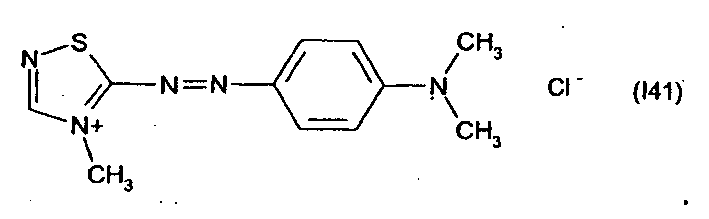

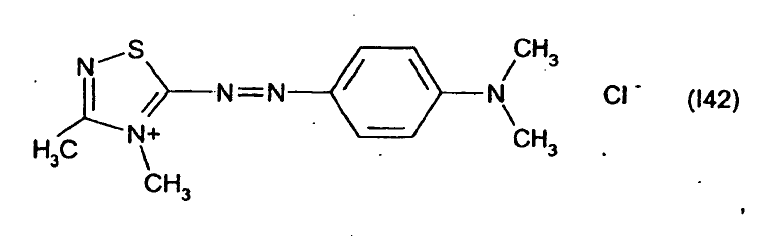

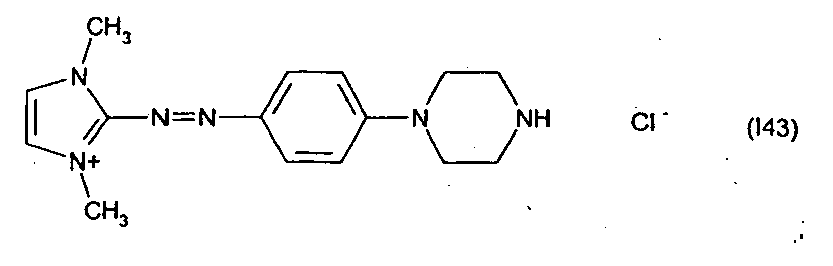

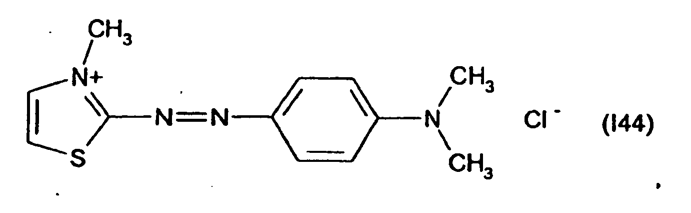

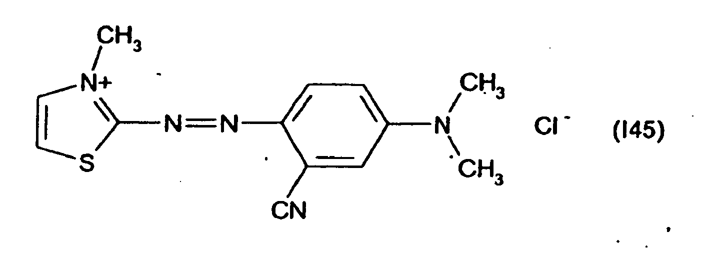

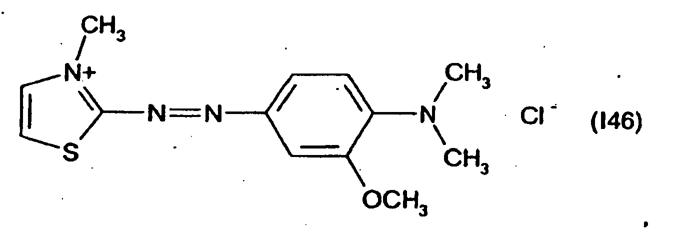

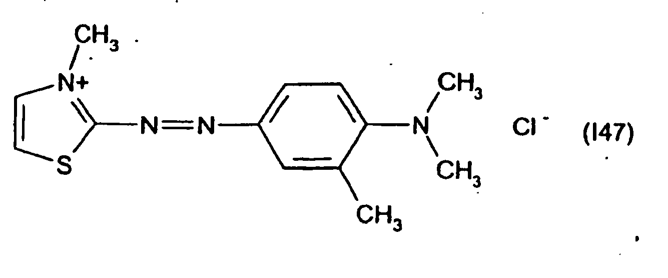

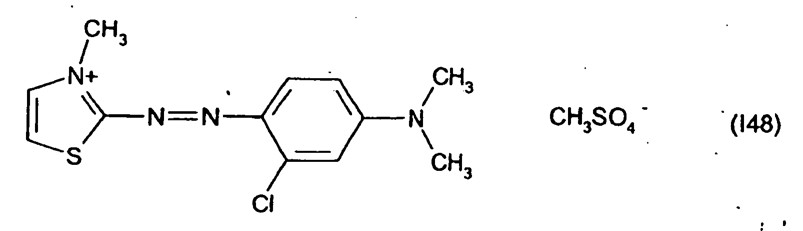

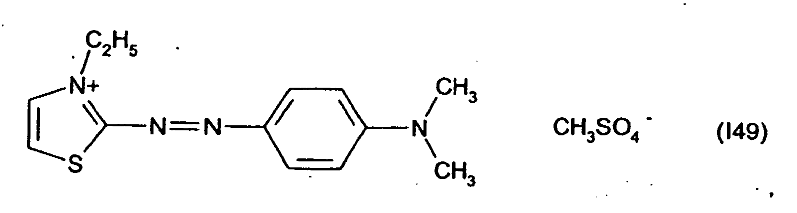

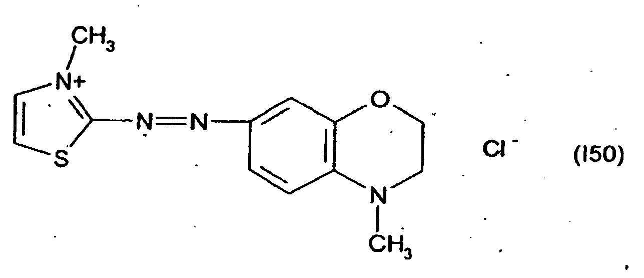

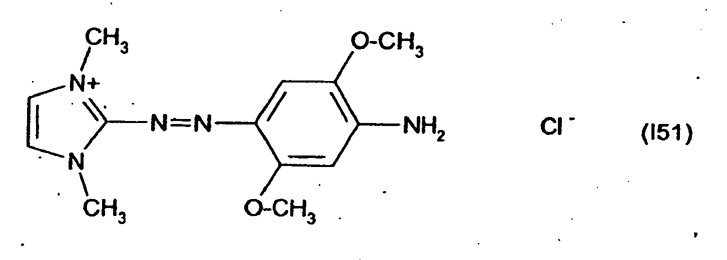

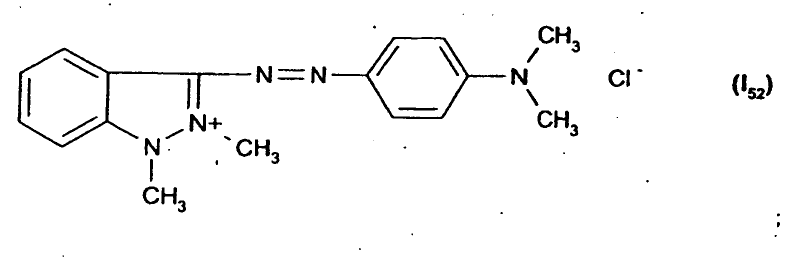

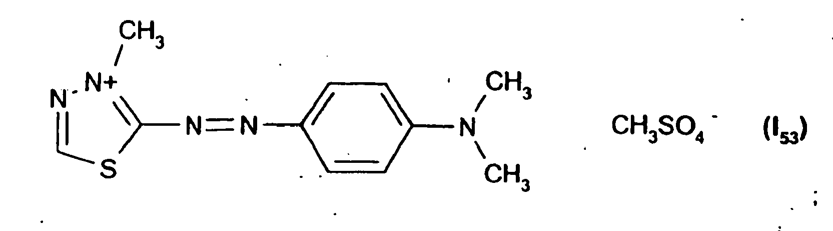

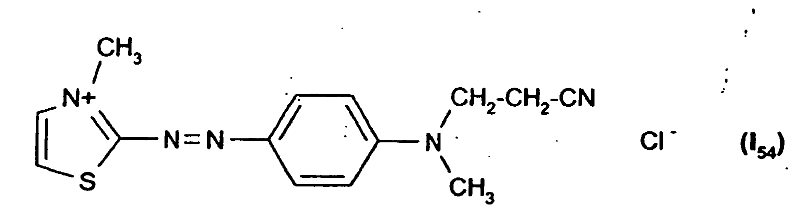

3. Composition according to Claim 2, **characterized in that** the cationic direct dyes correspond to structures (I1), (I2), (I14) and (I31).

4. Composition according to Claim 1, **characterized in that** the cationic direct dyes of formula (II) are chosen from the compounds corresponding to structures (II1) to (II9) below: 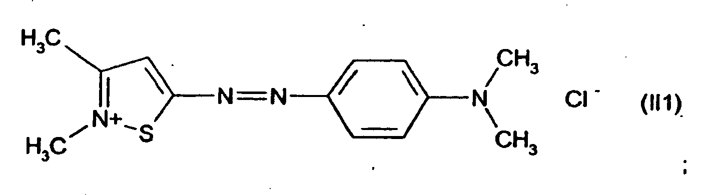 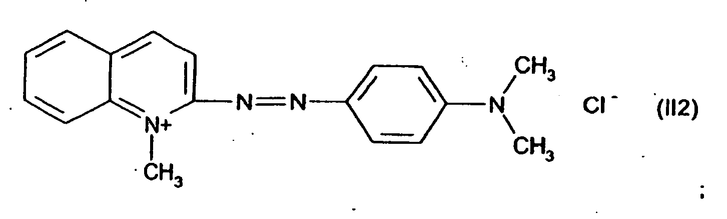 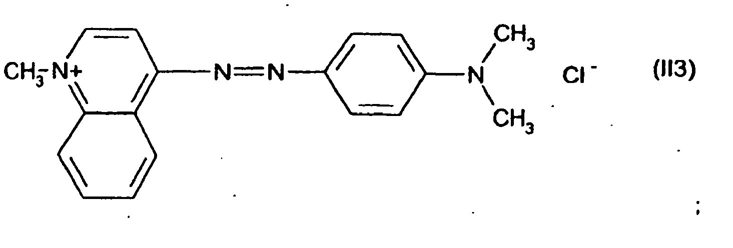 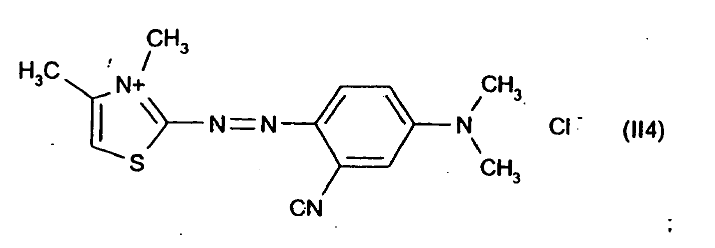 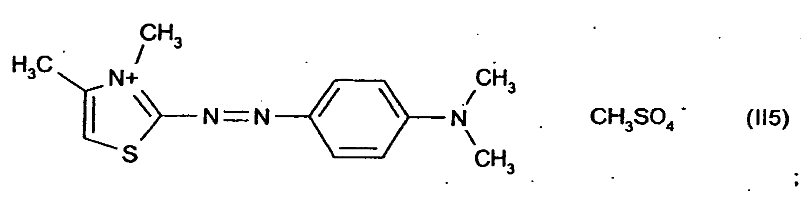 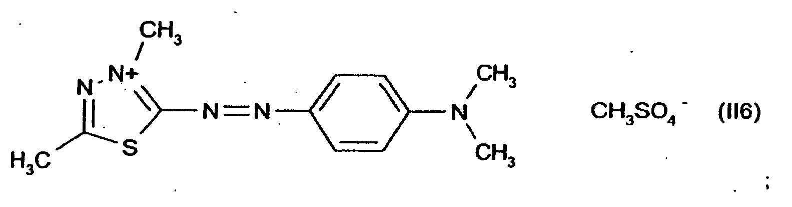 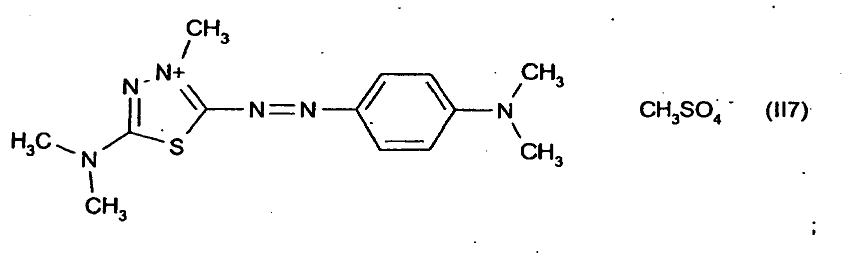 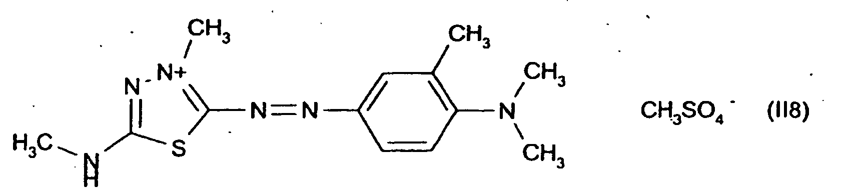 and

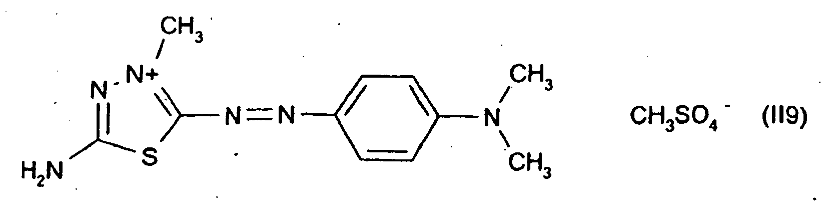

5. Composition according to Claim 1, **characterized in that** the cationic direct dyes of formula (III) are chosen from the compounds corresponding to structures (III1) to (III18) below: 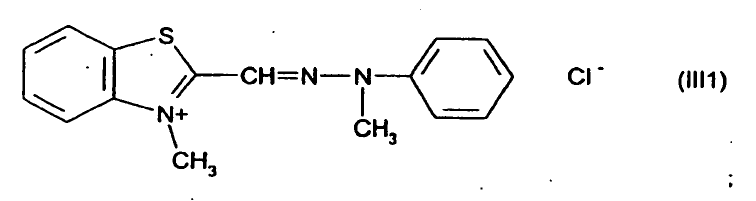 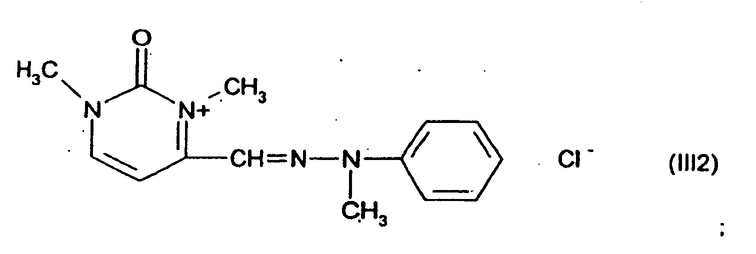 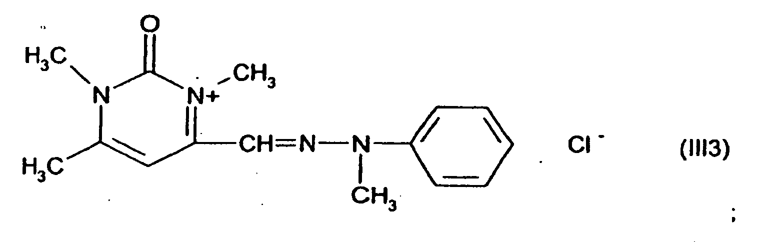 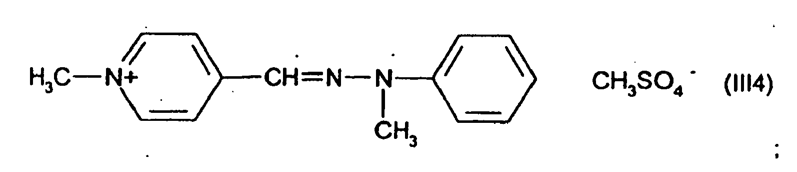 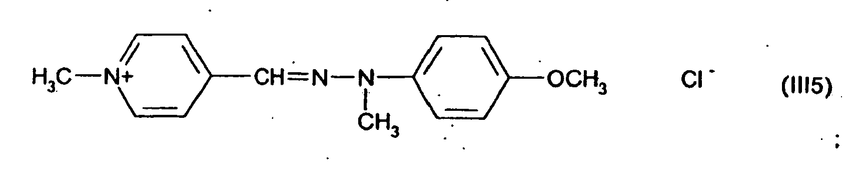 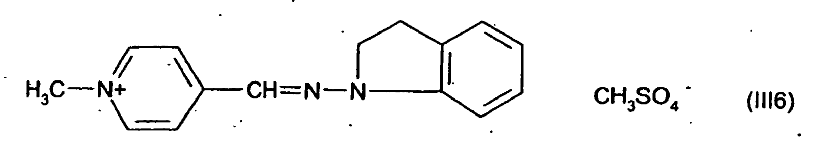 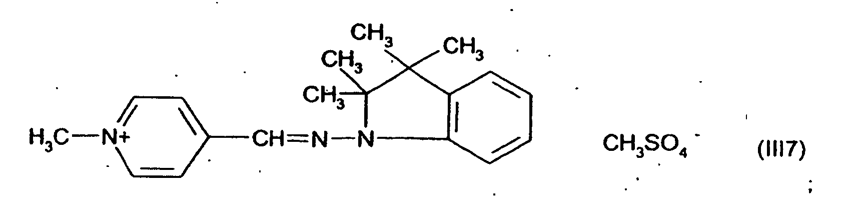 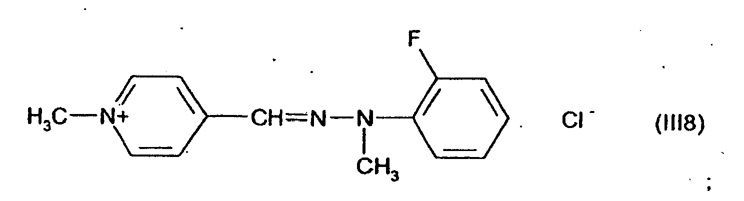 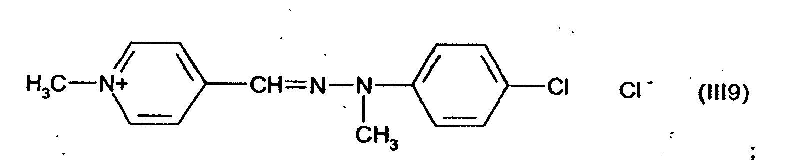 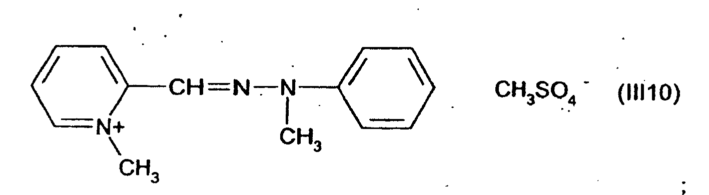 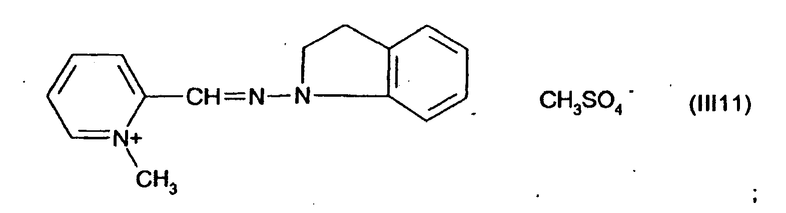 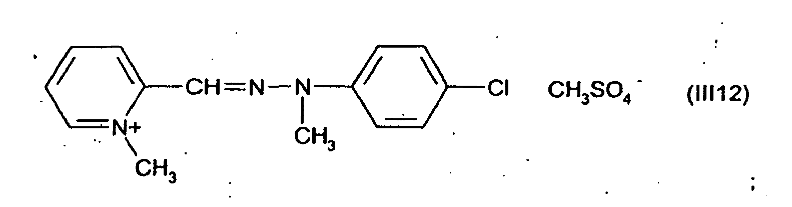 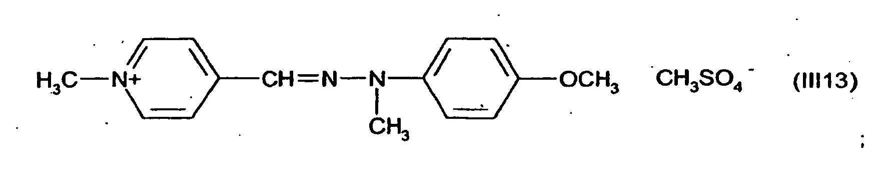 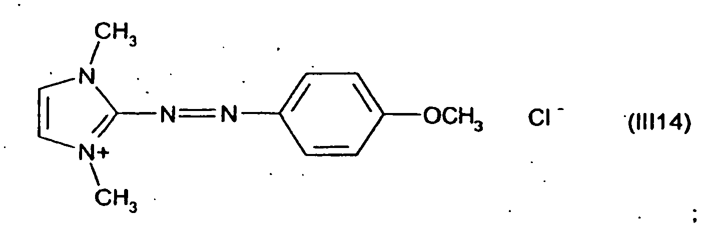 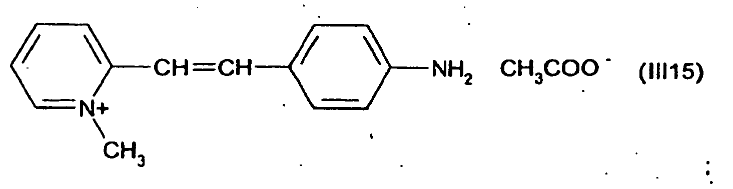 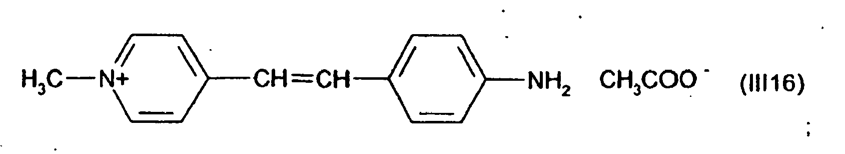 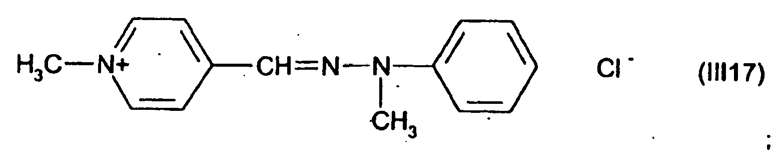 and

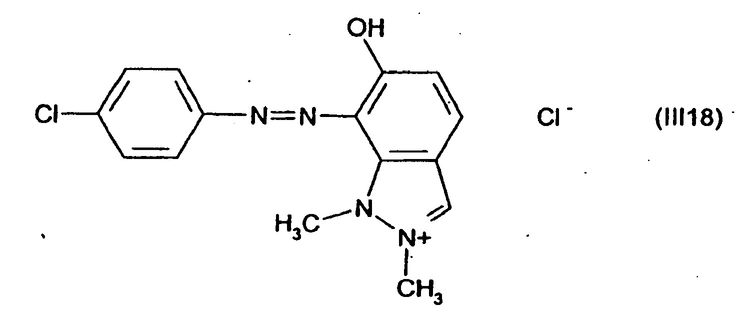

6. Composition according to Claim 5, **characterized in that** the cationic direct dyes of formula (III) are chosen from the compounds corresponding to structures (III4), (III5) and (IIII3).

7. Composition according to Claim 1, **characterized in that** the cationic direct dyes of formula (III') are chosen from the compounds corresponding to structures (III'1) to (III'3) below: 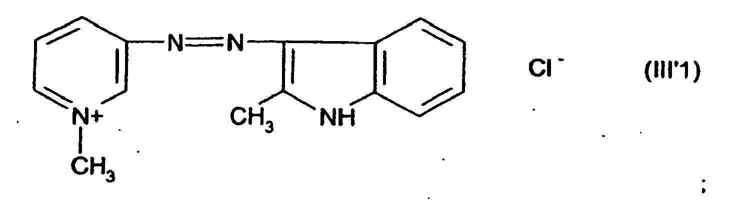 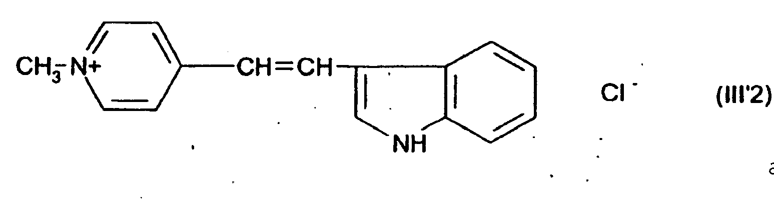 and

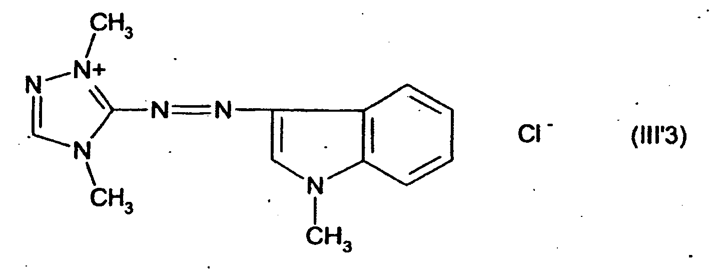

8. Composition according to any one of the preceding claims, **characterized in that** the cationic direct dye(s) of formulae (I), (II), (III) or (III') represent(s) from 0.001 to 10% by weight relative to the total weight of the composition.

9. Composition according to Claim 8, **characterized in that** the cationic direct dye(s) of formulae (I), (II), (III) or (III') represent (s) from 0.005 to 5% by weight relative to the total weight of the composition.

10. Composition according to any one of the preceding claims, **characterized in that** the polyol and/or the C₁-C₈ aliphatic ether of a C₃-C₉ polyol and/or the C₆-C₈ aromatic ether of a C₂-C₉ polyol represent from 0.1 to 40% by weight relative to the total weight of the composition.

11. Composition according to Claim 10, **characterized in that** the polyol and/or the C₁-C₈ aliphatic ether of a C₃-C₉ polyol and/or the C₆-C₈ aromatic ether of a C₂-C₉ polyol represent from 0.5 to 20% by weight relative to the total weight of the composition.

12. Composition according to any one of the preceding claims, **characterized in that** it also contains additional direct dyes.

13. Composition according to any one of the preceding claims, **characterized in that** the medium which is suitable for the keratin fibres (or support) consists of a mixture of water and at least one solvent chosen from the polyols and/or polyol ethers defined in Claim 1.

14. Composition according to Claim 13, **characterized in that** the medium also comprises at least one organic solvent.

15. Composition according to any one of the preceding claims, **characterized in that** it has a pH of between 2 and 11 and preferably between 5 and 10.

16. Composition according to any one of the preceding claims, **characterized in that** it is intended for lightening direct dyeing and **in that** it contains at least one oxidizing agent.

17. Process for the direct dyeing of keratin fibres, and in particular human keratin fibres such as the hair, **characterized in that** at least one dye composition is applied to the fibres, comprising in a medium which is suitable for dyeing and which is free of oxidases or oxidoreductases, **(i)** at least one cationic direct dye of formulae (I), (II), (III) and (III') below: 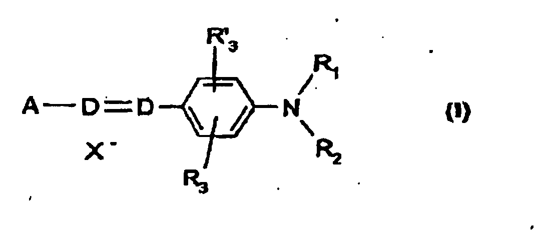 **in which formula (I):**
**D** represents a nitrogen atom or a -CH group,
**R₁** and **R₂,** which may be identical or different, represent a hydrogen atom; a C₁-C₄ alkyl radical which can be substituted with a -CN, -OH or -NH₂ radical; or form, with a carbon atom of the benzene ring, an optionally oxygenated or nitrogenous heterocycle which can be substituted with one or more C₁-C₄ alkyl radicals; a 4'-aminophenyl radical,
**R₃** and **R'₃,** which may be identical or different, represent a hydrogen or halogen atom chosen from chlorine, bromine, iodine and fluorine, or a cyano, C₁-C₄ alkyl, C₁-C₄ alkoxy or acetyloxy radical,
**X⁻** represents an anion preferably chosen from chloride, methyl sulphate and acetate,
**A** represents a group chosen from structures A1 to A18 below: 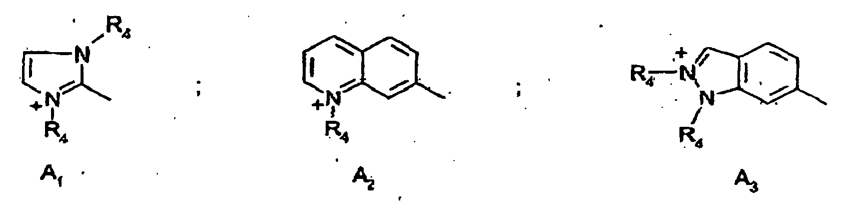 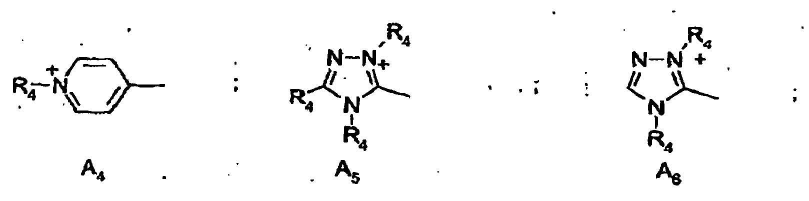 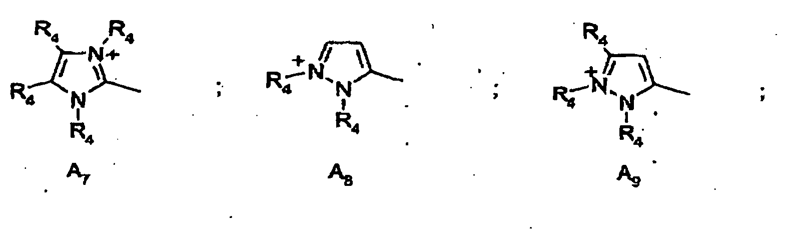 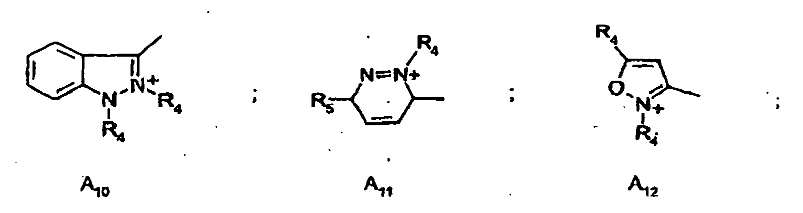 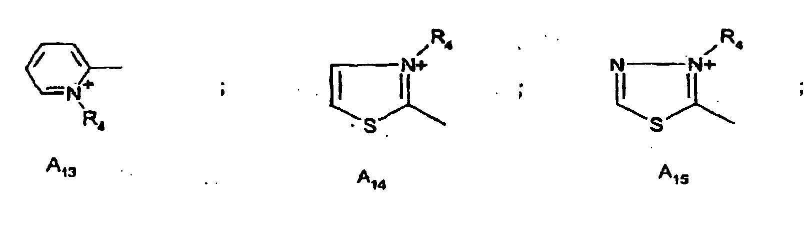 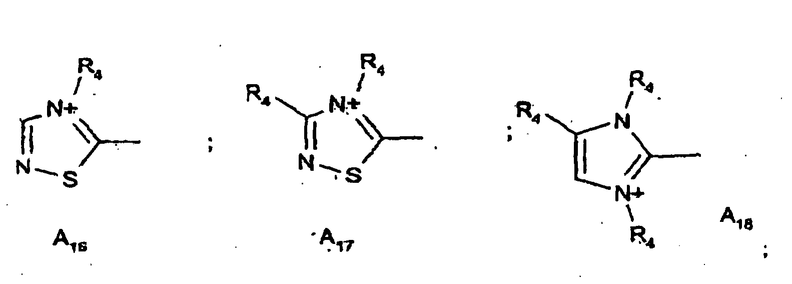
in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom; 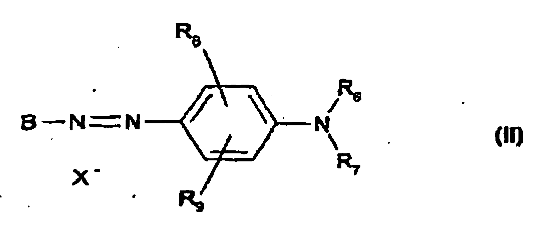 **in which formula (II):**
**R₆** represents a hydrogen atom or a C₁-C₄ alkyl radical,
**R₇** represents a hydrogen atom, an alkyl radical which can be substituted with a -CN radical or with an amino group, a 4'-aminophenyl radical, or forms, with R₆, an optionally oxygenated and/or nitrogenous heterocycle which can be substituted with a C₁-C₄ alkyl radical,
**R₈** and **R₉,** which may be identical or different, represent a hydrogen atom, a halogen atom such as bromine, chlorine, iodine or fluorine, a C₁-C₄ alkyl or C₁-C₄ alkoxy radical or a -CN radical,
**X⁻** represents an anion preferably chosen from chloride, methyl sulphate and acetate,
**B** represents a group chosen from structures B1 to B6 below: 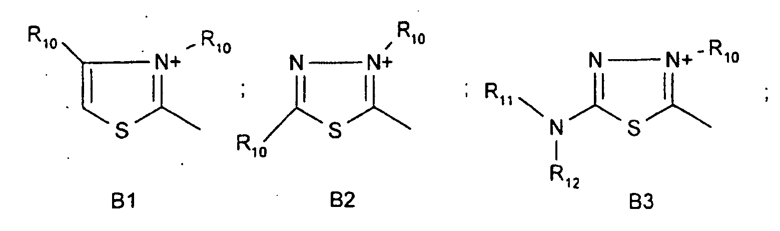 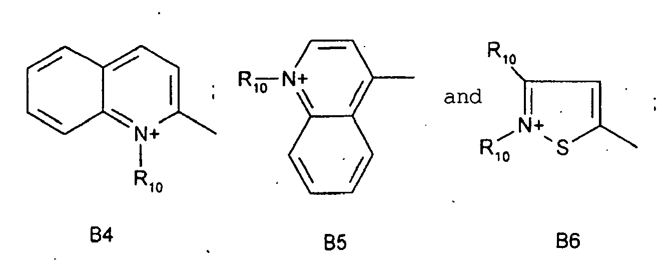
in which R₁₀ represents a C₁-C₄ alkyl radical, R₁₁ and R₁₂, which may be identical or different, represent a hydrogen atom or a C₁-C₄ alkyl radical; 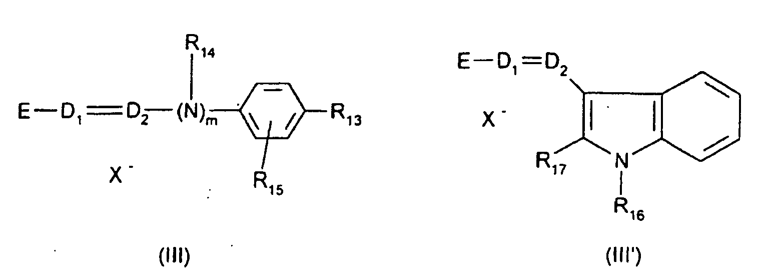 **in which formulae (III) and (III'):**
**R₁₃** represents a hydrogen atom, a C₁-C₄ alkoxy radical, a halogen atom such as bromine, chlorine, iodine or fluorine or an amino radical,
**R₁₄** represents a hydrogen atom, a C₁-C₄ alkyl radical or forms, with a carbon atom of the benzene ring, a heterocycle which is optionally oxygenated and/or substituted with one or more C₁-C₄ alkyl groups,
**R₁₅** represents a hydrogen or halogen atom such as bromine, chlorine, iodine or fluorine,
**R₁₆** and **R₁₇,** which may be identical or different, represent a hydrogen atom or a C₁-C₄ alkyl radical,
**D₁** and **D₂,** which may be identical or different, represent a nitrogen atom or a -CH group,
**m** = 0 or 1,
it being understood that when R₁₃ represents an unsubstituted amino group, then D₁ and D₂ simultaneously represent a -CH group and m = 0,
**X⁻** represents an anion preferably chosen from chloride, methyl sulphate and acetate,
**E** represents a group chosen from structures E1 to E8 below: 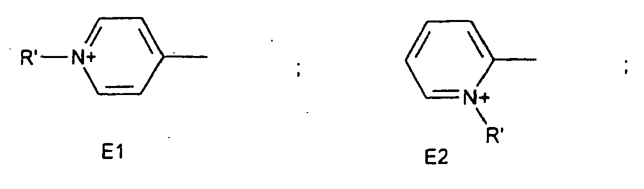 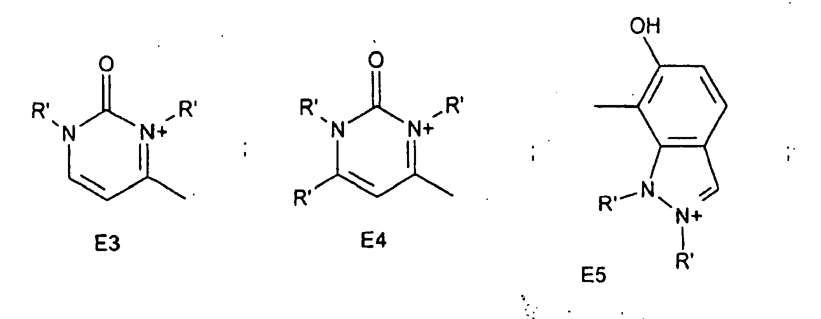 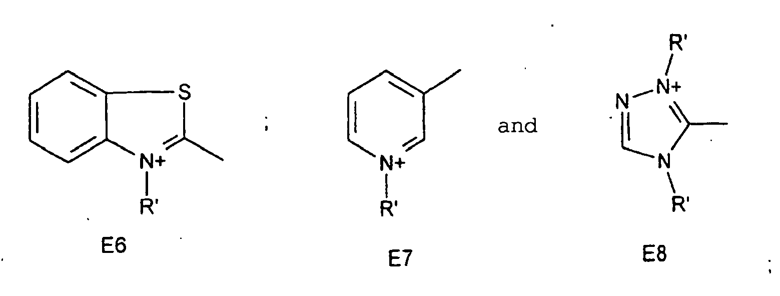
in which R' represents a C₁-C₄ alkyl radical;
when m = 0 and when D₁ represents a nitrogen atom, then E can also denote a group of structure E9 below: 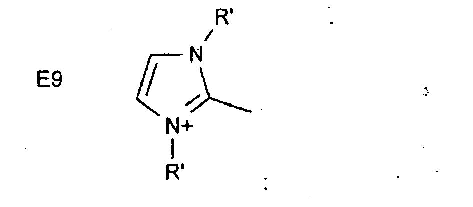 in which R' represents a C₁-C₄ alkyl radical;
the said composition being **characterized in that** it contains **(ii)** at least one polyol and/or C₁-C₈ aliphatic ether of a C₃-C₉ polyol and/or a C₆-C₈ aromatic ether of a C₂-C₉ polyol, and as defined in any one of Claims 2 to 16, for a period which is sufficient to develop the desired coloration, after which the fibres are rinsed, optionally washed with shampoo, rinsed again and dried.

18. Process for the direct dyeing of keratin fibres, and in particular human keratin fibres such as the hair, **characterized in that** at least one dye composition is applied to the fibres, comprising in a medium which is suitable for dyeing and which is free of oxidases or oxidoreductases, **(i)** at least one cationic direct dye of formulae (I), (II), (III) and (III') below: 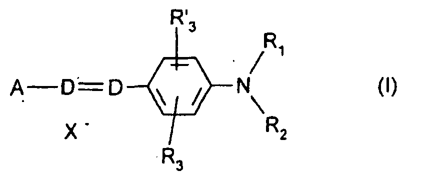 **in which formula (I):**
**D** represents a nitrogen atom or a -CH group,
**R₁** and **R₂,** which may be identical or different, represent a hydrogen atom; a C₁-C₄ alkyl radical which can be substituted with a -CN, -OH or -NH₂ radical; or form, with a carbon atom of the benzene ring, an optionally oxygenated or nitrogenous heterocycle which can be substituted with one or more C₁-C₄ alkyl radicals; a 4'-aminophenyl radical,
**R₃** and **R'₃,** which may be identical or different, represent a hydrogen or halogen atom chosen from chlorine, bromine, iodine and fluorine, or a cyano, C₁-C₄ alkyl, C₁-C₄ alkoxy or acetyloxy radical,
**X⁻** represents an anion preferably chosen from.chloride, methyl sulphate and acetate,
**A** represents a group chosen from structures A1 to A18 below: 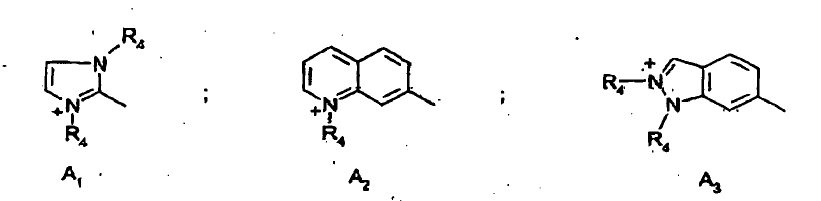 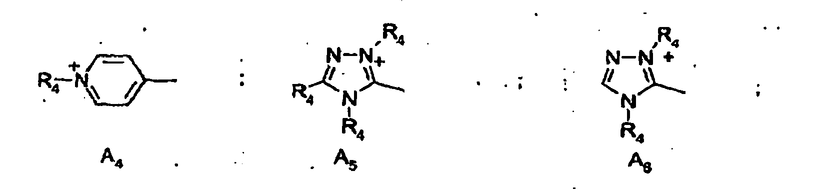 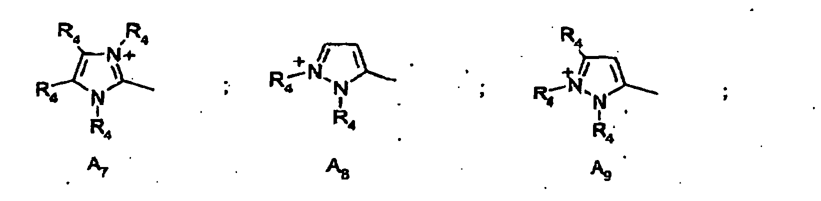 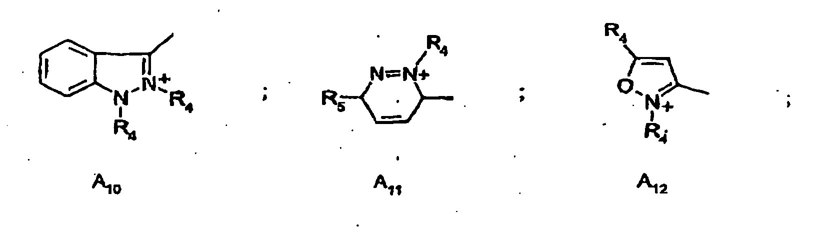 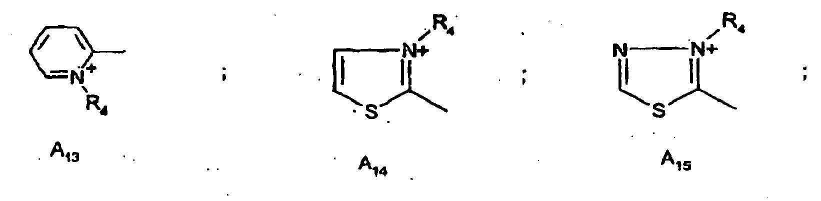 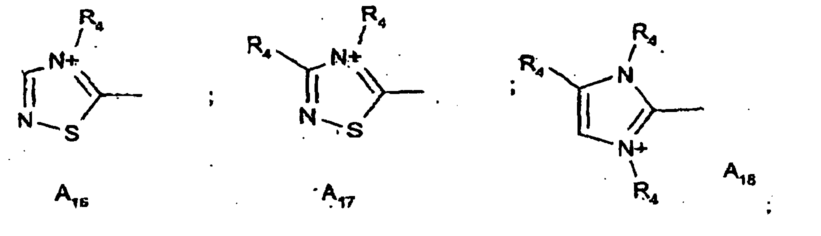
in which R₄ represents a C₁-C₄ alkyl radical which can be substituted with a hydroxyl radical and R₅ represents a C₁-C₄ alkoxy radical, with the proviso that when D represents -CH, when A represents A₄ or A₁₃ and when R₃ is other than an alkoxy radical, then R₁ and R₂ do not simultaneously denote a hydrogen atom; 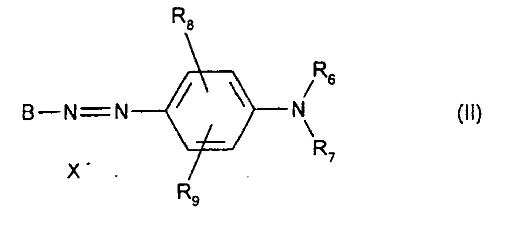 **in which formula (II):**
**R₆** represents a hydrogen atom or a C₁-C₄ alkyl radical,
**R₇** represents a hydrogen atom, an alkyl radical which can be substituted with a -CN radical or with an amino group, a 4'-aminophenyl radical, or forms, with R₆, an optionally oxygenated and/or nitrogenous heterocycle which can be substituted with a C₁-C₄ alkyl radical,
**R₈** and **R₉,** which may be identical or different, represent a hydrogen atom, a halogen atom such as bromine, chlorine, iodine or fluorine, a C₁-C₄ alkyl or C₁-C₄ alkoxy radical or a -CN radical,
**X⁻** represents an anion preferably chosen from chloride, methyl sulphate and acetate,
**B** represents a group chosen from structures B1 to B6 below: 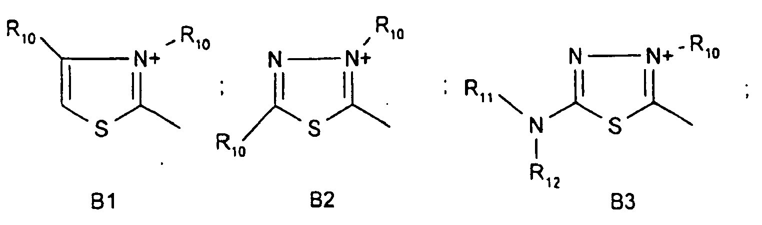 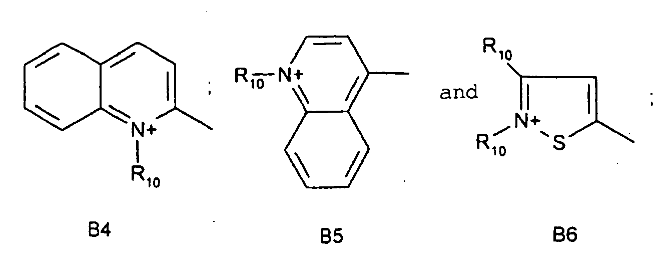
in which R₁₀ represents a C₁-C₄ alkyl radical, R₁₁ and R₁₂, which may be identical or different, represent a hydrogen atom or a C₁-C₄ alkyl radical; 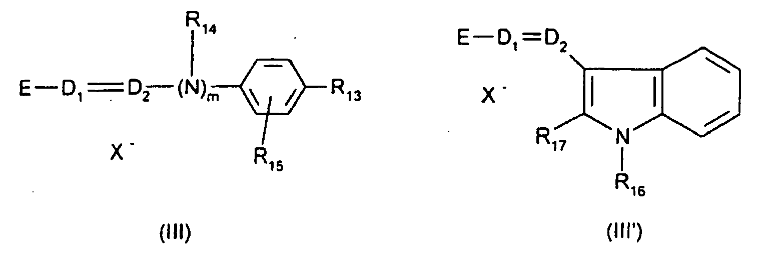 **in which formulae (III) and (III'):**
**R₁₃** represents a hydrogen atom, a C₁-C₄ alkoxy radical, a halogen atom such as bromine, chlorine, iodine or fluorine or an amino radical,
**R₁₄** represents a hydrogen atom, a C₁-C₄ alkyl radical or forms, with a carbon atom of the benzene ring, a heterocycle which is optionally oxygenated and/or substituted with one or more C₁-C₄ alkyl groups,
**R₁₅** represents a hydrogen or halogen atom such as bromine, chlorine, iodine or fluorine,
**R₁₆** and **R₁₇,** which may be identical or different, represent a hydrogen atom or a C₁-C₄ alkyl radical,
**D₁** and **D₂,** which may be identical or different, represent a nitrogen atom or a -CH group,
**m** = 0 or 1,
it being understood that when R₁₃ represents an unsubstituted amino group, then D₁ and D₂ simultaneously represent a -CH group and m = 0,
**X⁻** represents an anion preferably chosen from chloride, methyl sulphate and acetate,
**E** represents a group chosen from structures E1 to E8 below: 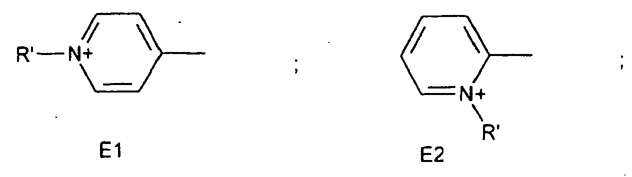 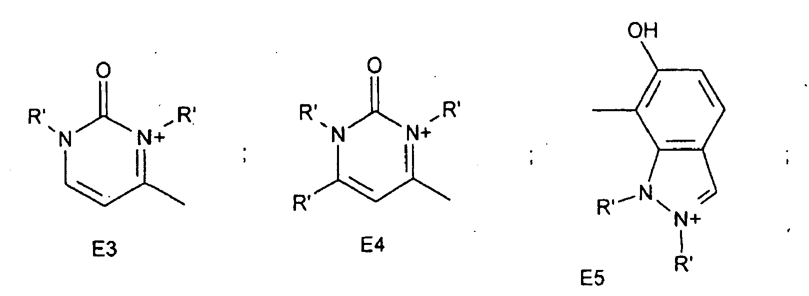 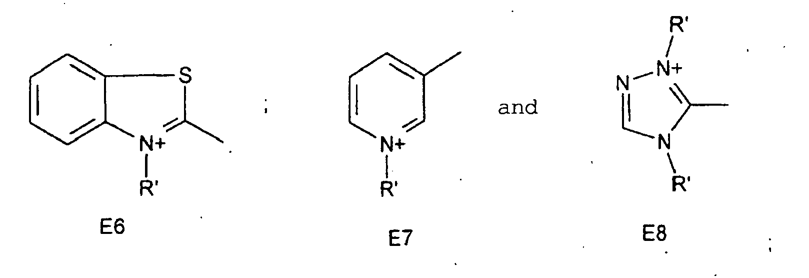
in which R' represents a C₁-C₄ alkyl radical;
when m = 0 and when D₁ represents a nitrogen atom, then E can also denote a group of structure E9 below: 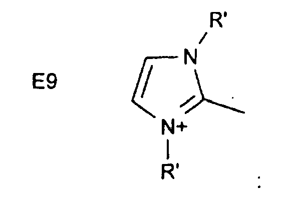 in which R' represents a C₁-C₄ alkyl radical; the said composition being **characterized in that** it contains **(ii)** at least one polyol and/or C₁-C₈ aliphatic ether of a C₃-C₉ polyol and/or a C₆-C₈ aromatic ether of a C₂-C₉ polyol, and as defined in any one of Claims 1 to 16, for a period which is sufficient to develop the desired coloration, without final rinsing.

19. Process for the direct dyeing of keratin fibres, and in particular human keratin fibres such as the hair, according to either of Claims 17 and 18, **characterized in that** it includes a preliminary step which consists in separately storing, on the one hand, a composition (A) comprising, in a medium which is suitable for dyeing, at least one cationic direct dye as defined in the preceding claims and, on the other hand, a composition (B) containing, in a medium which is suitable for dyeing, at least one oxidizing agent other than oxidases and/or oxidoreductases, and then in mixing them together at the time of use, after which this mixture is applied to the keratin fibres, composition (A) or composition (B) containing at least the polyol and/or polyol ether as defined in Claim 1.

20. Multi-compartment dyeing device or multi-compartment dyeing kit, **characterized in that** a first compartment contains composition (A) as defined in Claim 19 and a second compartment contains composition (B) as defined in Claim 19.

## Patentansprüche

1. Zusammensetzung für die direktziehende Färbung von Keratinfasern und insbesondere menschlichen Keratinfasern, wie Haaren, die in einem zum Färben geeigneten Medium, das keine Oxidasen oder Oxidoreduktasen aufweist, enthält: (i) mindestens einen kationischen Direktfarbstoff der folgenden Formeln (I), (II), (III) oder (III'): 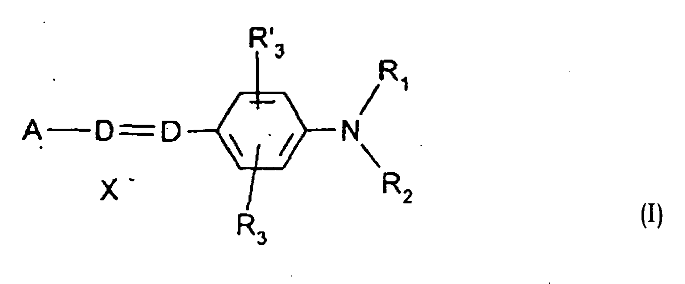 wobei in der Formel (I) bedeuten:
**D** ein Stickstoffatom oder die Gruppe -CH,
**R₁** und **R₂,** die gleich oder verschieden sind, ein Wasserstoffatom; eine C₁₋₄-Alkylgruppe, die mit einer Gruppe - CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder eine 4'-Aminophenylgruppe,
**R₃** und **R'₃,** die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder
Acetyloxy,
**X** ein Anion, das vorzugsweise unter Chlorid, Methylsulfat und
Acetat ausgewählt ist,
**A** eine Gruppe, die unter den folgenden Strukturen A1 bis A18 ausgewählt ist: 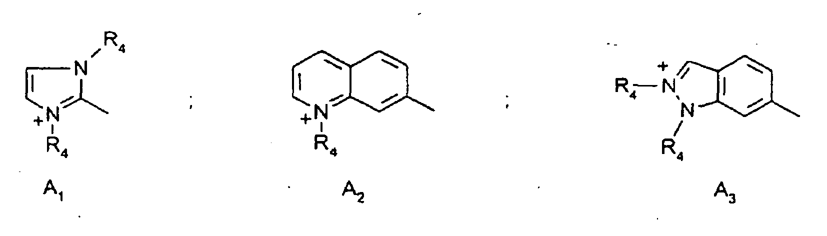 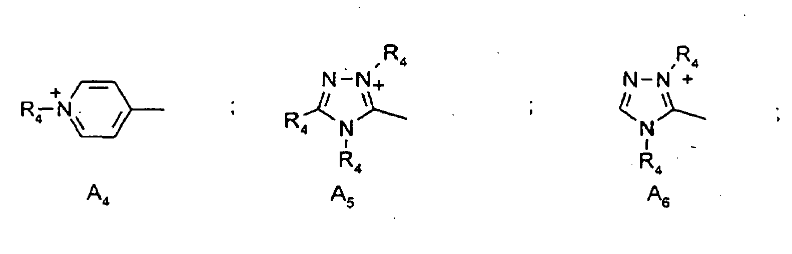 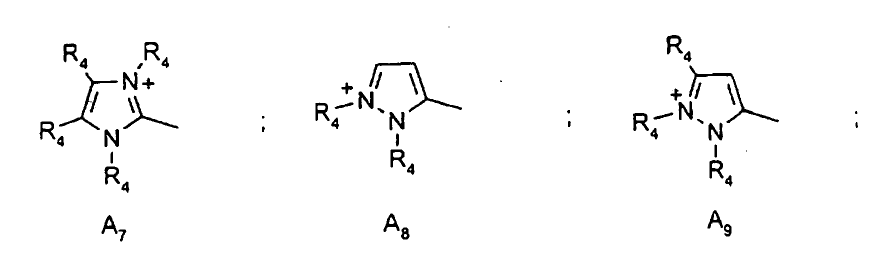 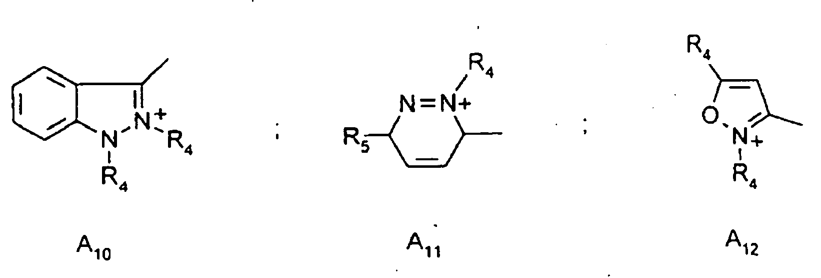 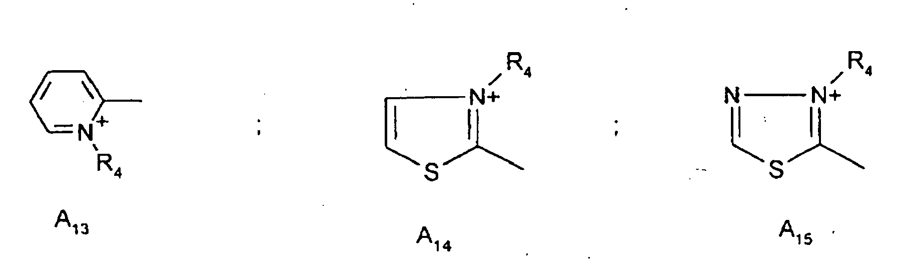 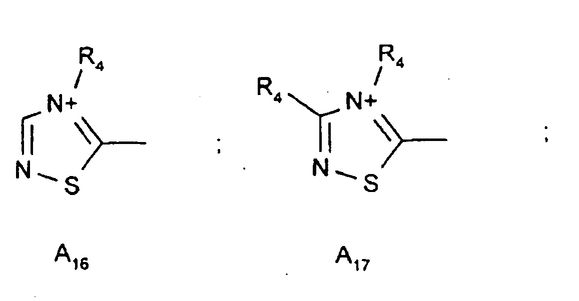
und 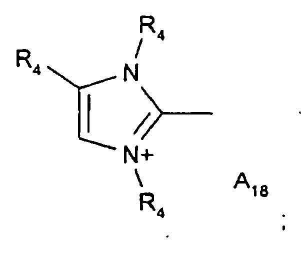 worin die Gruppe R₄ eine C₁₋₄-Alkylgruppe bedeutet, die mit einer Hydroxygruppe substituiert sein kann, und R₅ eine C₁₋₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von
Alkoxy verschieden ist; 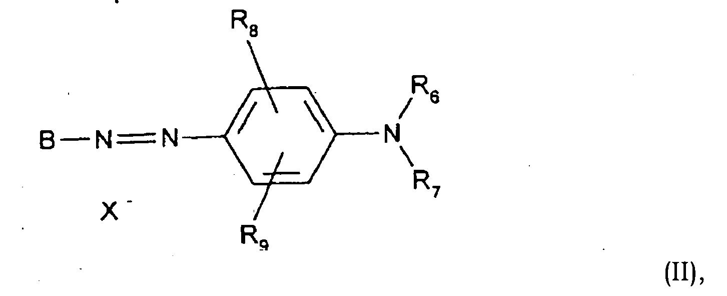 wobei in der Formel (II) bedeuten:
**R₆** ein Wasserstoffatom oder eine C₁₋₄-Alkylgruppe,
**R₇** ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
**R₈** und **R₉,** die gleich oder verschieden sind, ein Wasserstoffatom, ein Halogenatom, wie Brom, Chlor, Iod oder
Fluor, eine C₁₋₄-Alkylgruppe, eine C₁₋₄-Alkoxygruppe oder die Gruppe -CN,
**X⁻** ein Anion, das vorzugsweise unter Chlorid, Methylsulfat und
Acetat ausgewählt ist,
**B** eine Gruppe, die unter den folgenden Strukturen B1 bis B6 ausgewählt ist: 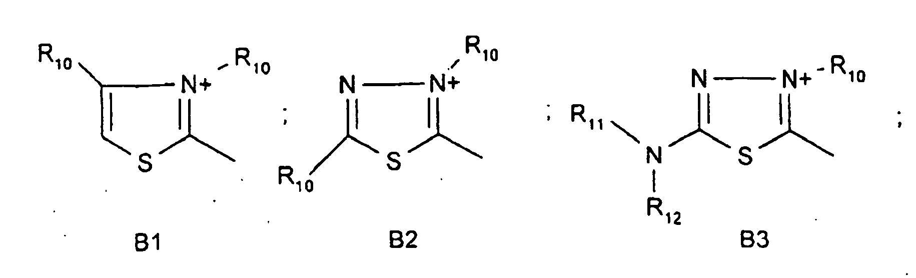 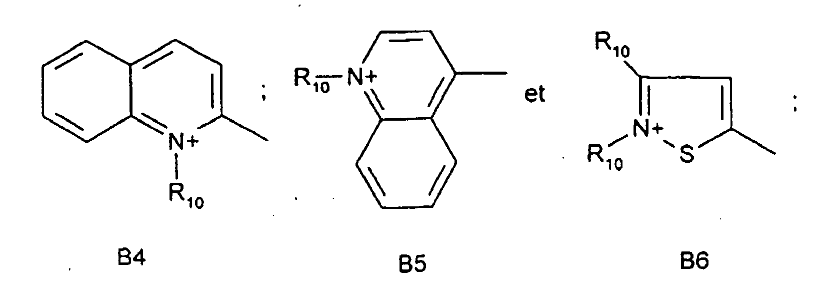
worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten; 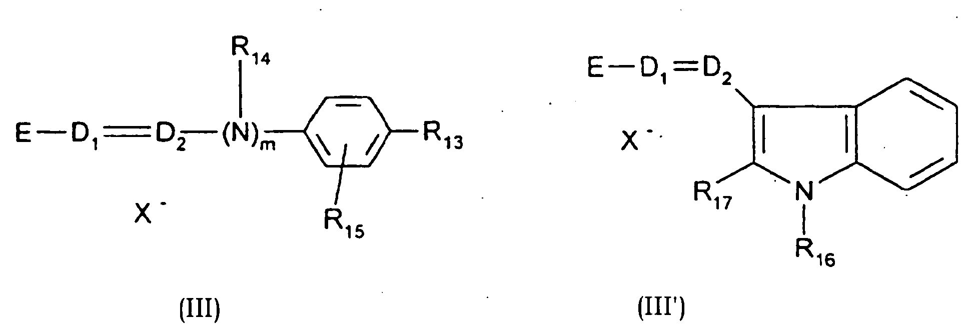 wobei in den Formeln (III) und (III') bedeuten:
**R₁₃** ein Wasserstoffatom, eine C₁₋₄-Alkoxygruppe, ein Halogenatom, wie Brom, Chlor, Iod oder Fluor, oder eine Aminogruppe,
**R₁₄** ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist;
**R₁₅** ein Wasserstoffatom oder ein Halogenatom, wie Brom, Chlor,
Iod oder Fluor,
**R₁₆** und **R₁₇,** die identisch oder voneinander verschieden sind,
Wasserstoff oder C₁₋₄-Alkyl,
**D₁** und **D₂,** die gleich oder verschieden sind, ein Stickstoffatom oder die Gruppe -CH,
**m** = 0 oder 1, mit der Maßgabe, dass die Gruppen D₁ und D₂ gleichzeitig -CH bedeuten und m = 0, wenn R₁₃ eine unsubstituierte Aminogruppe ist,
**X⁻** ein Anion, das vorzugsweise unter Chlorid, Methylsulfat und
Acetat ausgewählt ist,
**E** eine Gruppe, die unter den folgenden Strukturen E1 bis E8 ausgewählt ist: 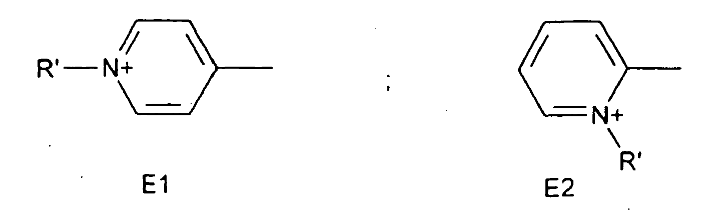 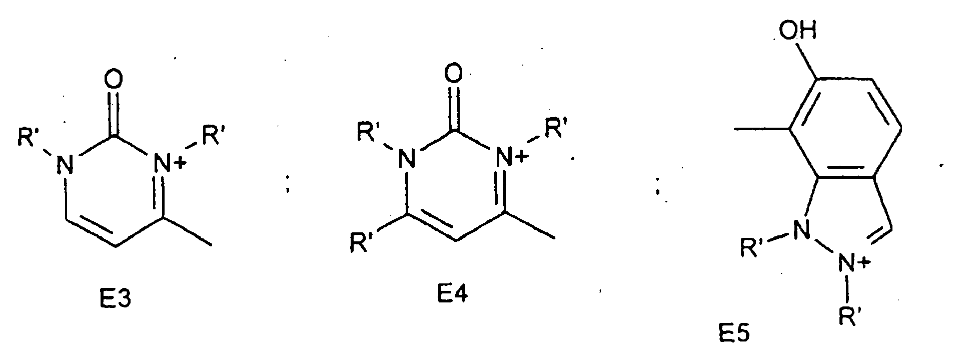 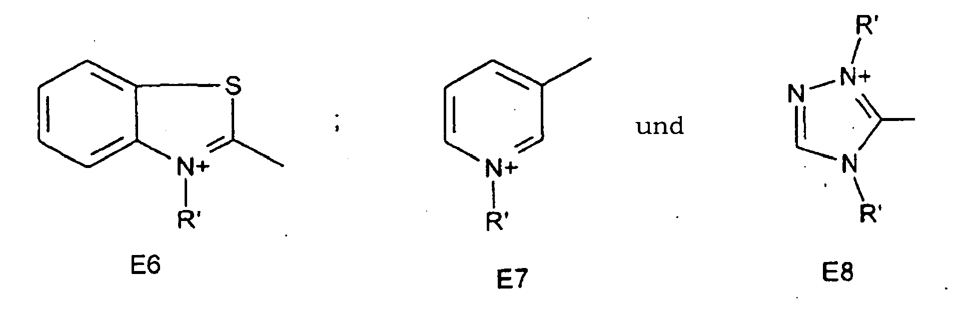
wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 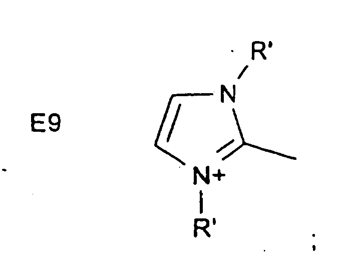 wobei R' eine C₁₋₄-Alkylgruppe bedeutet;
wobei die Zusammensetzung **dadurch gekennzeichnet ist, dass** sie ferner **(ii)** mindestens ein Polyol und/oder mindestens einen aliphatischen C₁₋₈-Ether eines C₃₋₉-Polyols und/oder aromatischen C₆₋₈-Ether eines C₂₋₉-Polyols enthält, die unter 1,3-Propandiol, 2-Buten-1,4-diol, Pentan-1,5-diol, 2,2-Dimethylpropan-1,3-diol, 3-Methyl-pentan-1,5-diol, Pentan-1,2-diol, 2,2,4,-Trimetyhl-pentan-1,3-diol, 2-Methyl-propan-1,3-diol, Hexylenglykol, 1,3-Butylenglykol, Diethylenglykol, Triethylenglykol, Propylenglykolmonoethylether, Isopropylenglykoldimethylether, Dipropylenglykolmonomethylether, Tripropylenglykolmonomethylether, Diethylenglykoldimethylether, Ethylenglykolmonophenylether, Ethylenglykolmonobenzylether, Propylenglykolmonophenylether, Propylenglykolmonobenzylether, Diethylenglykolmonophenylether und Diethylenglykolmonobenzylether ausgewählt sind.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

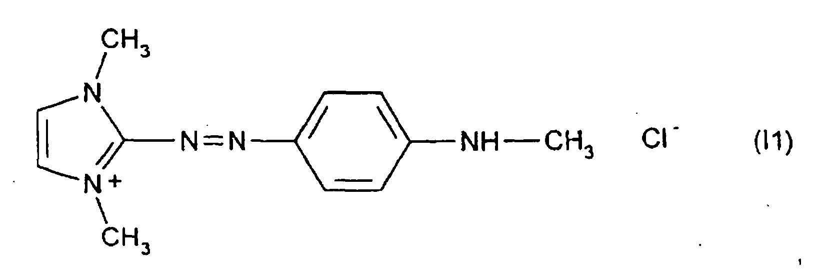

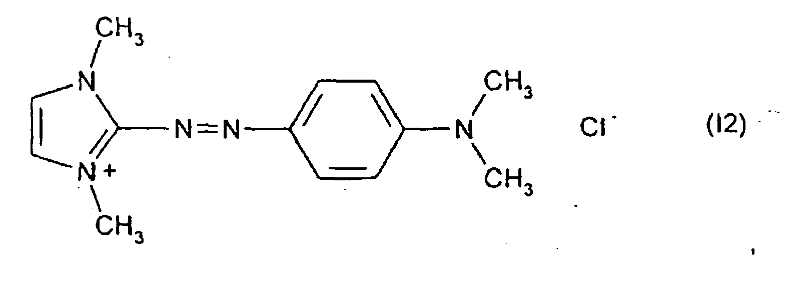

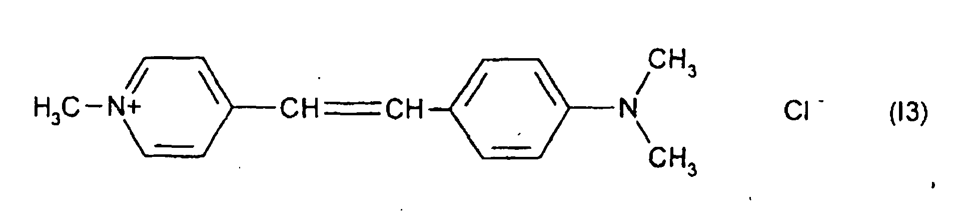

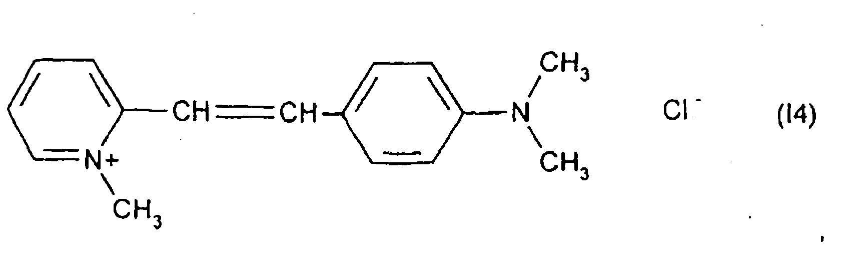

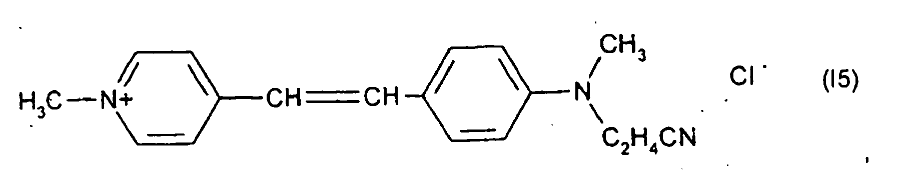

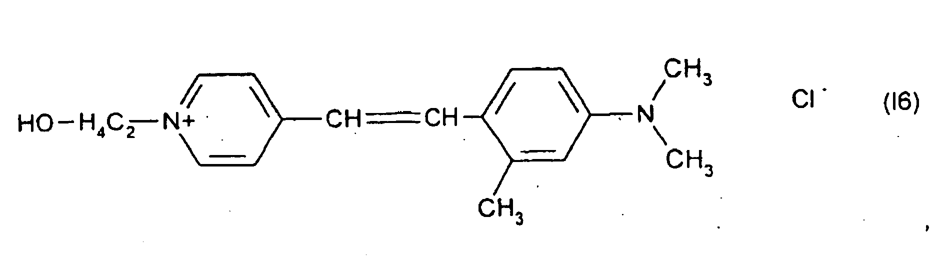

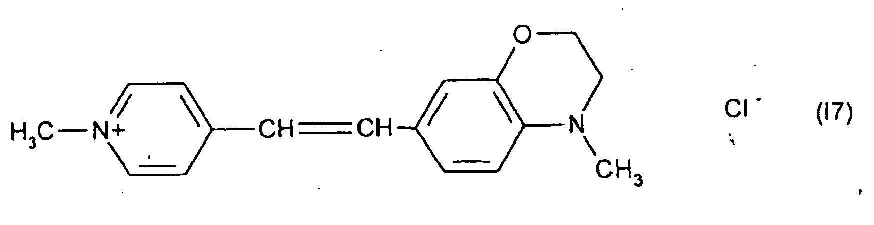

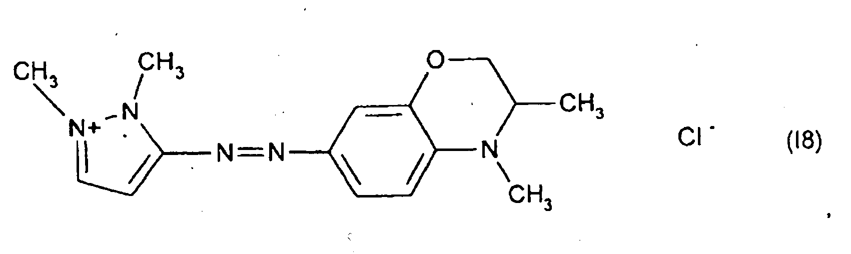

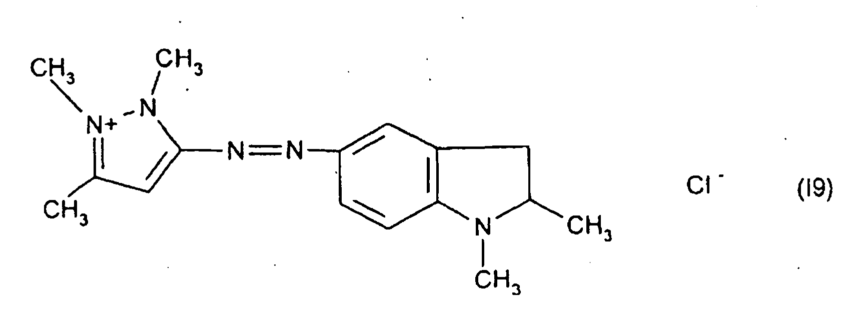

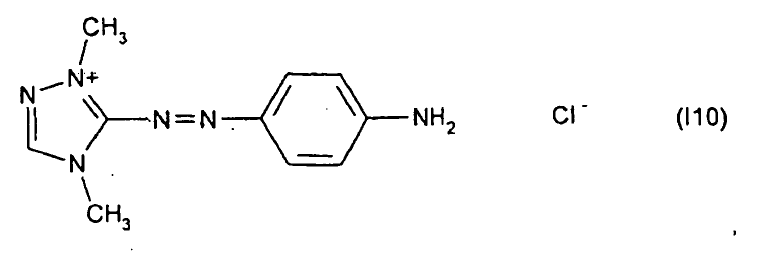

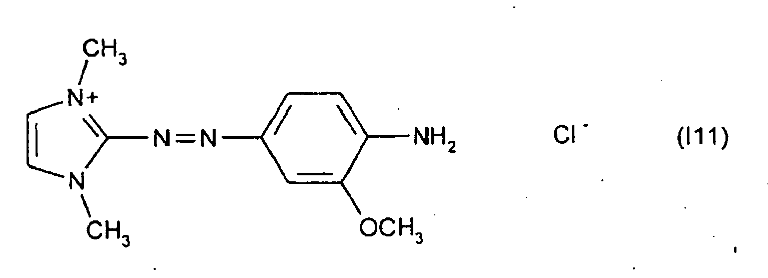

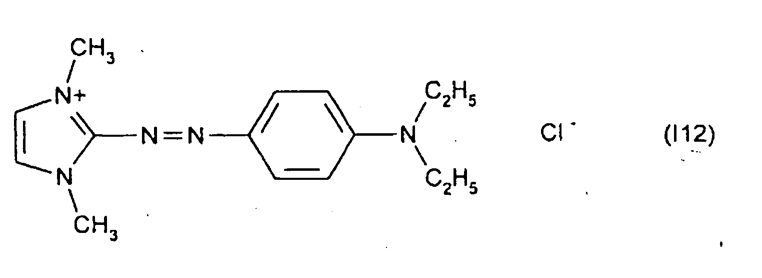

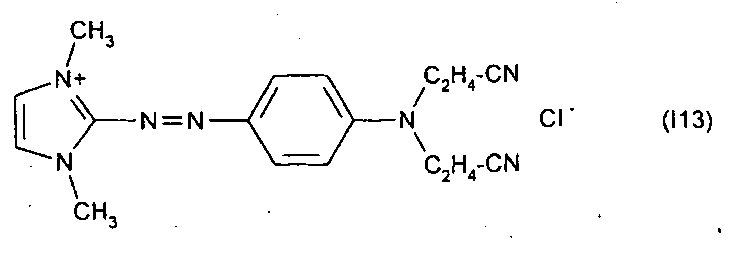

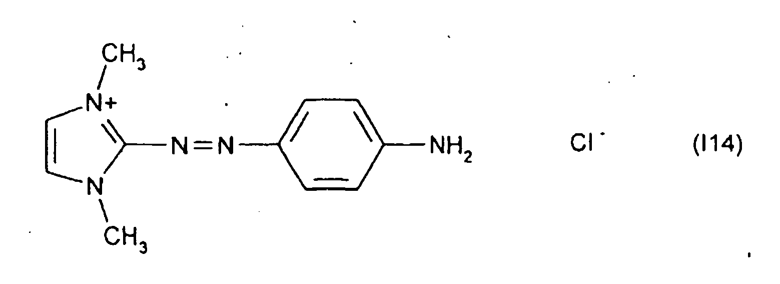

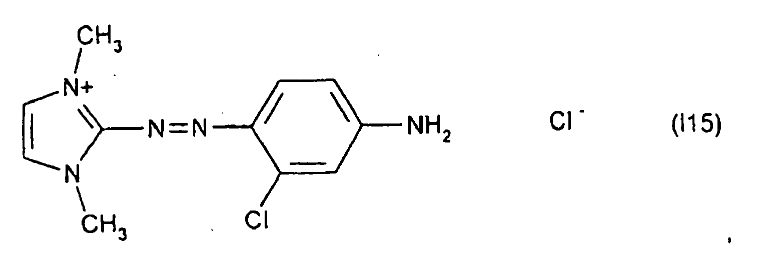

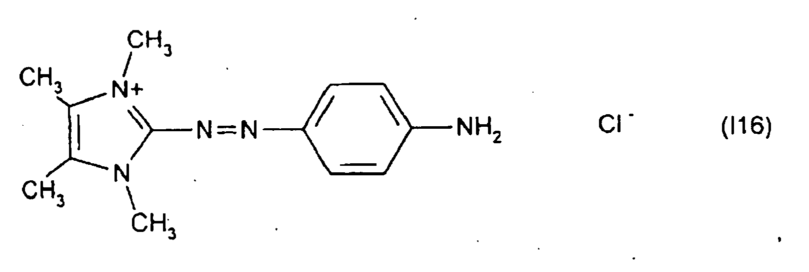

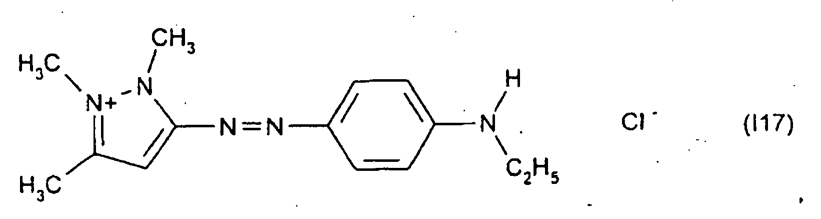

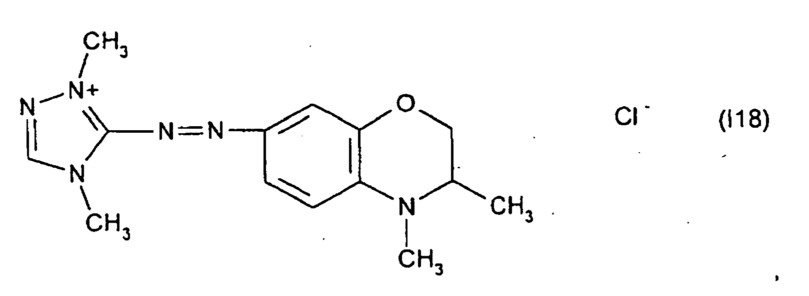

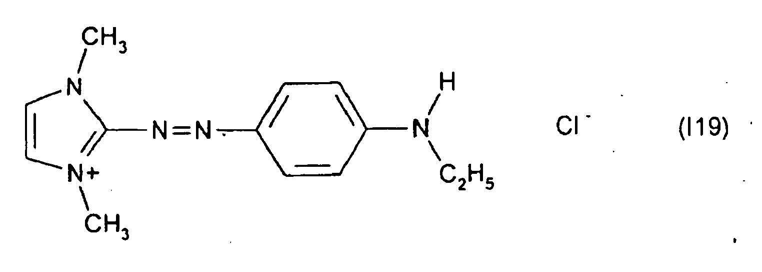

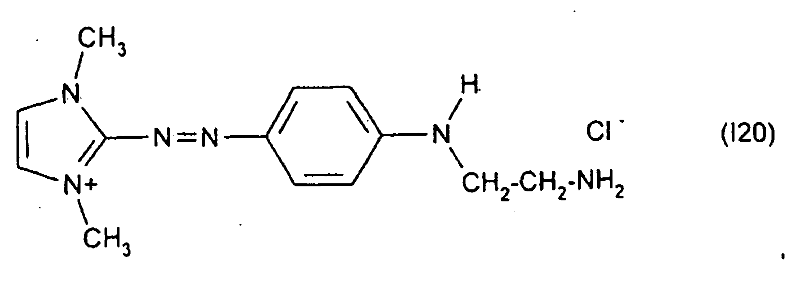

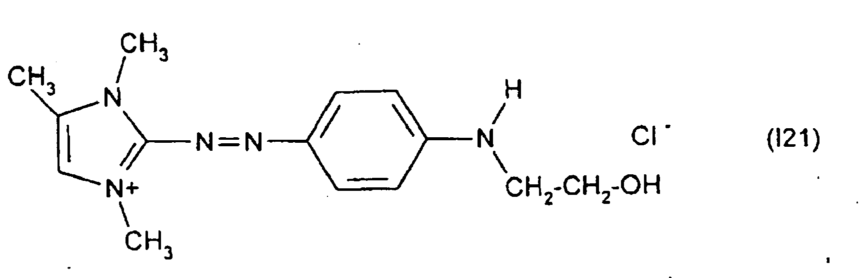

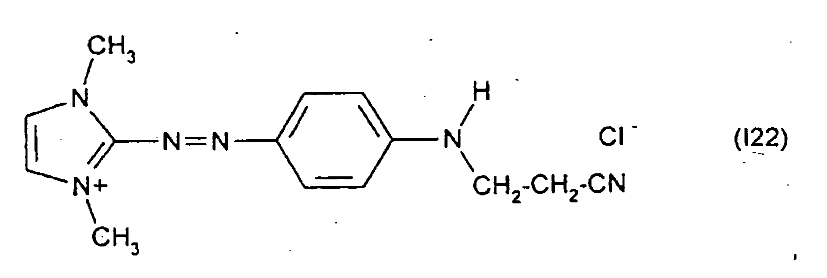

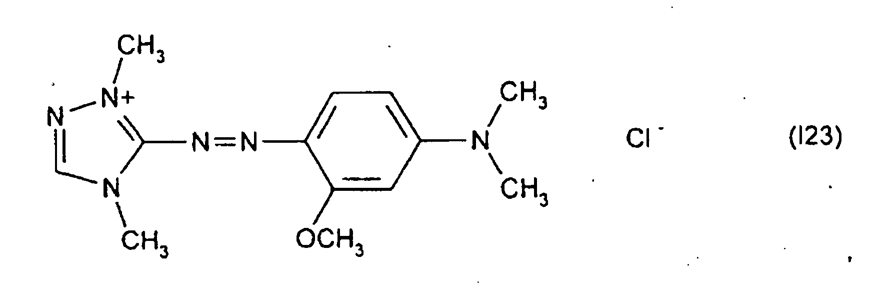

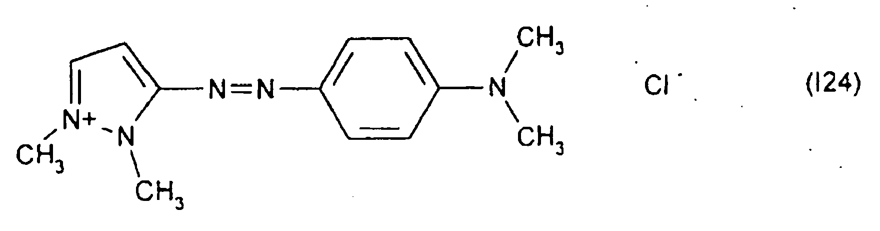

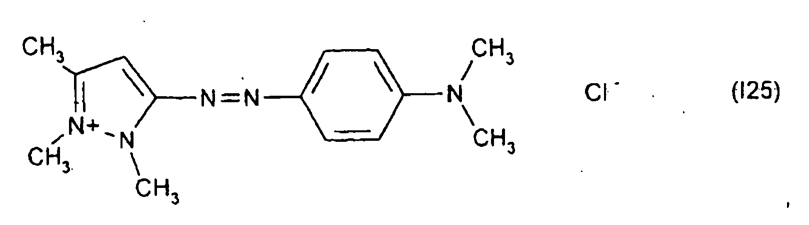

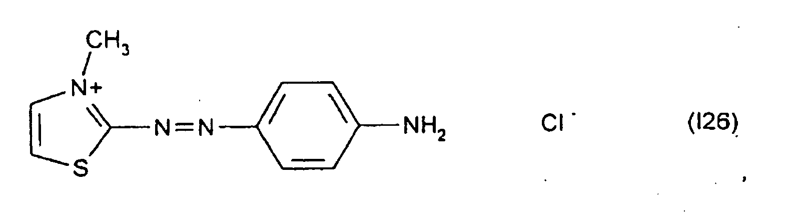

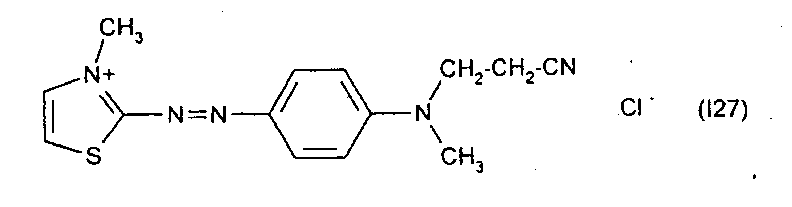

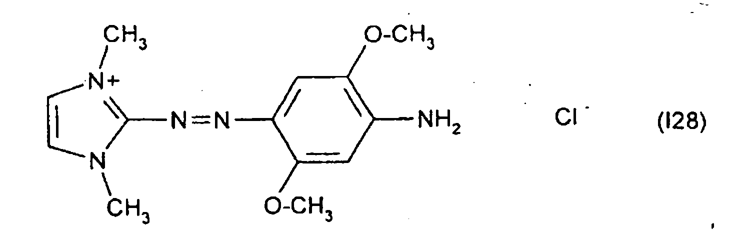

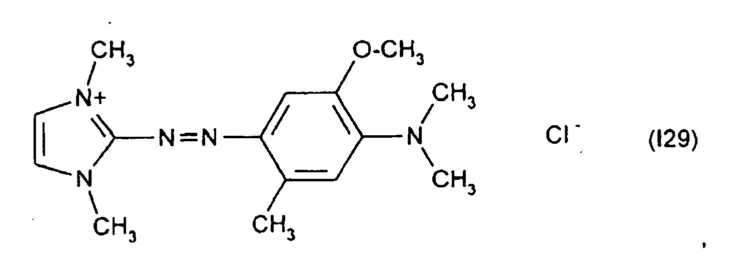

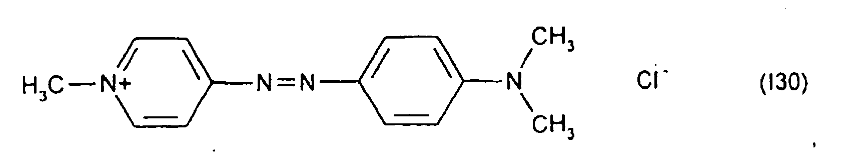

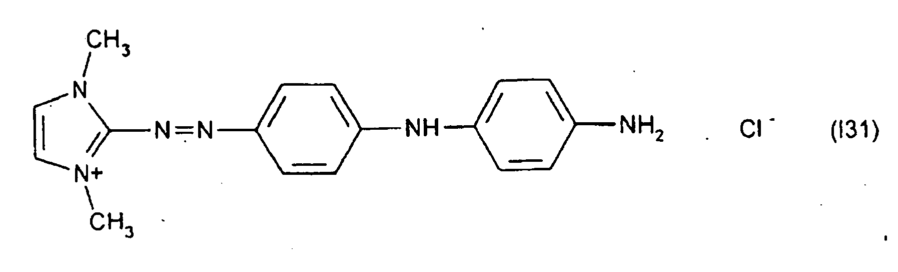

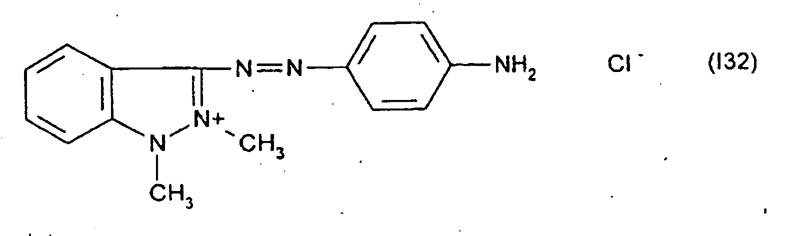

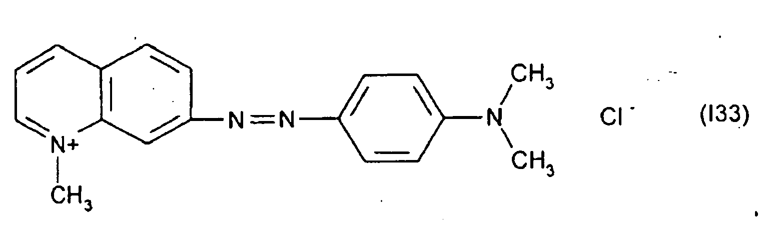

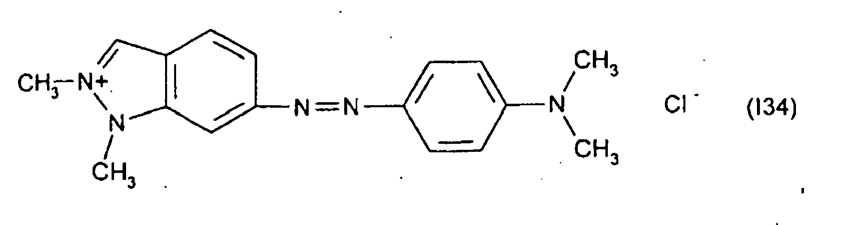

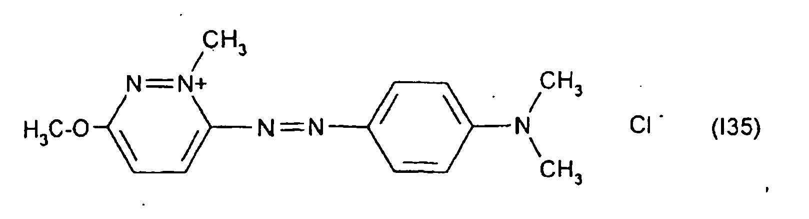

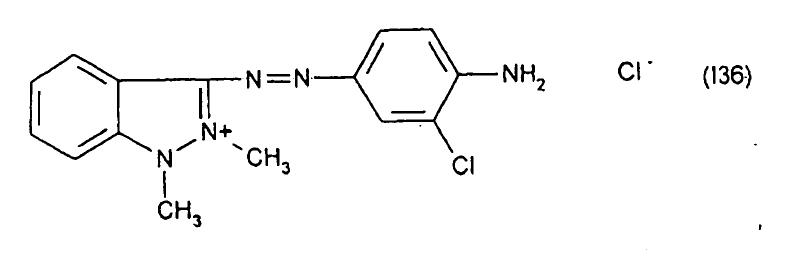

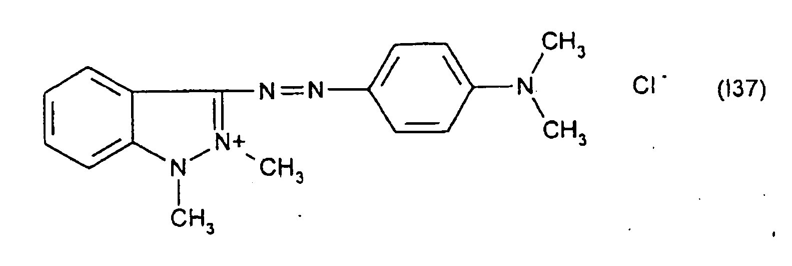

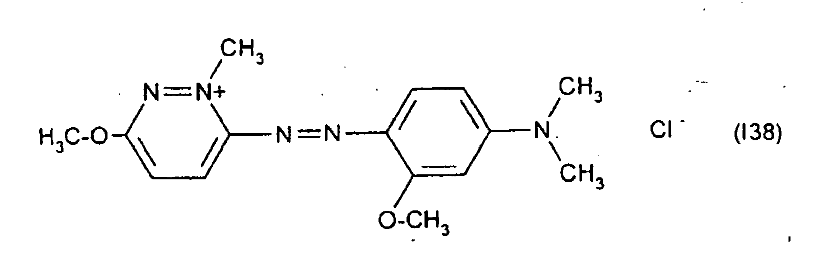

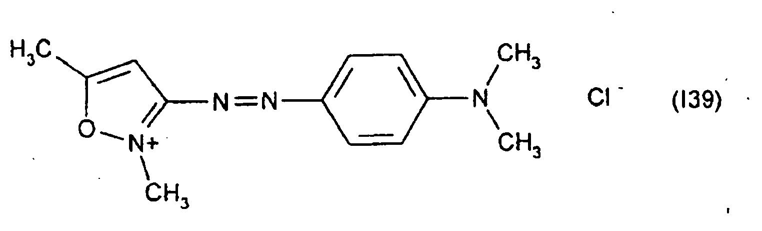

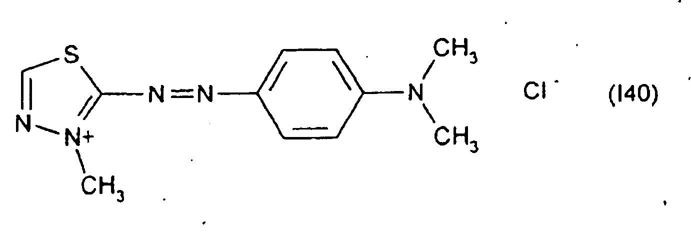

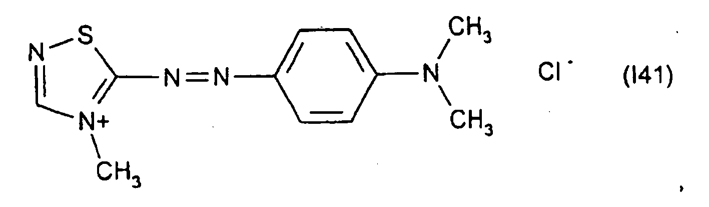

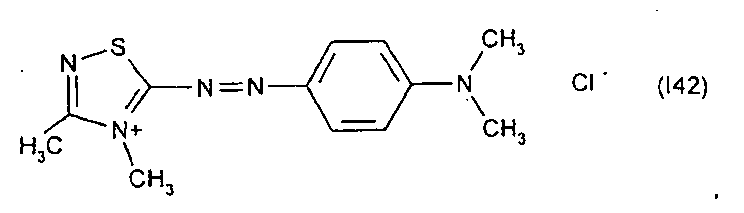

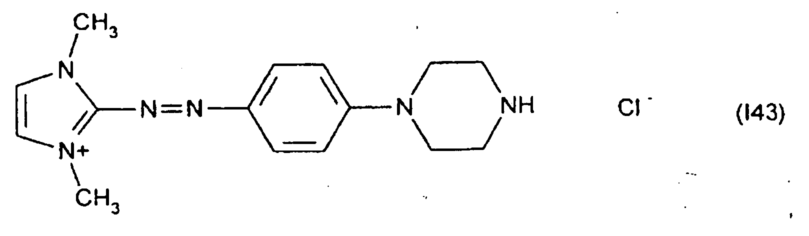

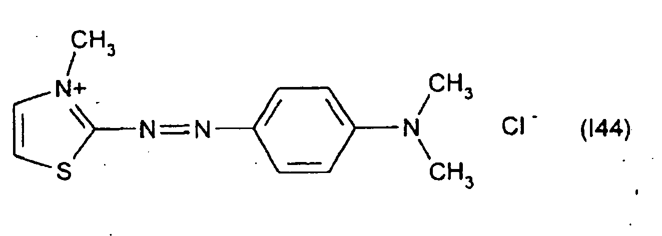

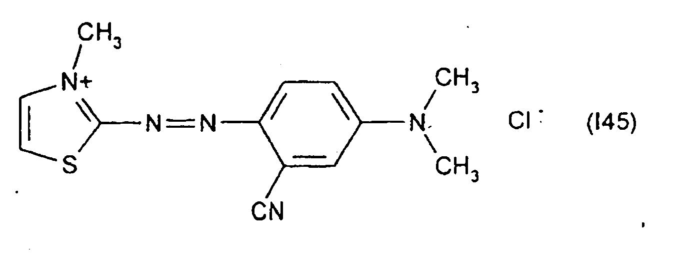

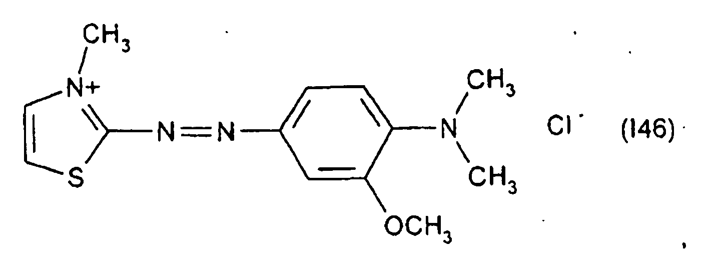

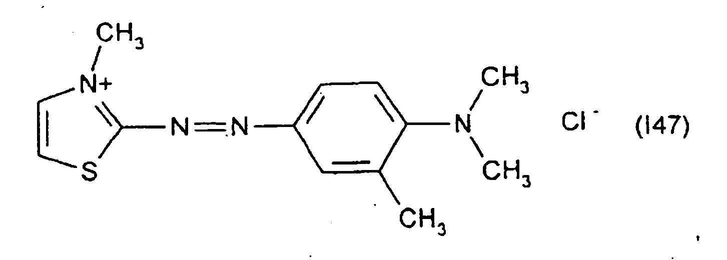

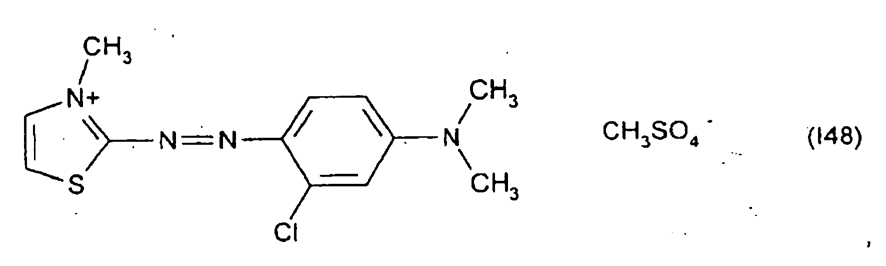

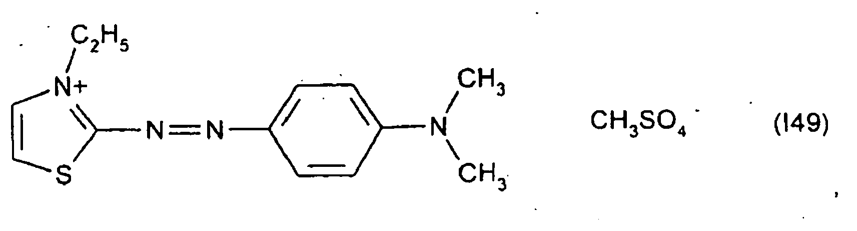

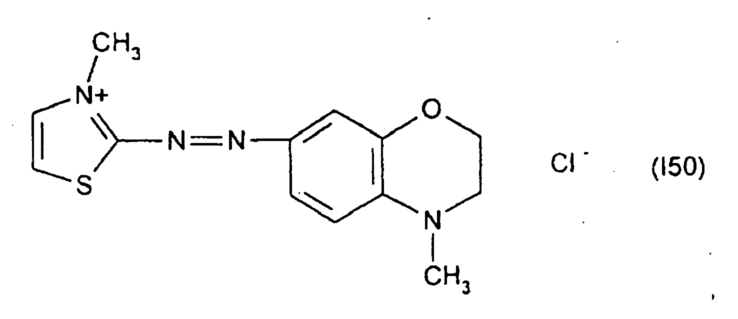

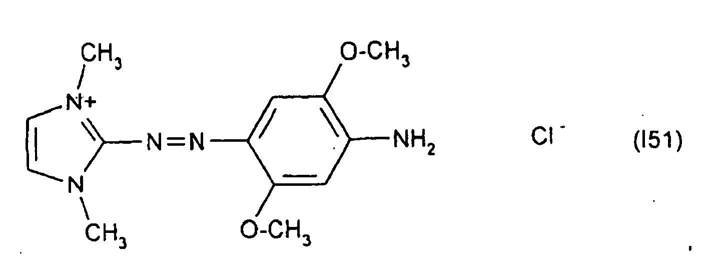

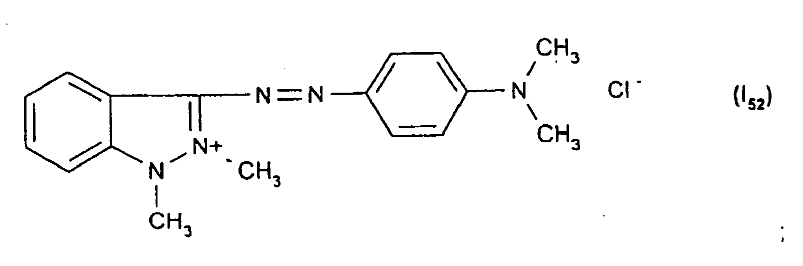

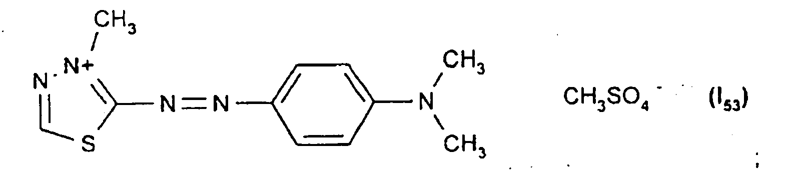

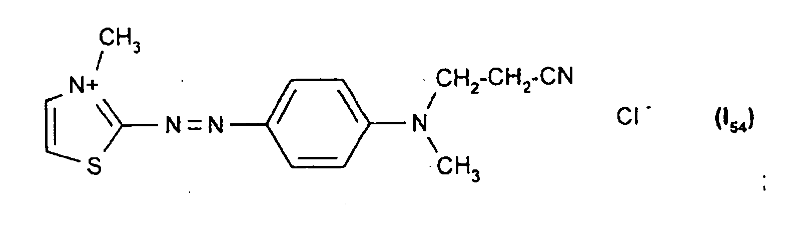

3. Zusammensetzung nach Anspruch 2, **dadurch gekennzeichnet, dass** die kationischen Direktfarbstoffe den Strukturen (I1), (I2), (I14) und (I11) entsprechen.

4. Zusammensetzung nach Anspruch 1, **dadurch gekennzeichnet, dass** die kationischen Direktfarbstoffe der Formel (II) unter den Verbindungen der folgenden Strukturen (II1) bis (II9) ausgewählt sind: 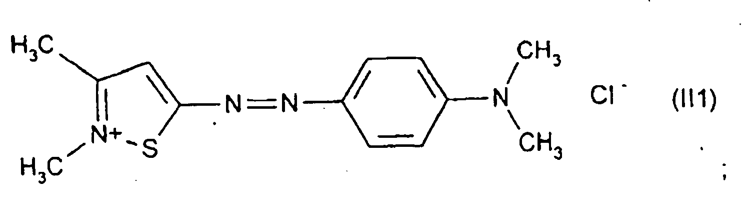 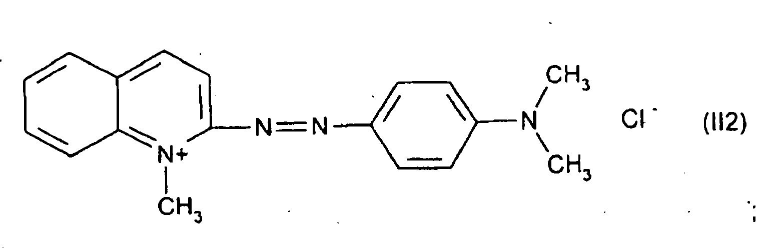 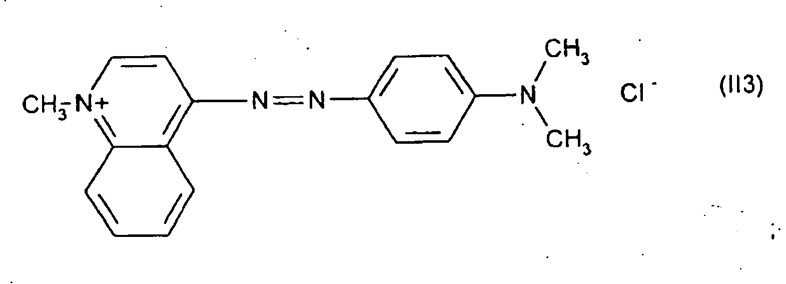 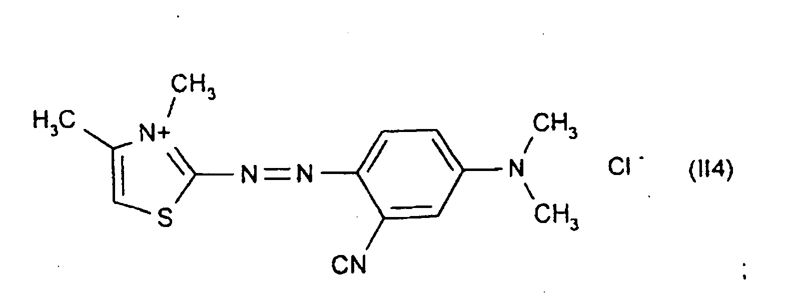 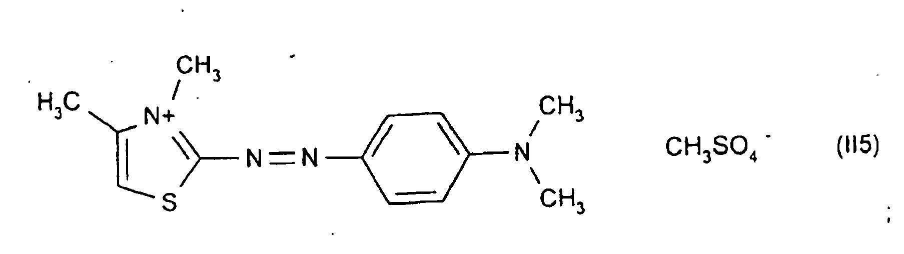 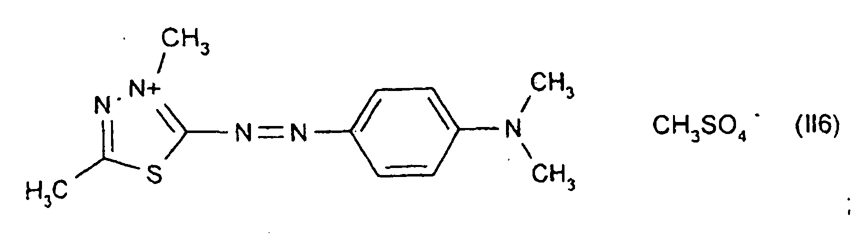 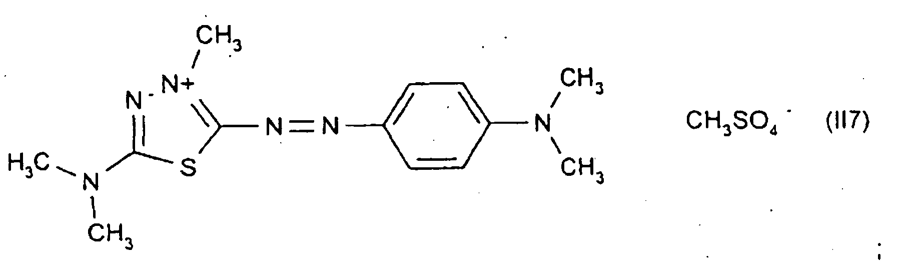 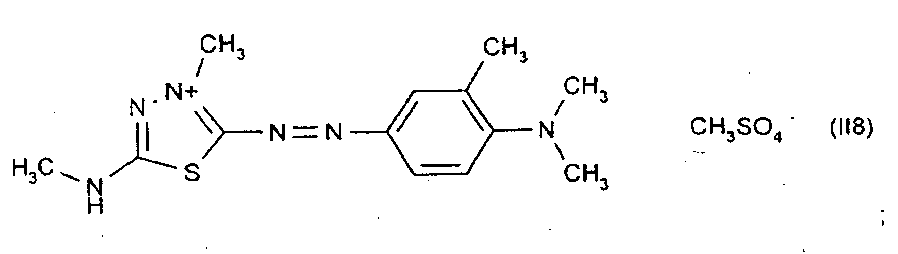 und

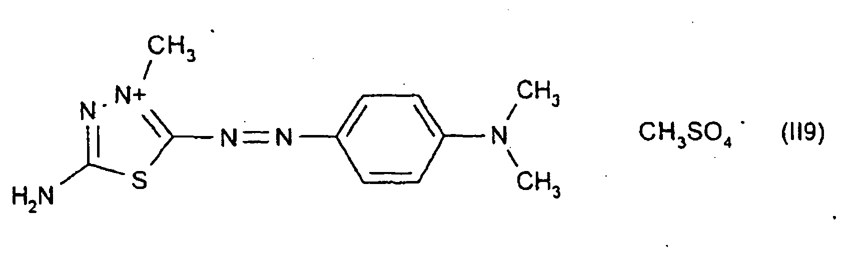

5. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind: 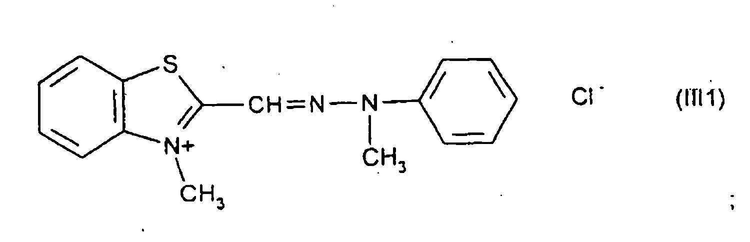 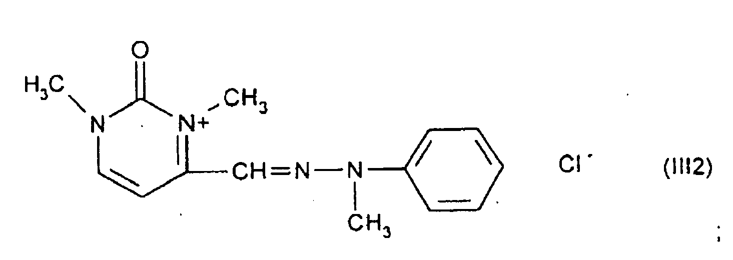 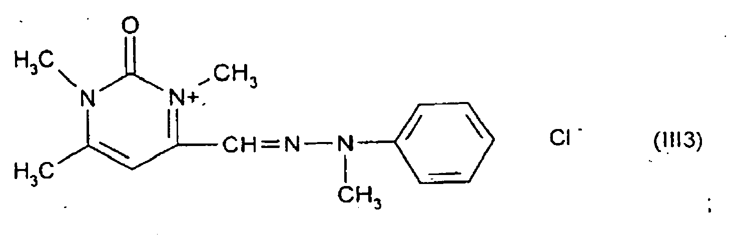 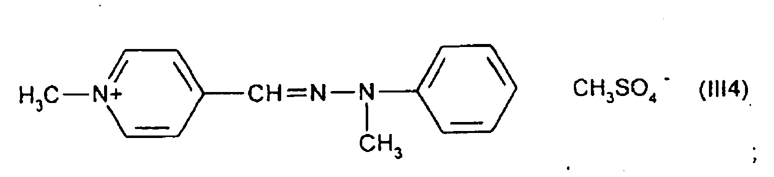 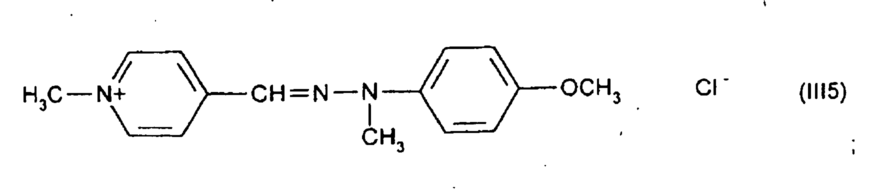 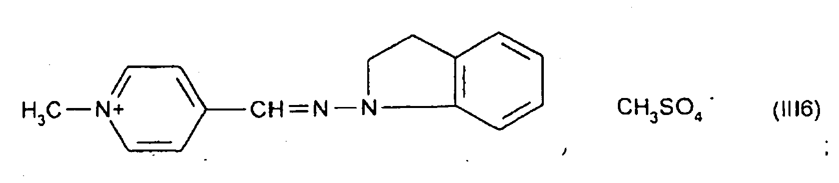 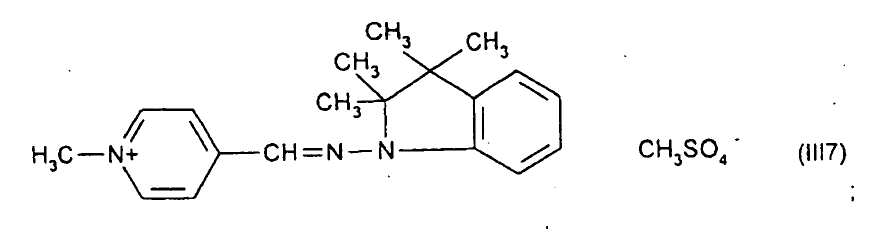 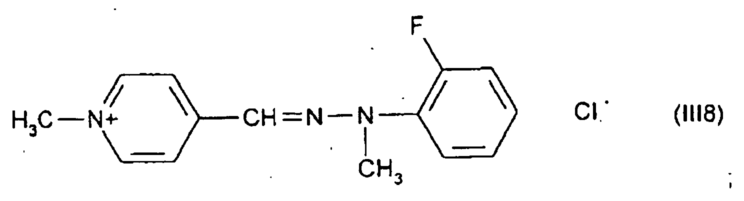 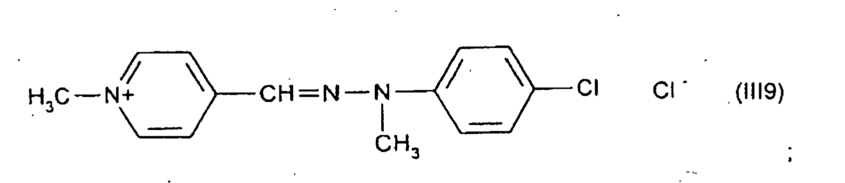 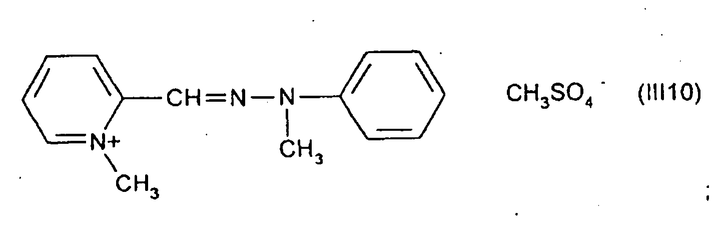 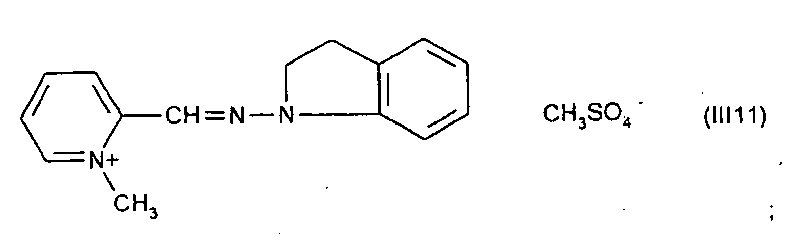 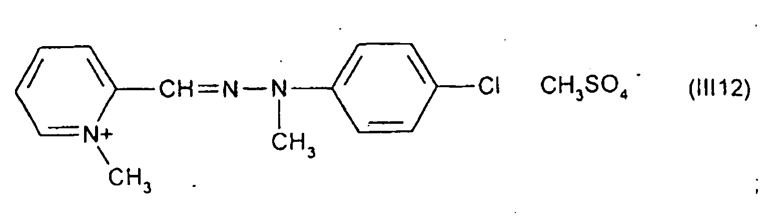 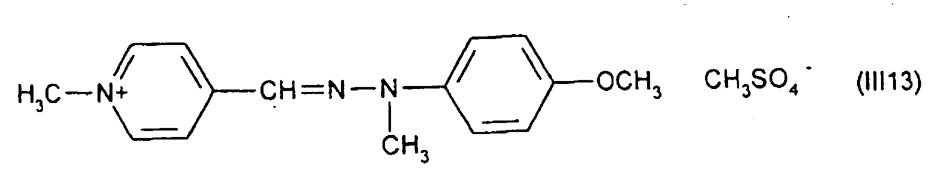 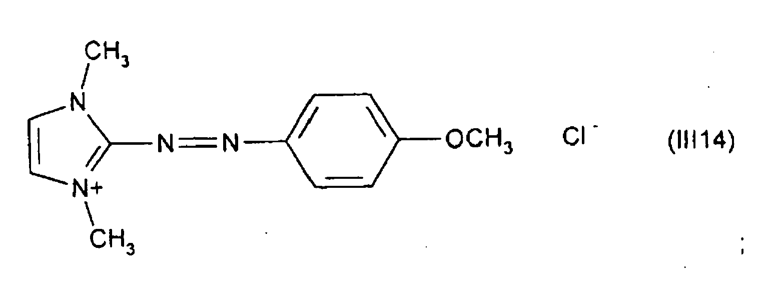 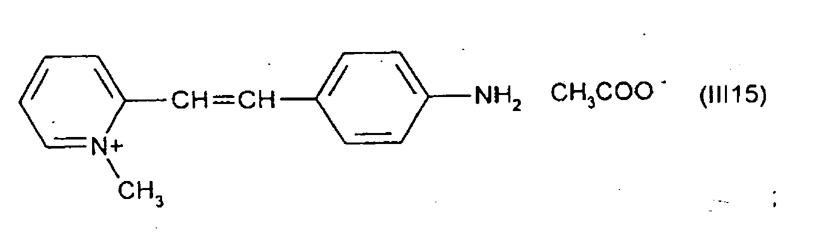 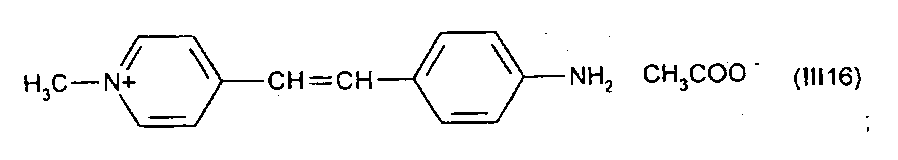 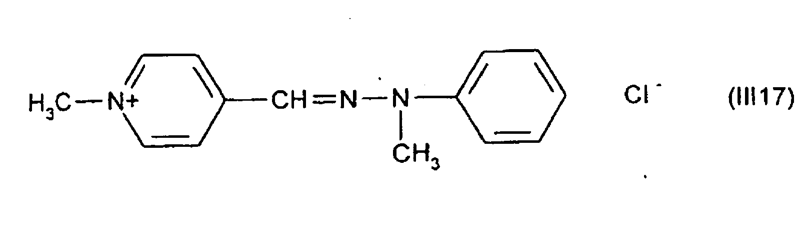 und

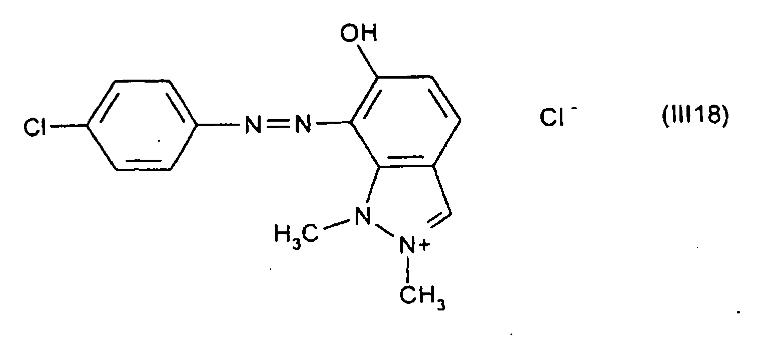

6. Zusammensetzung nach Anspruch 5, **dadurch gekennzeichnet, dass** die kationischen Direktfarbstoffe der Formel (III) den Strukturen (III4), (III5) und (III13) entsprechen.

7. Zusammensetzung nach Anspruch 1, **dadurch gekennzeichnet, dass** die kationischen Direktfarbstoffe der Formel (III') unter den Verbindungen der folgenden Strukturen (III'1) bis (III'3) ausgewählt sind: 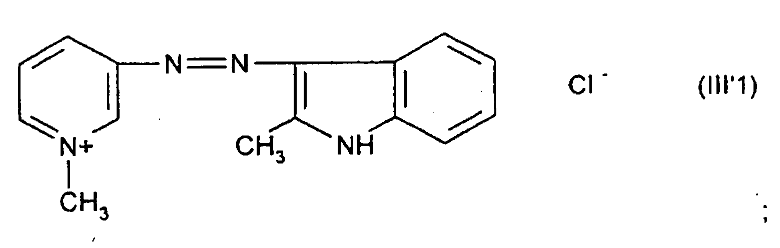 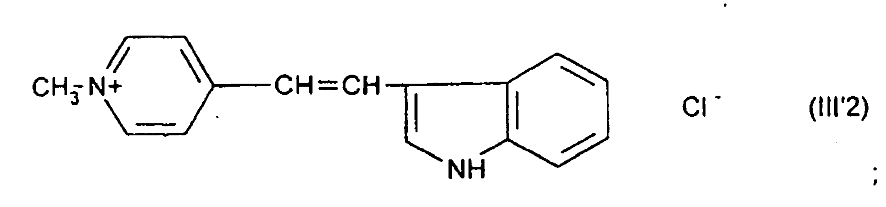 und

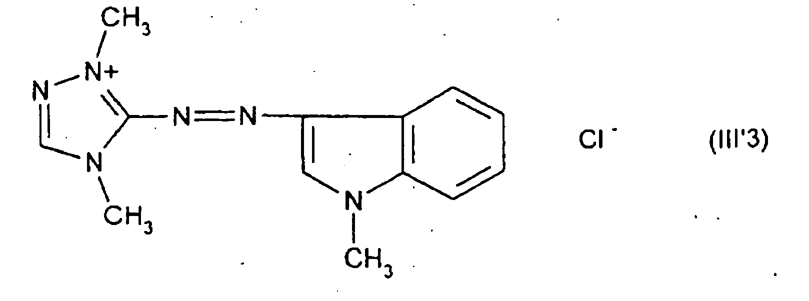

8. Zusammensetzung nach einem der vorhergehenden Ansprüche, **dadurch gekennzeichnet, dass** der oder die kationische(n) Direktfarbstoff(e) der Formeln (I), (II), (III) oder (III') 0,001 bis 10 Gew.-% des Gesamtgewichts der Zusammensetzung ausmachen.

9. Zusammensetzung nach Anspruch 8, **dadurch gekennzeichnet, dass** der oder die kationische(n) Direktfarbstoff(e) der Formeln (I), (II), (III) oder (III') 0,005 bis 5 Gew.-% des Gesamtgewichts der Zusammensetzung ausmachen.

10. Zusammensetzung nach einem der vorhergehenden Ansprüche, **dadurch gekennzeichnet, dass** das Polyol und/oder der aliphatische C₁₋₈-Ether eines C₃₋₉-Polyols und/oder aromatische C₆₋₈-Ether eines C₂₋₉-Polyols 0,1 bis 40 Gew.-% des Gesamtgewichts der Zusammensetzung ausmachen.

11. Zusammensetzung nach Anspruch 10, **dadurch gekennzeichnet, dass** das Polyol und/oder der aliphatische C₁₋₈-Ether eines C₃₋₉-Polyols und/oder aromatische C₆₋₈-Ether eines C₂₋₉-Polyols 0,5 bis 20 Gew.-% des Gesamtgewichts der Zusammensetzung ausmachen.

12. Zusammensetzung nach einem der vorhergehenden Ansprüche, **dadurch gekennzeichnet, dass** sie ferner weitere Direktfarbstoffe enthält.

13. Zusammensetzung nach einem der vorhergehenden Ansprüche, **dadurch gekennzeichnet, dass** das zum Färben geeignete Medium (oder der Träger) aus einem Gemisch von Wasser und mindestens einem Lösungsmittel besteht, welches unter den Polyolen und/oder Polyolethern ausgewählt ist, die in Anspruch 1 definiert sind.

14. Zusammensetzung nach Anspruch 13, **dadurch gekennzeichnet, dass** das Medium ferner ein organisches Lösemittel enthält.

15. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

16. Zusammensetzung nach einem der vorhergehenden Ansprüche, **dadurch gekennzeichnet, dass** sie für die aufhellende direktziehende Färbung vorgesehen ist und mindestens ein Oxidationsmittel enthält.

17. Verfahren zum direkten Färben von Keratinfasern und insbesondere menschlichen Keratinfasern, wie dem Haar, **dadurch gekennzeichnet, dass** auf die Fasern mindestens eine Farbmittelzusammensetzung, die in einem zum Färben geeigneten Medium, das keine Oxidasen oder Oxidoreduktasen aufweist, enthält: **(i)** mindestens einen kationischen Direktfarbstoff der folgenden Formeln (I), (II), (III) oder (III'): 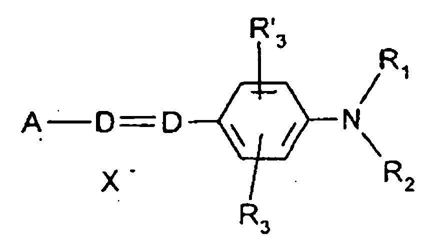 wobei in der Formel (I) bedeuten:
**D** ein Stickstoffatom oder die Gruppe -CH,
**R₁** und **R₂,** die gleich oder verschieden sind, ein Wasserstoffatom; eine C₁₋₄-Alkylgruppe, die mit einer Gruppe - CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder
eine 4'-Aminophenylgruppe,
**R₃** und **R'₃,** die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder
Acetyloxy,
**X⁻** ein Anion, das vorzugsweise unter Chlorid, Methylsulfat und
Acetat ausgewählt ist,
**A** eine Gruppe, die unter den folgenden Strukturen A1 bis A18 ausgewählt ist: 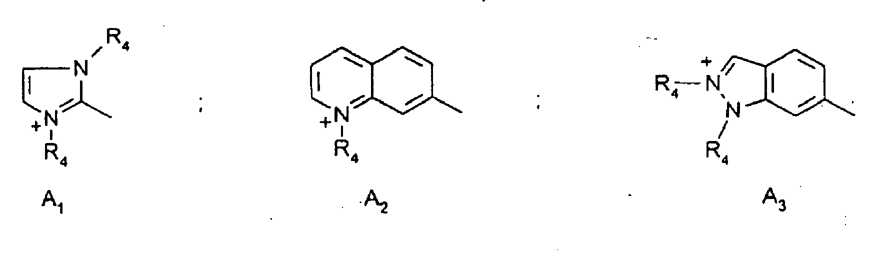 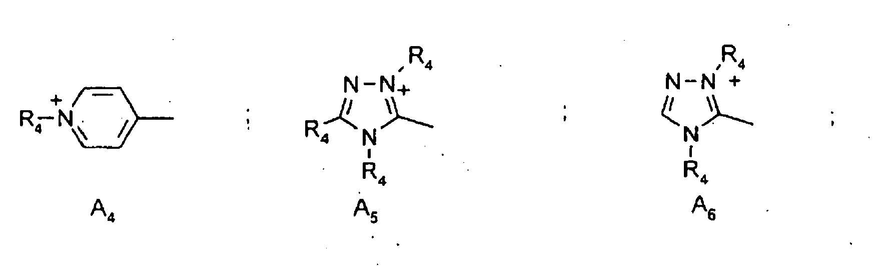 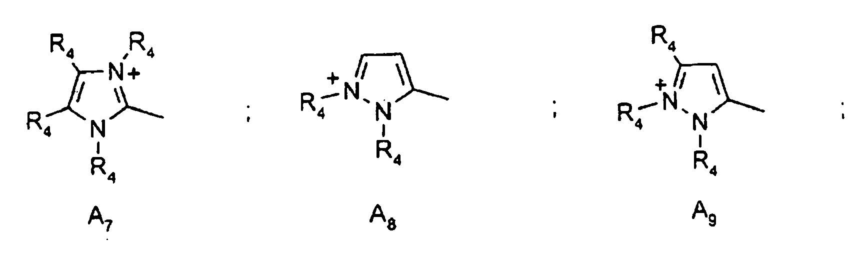 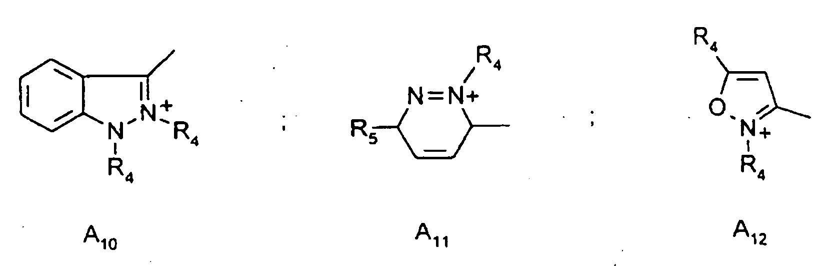 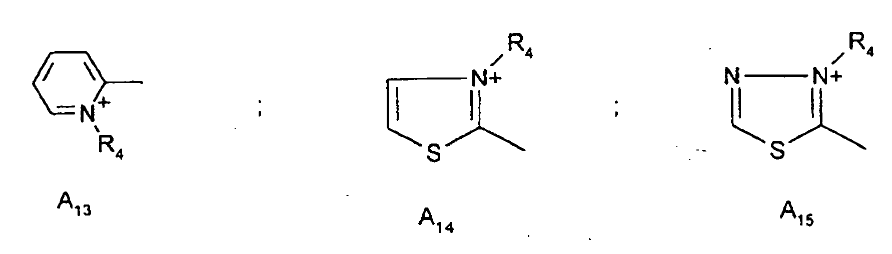 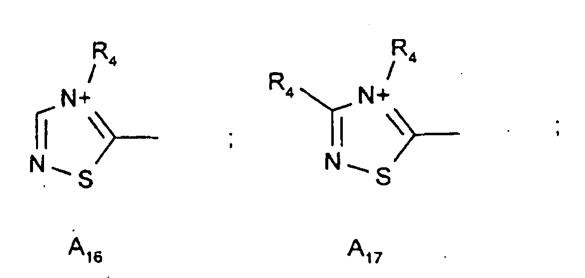
und 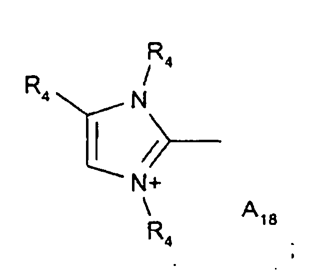 worin die Gruppe R₄ eine C₁₋₄-Alkylgruppe bedeutet, die mit einer Hydroxygruppe substituiert sein kann, und R₅ eine C₁₋₄-Alkoxygruppe ist, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist; 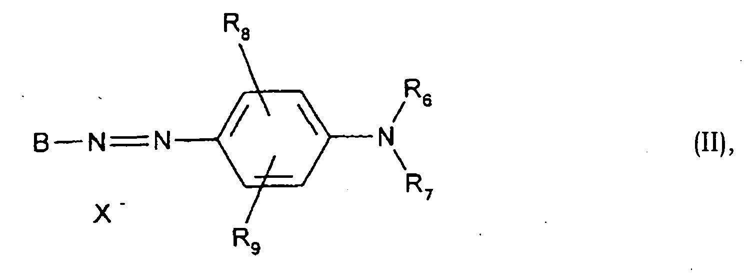 wobei in der Formel (II) bedeuten:
**R₆** ein Wasserstoffatom oder eine C₁₋₄-Alkylgruppe,
**R₇** ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
**R₈** und **R₉,** die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
**X⁻** ein Anion, das vorzugsweise unter Chlorid, Methylsulfat und Acetat ausgewählt ist,
**B** eine Gruppe, die unter den folgenden Strukturen B1 bis B6 ausgewählt ist: 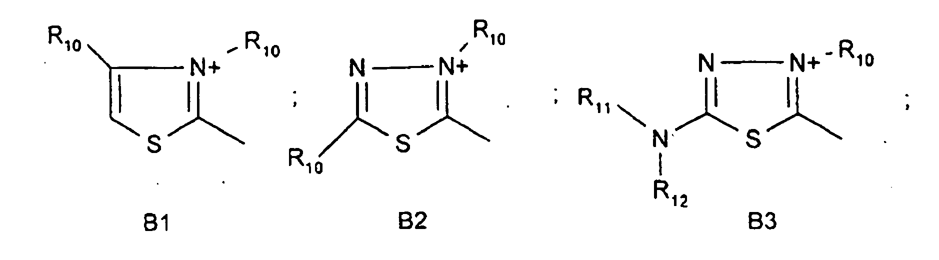 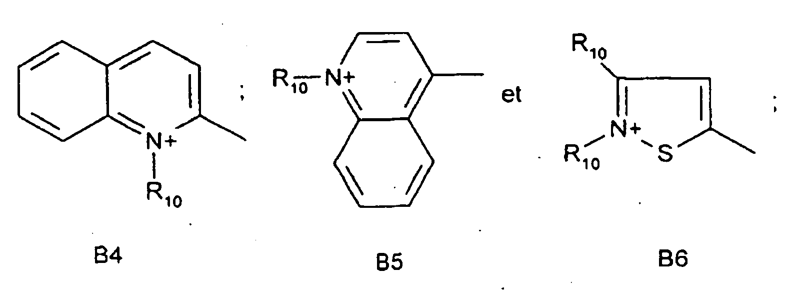
worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten; 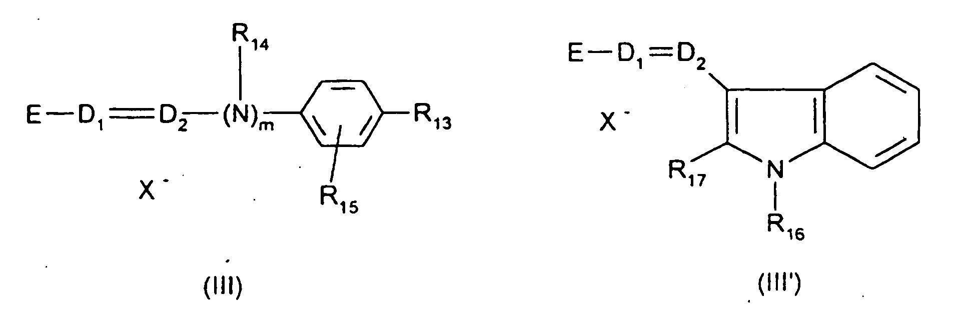 wobei in den Formeln (III) und (III') bedeuten:
**R₁₃** ein Wasserstoffatom, eine C₁₋₄-Alkoxygruppe, ein Halogenatom, wie Brom, Chlor, Iod oder Fluor, oder eine Aminogruppe,
**R₁₄** ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist;
**R₁₅** ein Wasserstoffatom oder ein Halogenatom, wie Brom, Chlor, Iod oder Fluor,
**R₁₆** und **R₁₇,** die identisch oder voneinander verschieden sind, Wasserstoff oder C₁₋₄-Alkyl,
**D₁** und **D₂,** die gleich oder verschieden sind, ein Stickstoffatom oder die Gruppe -CH,
**m** = 0 oder 1, mit der Maßgabe, dass die Gruppen D₁ und D₂ gleichzeitig -CH bedeuten und m = 0, wenn R₁₃ eine unsubstituierte Aminogruppe ist,
**X⁻** ein Anion, das vorzugsweise unter Chlorid, Methylsulfat und
Acetat ausgewählt ist,
**E** eine Gruppe, die unter den folgenden Strukturen E1 bis E8 ausgewählt ist: 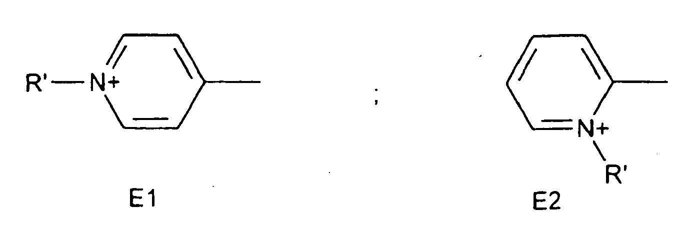 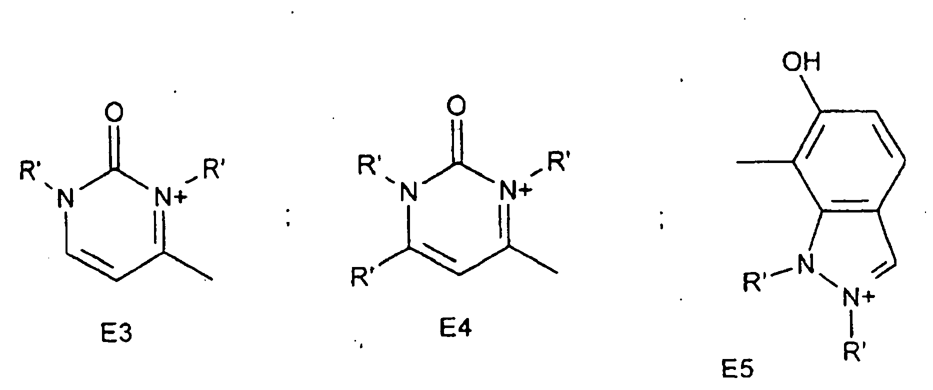 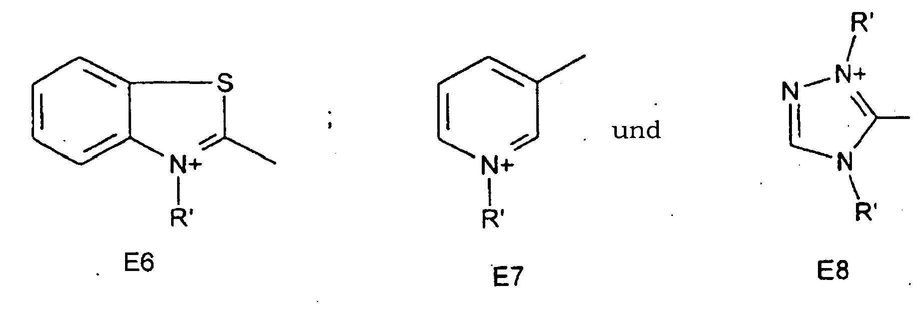
wenn m = 0 und D₁ ein Stickstoffatom bedeutet, kann die Gruppe E auch eine Gruppe der folgenden Struktur E9 sein: 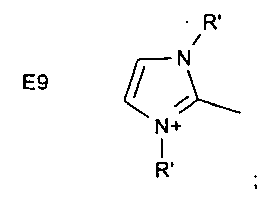 wobei R' eine C₁₋₄-Alkylgruppe bedeutet;
wobei die Zusammensetzung **dadurch gekennzeichnet ist, dass** sie ferner **(ii)** mindestens ein Polyol und/oder mindestens einen aliphatischen C₁₋₈-Ether eines C₃₋₉-Polyols und/oder aromatischen C₆₋₈-Ether eines C₂₋₉-Polyols enthält und wie in Anspruch 2 bis 16 definiert ist, während einer Zeitspanne aufgebracht wird, die zur Bildung der gewünschten Färbung ausreichend ist, und dann gespült, gegebenenfalls mit Haarwaschmittel gewaschen, nochmals gespült und getrocknet wird.

18. Verfahren zum direkten Färben von Keratinfasern und insbesondere menschlichen Keratinfasern, wie Haaren, **dadurch gekennzeichnet, dass** auf die Fasern mindestens eine Farbmittelzusammensetzung, die in einem zum Färben geeigneten Medium, das keine Oxidasen oder Oxidoreduktasen aufweist, enthält: **(i)** mindestens einen kationischen Direktfarbstoff der folgenden Formeln (I), (II), (III) oder (III'): 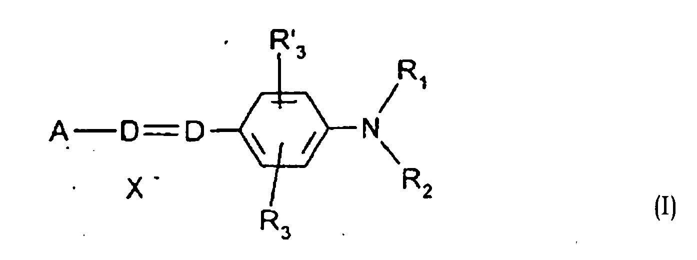 wobei in der Formel (I) bedeuten:
**D** ein Stickstoffatom oder die Gruppe -CH,
**R₁** und **R₂,** die gleich oder verschieden sind, ein Wasserstoffatom; eine C₁₋₄-Alkylgruppe, die mit einer Gruppe - CN, -OH oder -NH₂ substituiert sein kann oder mit einem Kohlenstoffatom des Benzolrings einen gegebenenfalls sauerstoff- oder stickstoffhaltigen Heterocyclus bilden kann, der mit einer oder mehreren C₁₋₄-Alkylgruppen substituiert sein kann; oder eine 4'-Aminophenylgruppe,
**R₃** und **R'₃,** die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder
Acetyloxy,
**X⁻** ein Anion, das vorzugsweise unter Chlorid, Methylsulfat und
Acetat ausgewählt ist,
**A** eine Gruppe, die unter den folgenden Strukturen A1 bis A18 ausgewählt ist: 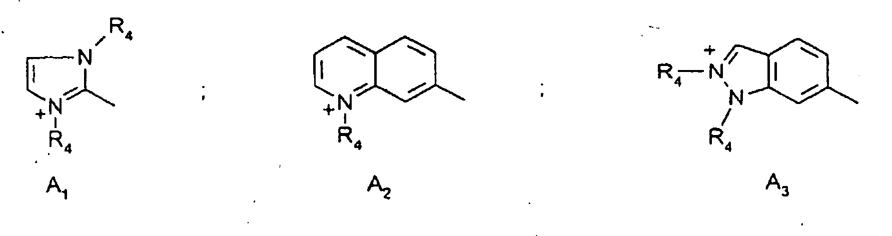 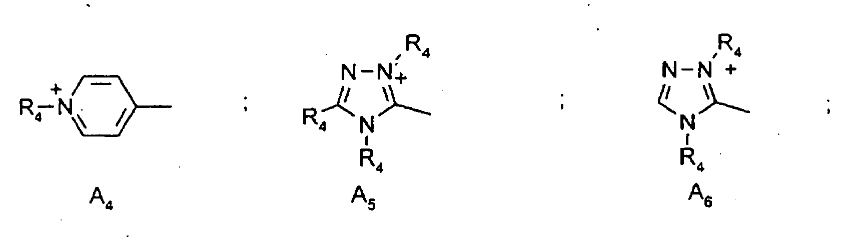 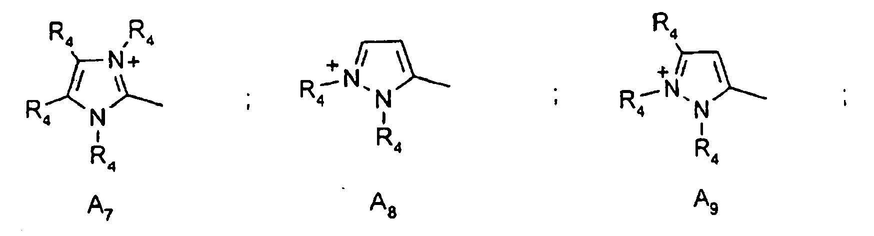 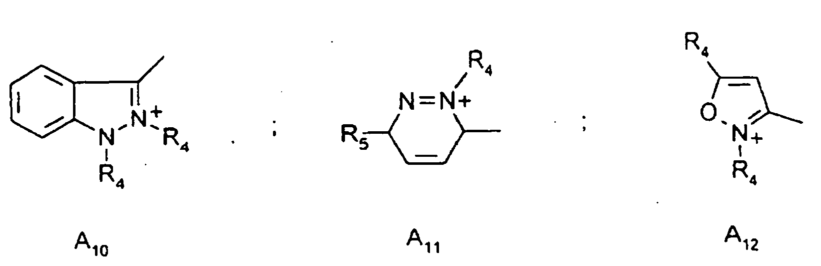 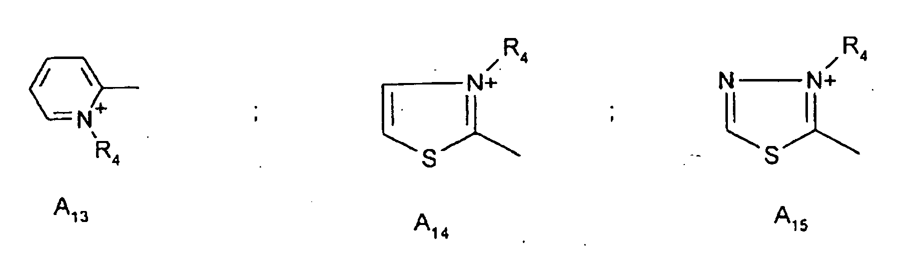 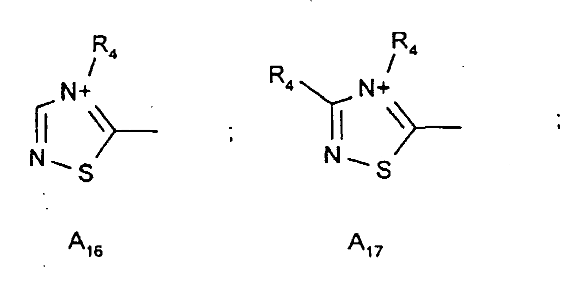
und 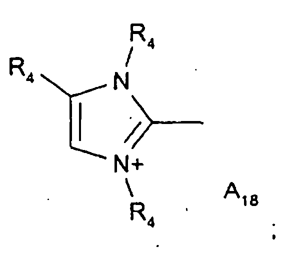 worin die Gruppe R₄eine C₁₋₄-Alkylgruppe bedeutet, die mit einer Hydroxygruppe substituiert sein kann, und R₅ eine C₁₋₄-Alkoxygruppe bedeutet, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist; 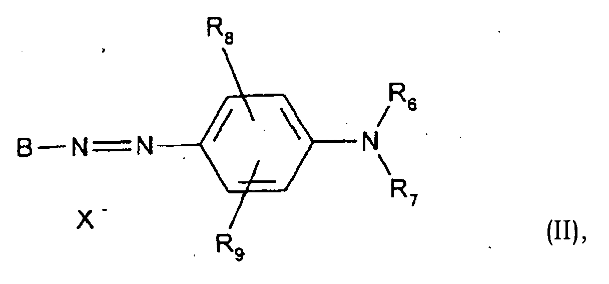 wobei in der Formel (II) bedeuten:
**R₆** ein Wasserstoffatom oder eine C₁₋₄-Alkylgruppe,
**R₇** ein Wasserstoffatom, eine Alkylgruppe, die mit der Gruppe -CN oder einer Aminogruppe substituiert sein kann, oder 4'-Aminophenyl oder R₇ bildet mit R₆ einen gegebenenfalls sauerstoffhaltigen und/oder stickstoffhaltigen Heterocyclus, der mit einer C₁₋₄-Alkylgruppe substituiert sein kann,
**R₈** und **R₉,** die gleich oder verschieden sind, ein Wasserstoffatom, ein Halogenatom, wie Brom, Chlor, Iod oder Fluor, eine C₁₋₄-Alkylgruppe, eine C₁₋₄-Alkoxygruppe oder die Gruppe -CN,
**X⁻** ein Anion, das vorzugsweise unter Chlorid, Methylsulfat und Acetat ausgewählt ist,
**B** eine Gruppe, die unter den folgenden Strukturen B1 bis B6 ausgewählt ist: 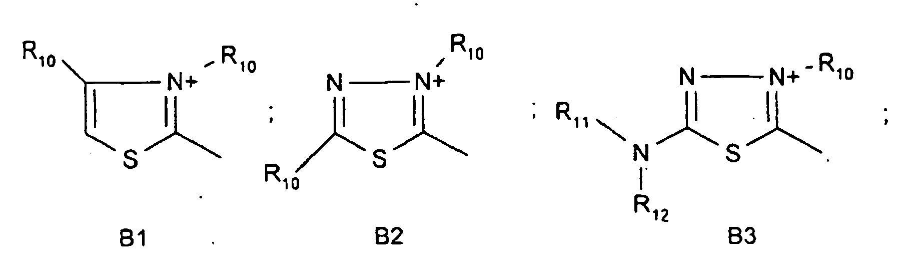 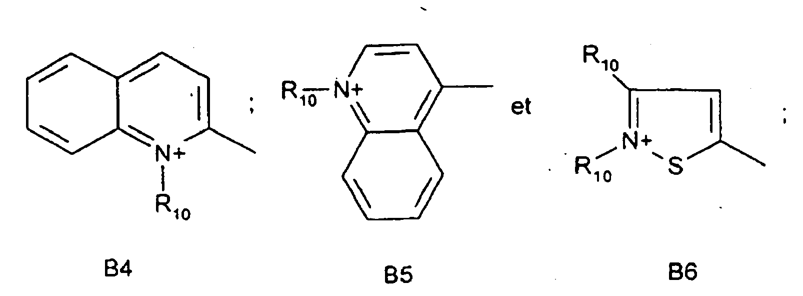
worin die Gruppe R₁₀ eine C₁₋₄-Alkylgruppe bedeutet und die Gruppen R₁₁ und R₁₂, die gleich oder verschieden sind, Wasserstoff oder C₁₋₄-Alkyl bedeuten; 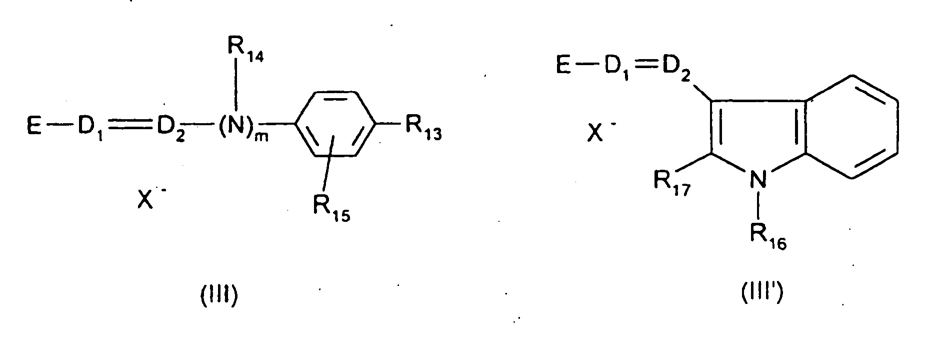 wobei in den Formeln (III) und (III') bedeuten:
**R₁₃** ein Wasserstoffatom, eine C₁₋₄-Alkoxygruppe, ein Halogenatom, wie Brom, Chlor, Iod oder Fluor, oder eine Aminogruppe,
**R₁₄** ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig ist und/oder mit einer oder mehreren C₁₋₄-Alkylgruppen substituiert ist;
**R₁₅** ein Wasserstoffatom oder ein Halogenatom, wie Brom, Chlor,
Iod oder Fluor,
**R₁₆** und **R₁₇,** die identisch oder voneinander verschieden sind,
Wasserstoff oder C₁₋₄-Alkyl,
**D₁** und **D₂,** die gleich oder verschieden sind, ein Stickstoffatom oder die Gruppe -CH,
**m** = 0 oder 1, mit der Maßgabe, dass die Gruppen D₁ und D₂ gleichzeitig -CH bedeuten und m = 0, wenn R₁₃ eine unsubstituierte Aminogruppe ist,
**X⁻** ein Anion, das vorzugsweise unter Chlorid, Methylsulfat und
Acetat ausgewählt ist,
**E** eine Gruppe, die unter den folgenden Strukturen E 1 bis E8 ausgewählt ist: 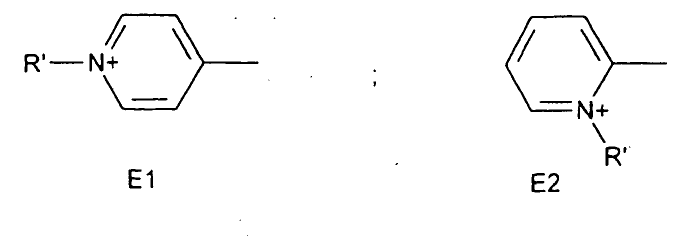 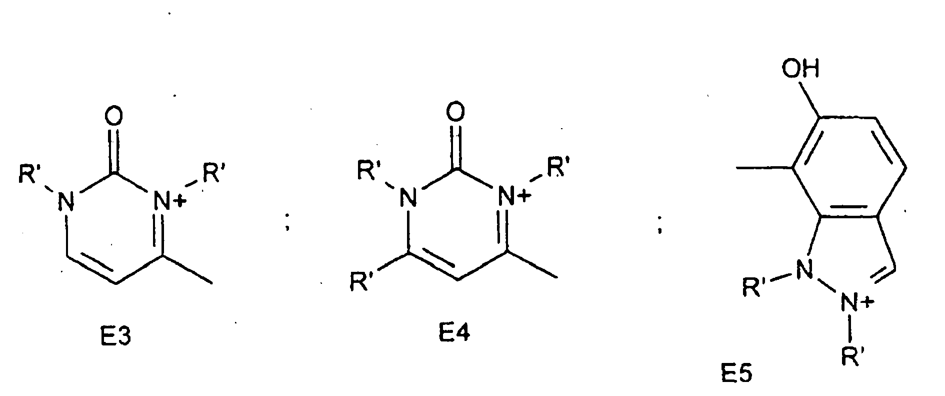 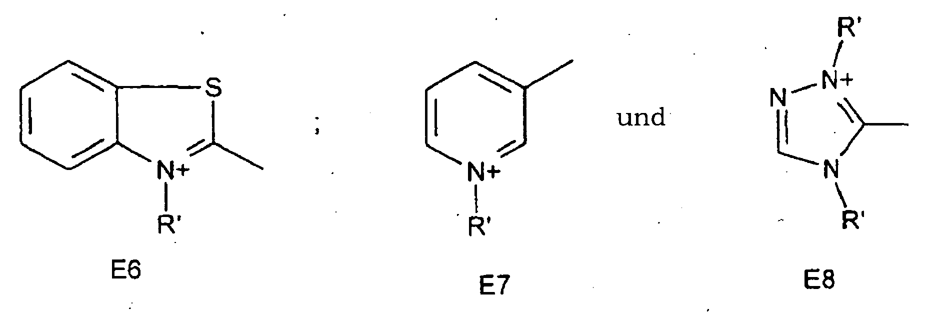
wobei R' eine C₁₋₄-Alkylgruppe bedeutet;
wenn m = 0 und D₁ ein Stickstoffatom bedeutet, kann die Gruppe E auch eine Gruppe der folgenden Struktur E9 sein: 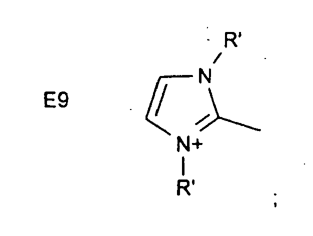 wobei R' eine C₁₋₄-Alkylgruppe bedeutet;
wobei die Zusammensetzung **dadurch gekennzeichnet ist, dass** sie ferner **(ii)** mindestens ein Polyol und/oder mindestens einen aliphatischen C₁₋₈-Ether eines C₃₋₉-Polyols und/oder aromatischen C₆₋₈-Ether eines C₂₋₉-Polyols enthält und wie in Anspruch 1 bis 16 definiert ist, während einer Zeitspanne aufgebracht wird, die zur Bildung der gewünschten Färbung ausreichend ist, ohne dass die Haare am Ende ausgespült werden.

19. Verfahren zum direkten Färben von Keratinfasern und insbesondere menschlichen Keratinfasern, wie dem Haar, nach einem der Ansprüche 17 oder 18, **dadurch gekennzeichnet, dass** es einen vorbereitenden Schritt umfasst, der darin besteht, einerseits eine Zusammensetzung (A), die in einem zum Färben geeigneten Medium mindestens einen in den vorhergehenden Ansprüchen definierten Direktfarbstoff (i) enthält, und andererseits eine Zusammensetzung (B) getrennt voneinander aufzubewahren, die in einem zum Färben geeigneten Medium mindestens ein Oxidationsmittel enthält, das von Oxidasen und/oder Oxidoreduktasen verschieden ist, und diese bei der Anwendung zu vermischen, bevor das Gemisch auf die Keratinfasern aufgebracht wird, wobei die Zusammensetzung (A) oder die Zusammensetzung (B) mindestens ein Polyol und/oder mindestens einen Polyolether, wie sie in Anspruch 1 definiert sind, enthält.

20. Vorrichtung mit mehreren Abteilungen oder "Kit" zum Färben mit mehreren Abteilungen, **dadurch gekennzeichnet, dass** eine erste Abteilung die Zusammensetzung (A) nach Anspruch 19 und eine andere Abteilung die Zusammensetzung (B) nach Anspruch 19 enthält.
